# EUROPEAN PATENT APPLICATION

(11) **EP 1 803 719 A1**
(43) Date of publication of application: **04.07.2007**
(21) Application number: 05795875.3
(22) Date of filing: 19.10.2005
(51) Int. Cl.: C07D 401/14, A61K 31/4439, A61K 31/4545, A61K 31/496, A61K 31/497, A61P 7/02, A61P 9/10, A61P 43/00, C07D 405/14

(54) **1,5-DIHETEROCYCLE-1H-TRIAZOLE DERIVATIVE**

(30) Priority: 19.10.2004 JP 2004303851
(71) Applicant: DAIICHI PHARMACEUTICAL CO., LTD., Chuo-ku, Tokyo 103-8234 (JP)
(72) Inventor: KANAYA, Naoaki, c/o Daiichi Pharmaceutical Co.Ltd, Edogawa-ku, Tokyo 1348630 (JP); FUJII, Kunihiko, c/o Daiichi Pharmaceutical Co.Ltd, Edogawa-ku, Tokyo 1348630 (JP)
(74) Representative: Blodig, Wolfgang
(86) International application number: PCT/JP2005/019207
(87) International publication number: WO 2006/043594

(57) **Abstract**

The present invention relates to a compound represented by Formula (I): 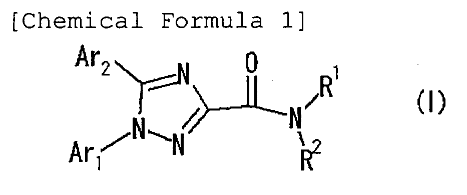 wherein Ar₁, Ar₂, R¹ and R² each represent a substituent,
a salt thereof, or a solvate of the compound or the salt, and to a medicine containing the same.

According to the present invention, a potent platelet aggregation suppressant which does not inhibit COX-1 and COX-2 is provided.

## Description

### Technical Field

The present invention relates to a triazole derivative having an inhibitory effect on platelet aggregation.

### Background Art

Platelets play an important role in preventing hemorrhage by aggregating and forming hemostatic thrombi when a blood vessel is damaged. On the other hand, plates are responsible for the formation of thrombus or embolus at a damaged site of vascular endothelium or in a narrowed area of blood vessel. These thrombus and embolus could lead to ischemic diseases such as myocardial infarction, angina pectoris, ischemic cerebrovascular disorder, peripheral vascular disorder and the like. So far, various platelet aggregation inhibitors have been used to prevent or treat ischemic diseases. Low-dose aspirin, among others, has been traditionally used as a platelet aggregation inhibitor, and its effects have been confirmed by APT (Antiplatelet Trialists' Collaboration) which carried out a meta-analysis on some clinical test results obtained by administering to 100, 000 patients (Non-Patent Document 1).
However, aspirin is known to have adverse side effects such as gastrointestinal hemorrhage and the like, namely, the so-called "aspirin-induced ulcer", and these side effects occur at a rate of one in 100 patients, irrespective of differences of the amount of aspirin administered to each patient (Non-Patent Document 2).
The inhibitory effect of aspirin on platelet aggregation is known to be associated with the inhibitory action of cyclooxygenases. Cyclooxygenases include cyclooxygenase-1 (COX-1) and cyclooxygenase-2 (COX-2). Aspirin inhibits platelet aggregation by inhibiting COX-1 selectively and irreversibly at a low of dose. However, such inhibition of COX-1 is causative of aspirin-induced ulcer (Non-Patent Documents 3 and 4). Antiplatelet drugs having COX-1 inhibition as their mechanism of action are concerned about induction of similar side effects. Thus there has been a growing demand for a platelet aggregation inhibitor which does not inhibit COX-1.

Besides, nonsteroidal antiinflammatory drugs are known to exhibit an antiinflammatory action by inhibiting COX-2.
Certain types of selective COX-2 inhibitors are reported to have side effects on cardiovascular vessels and cause thrombosis (Non-Patent Documents 5 to 7).
Some of the triazole derivatives are known to exhibit a COX-1 inhibitory action (Patent Documents 1 and 2).
[Patent Document 1] Pamphlet of International Patent Publication WO 03/040110
[Patent Document 2] Pamphlet of International Patent Publication WO 2004/060367
[Non-Patent Document 1] BMJ, Vol. 308, pp. 81-106 (1994)
[Non-Patent Document 2] BMJ, Vol. 321, pp. 1183-1187 (2000)
[Non-Patent Document 3] Neurology, Vol. 57, Suppl. 2, pp. S5-S7 (2001)
[Non-Patent Document 41] Drugs Today, Vol. 35, pp. 251-265 (1999)
[Non-Patent Document 5] N. Eng. J. Med, Vol. 343, pp. 1520-1528 (2000)
[Non-Patent Document 6] JAMA, Vol. 286, pp. 954-959 (2001)
[Non-Patent Document 7] Arthritis Rheum., Vol. 43, pp. 1891-1896 (2000)

### Disclosure of the Invention

### Problem to be solved by the Invention

It is an object of the present invention to provide a potent platelet aggregation inhibitor which does not inhibit COX-1 and COX-2.

The present inventors made an intensive study with the aim of obtaining such a platelet aggregation inhibitor, and as a result, found that a triazole derivative represented by the following Formula (I) has a strongly inhibitory effect on platelet aggregation without inhibiting COX-1 and COX-2. The present invention was accomplished as a cosequnce.

Thus, the present invention provides a compound represented by Formula (I):

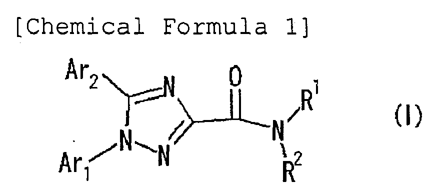

wherein Ar₁ and Ar₂ each independently represent a 5- or 6-membered aromatic heterocyclic group which may be substituted with 1 to 3 groups or atoms selected from a lower alkyl group which may be substituted, a lower alkynyl group, a carbamoyl group which may be substituted, a cyano group, an amino group which may be substituted, a hydroxyl group, a lower alkoxy group and a halogen atom; and R¹ and R² each independently represent a hydrogen atom, a lower alkyl group which may be substituted, an alicyclic heterocyclic group which may be substituted, a carbamoyl group which may be substituted, a hydroxyl group, a lower alkoxy group, or an amino group which may be substituted; or R¹ and R² represent, together with the nitrogen atom substituted with R¹ and R², a 4- to 7-membered alicyclic heterocyclic group formed thereby, wherein the 4- to 7-membered alicyclic heterocyclic group may have one nitrogen atom or oxygen atom, in addition to the nitrogen atom indicated in the formula, as the constituent atom, and the 4- to 7-membered alicyclic heterocyclic group may be substituted with one, or two to four identical or different substituents selected from the group consisting of a lower alkyl group which may be substituted, a carbamoyl group which may be substituted, an amino group which may be substituted, a hydroxyl group, a lower alkoxy group, an oxo group, a lower alkanoyl group, a lower alkylsulfonyl group and a halogen atom,
a salt thereof, or a solvate of the compound or the salt.

The present invention also provides a pharmaceutical composition containing the compound (I), a salt thereof, or a solvate of the compound or the salt, and a pharmaceutically acceptable carrier; a medicine containing the compound (I), a salt thereof, or a solvate of the compound or the salt as an active ingredient; a platelet aggregation suppressant containing the compound (I), a salt thereof, or a solvate of the compound or the salt as an active ingredient; and a prophylactic and/or therapeutic agent for ischemic diseases containing the compound (I), a salt thereof, or a solvate of the compound or the salt as an active ingredient.
The present invention also provides a method of inhibiting platelet aggregation, the method comprising administering an effective amount of the compound (I), a salt thereof, or a solvate of the compound or the salt; and a method of preventing and/or treating ischemic diseases, the method comprising administering an effective amount of the compound (I), a salt thereof, or a solvate of the compound or the salt.
Furthermore, the present invention provides a use of the compound (I), a salt thereof, or a solvate of the compound or the salt, for the production of a platelet aggregation inhibitor; and a use of the compound (I), a salt thereof, or a solvate of the compound or the salt, for the production of a prophylactic and/or therapeutic agent for ischemic diseases.

### Effect of the Invention

The compound (I) of the present invention, a salt thereof, or a solvate of the compound or the salt has an effect of inhibiting thrombus formation by potently suppressing platelet aggregation without inhibiting COX-1 and COX-2. Thus, the compound (I), a salt thereof, and a solvate of the compound or the salt are useful for the prevention and/or treatment of ischemic diseases caused by thrombi and emboli, such as myocardial infarction, angina pectoris (chronic stable angina, unstable angina, and the like), ischemic cerebrovascular disorder (transient ischemic attack (TIA), cerebral infarction, and the like), peripheral vascular disorder, occlusion after replacement with an artificialvessel,thrombotic occlusion after coronary artery intervention (coronary artery bypass grafting(CABG), percutaneous transluminal coronary angioplasty (PTCA), stent placement, and the like), diabetic retinopathy and nephropathy, occlusion after replacement with an artificial heart valve, and the like. They are also useful for the prevention and/or treatment of thrombi and emboli associated with vascular surgery, blood extracorporeal circulation and the like. Furthermore, they are useful for an improvement in ischemic symptoms associated with chronic arterial occlusion, such as ulcer, pain, cold sensation and the like.

### Best Mode for Carrying Out the Invention

Ar₁ and Ar₂ of the compound represented by Formula (I) each independently represent a 5- or 6-membered aromatic heterocyclic group which may be substituted with one to three groups or atoms selected from a lower alkyl group which may be substituted, a lower alkynyl group, a carbamoyl group which may be substituted, a cyano group, an amino group which may be substituted, a hydroxyl group, a lower alkoxy group and a halogen atom.

The 5-membered aromatic heterocyclic group may be exemplified by a pyrrolyl group, a pyrazolyl group, an imidazolyl group, a triazolyl group, a furyl group, a thienyl group, an oxazolyl group, an isoxazolyl group, an oxadiazolyl group, a thiazolyl group, an isothiazolyl group, a thiadiazolyl group, or the like. Among them, a pyrrolyl group, an imidazolyl group, a pyrazolyl group, an oxazolyl group, a triazolyl group, and a thiazolyl group are preferred. Among them, a 1H-pyrrol-1-yl group, a 1H-pyrrol-2-yl group, a 1H-pyrrol-3-yl group, a 1H-pyrazol-3-yl group, a 1H-imidazol-2-yl group, a 1H-imidazol-4-yl group, an oxazol-2-yl group, an oxazol-4-yl group, a 1H-1,2,4-triazol-3-yl group, and a thiazol-4-yl group are preferred.

The 6-membered aromatic heterocyclic group is preferably a 6-membered nitrogen-containing aromatic heterocyclic group, and may be exemplified by a pyridyl group, a pyridazinyl group, a pyrimidinyl group, a pyrazinyl group, a triazinyl group, or the like. Among them, a pyridyl group, a pyridazinyl group, a pyrimidinyl group, and a pyrazinyl group are preferred. Further, a 2-pyridyl group, a 3-pyridyl group, a 3-pyridazinyl group, a 2-pyrimidinyl group, a 4-pyrimidinyl group, and a 2-pyrazinyl group are more preferred, and a 2-pyridyl group, a 3-pyridyl group, a 3-pyridazinyl group, and a 2-pyrazinyl group is most preferred.

Furthermore, when Ar₁ is a 3-pyridyl group, a 3-pyridazinyl group or a 2-pyrazinyl group, Ar₂ is preferably a 1H-imidazol-4-yl group, a 1H-pyrazol-3-yl group, a 2-pyridyl group, a 4-pyrimidinyl group, or a 2-pyrazinyl group, and most preferably a 1H-imidazol-4-yl group, a 1H-pyrazol-3-yl group, a 2-pyridyl group, or a 2-pyrazinyl group. When Ar₁ is a 2-pyrdinyl group, a 2-pyrimidinyl group or a 4-pyrimidinyl group, Ar₂ is preferably a 2-pyridyl group, a 3-pyridyl group, a 3-pyridazinyl group, a 4-pyrimidinyl group, or a 2-pyrazinyl group, and most preferably a 3-pyridyl group or a 2-pyrazinyl group.

The 5- or 6-membered aromatic heterocyclic group represented by Ar₁ or Ar₂ may be substituted with: 1) a lower alkyl group which may be substituted, 2) a lower alkynyl group, 3) a carbamoyl group which may be substituted, 4) a cyano group, 5) an amino group which may be substituted, 6) a hydroxyl group, 7) a lower alkoxy group, or 8) a halogen atom. The number of substituent is one, or two or three of identical or different ones, and is preferably one. For the 6-membered aromatic heterocyclic group, the position of substitution is preferably the p-position to the bonding to the triazole ring.

Specific examples of the substituents of the 5- or 6-membered aromatic heterocyclic ring include the following.
1) A lower alkyl group which may be substituted: the lower alkyl group of a lower alkyl group which may be substituted means a straight-chained, branched or cyclic alkyl group having 1 to 6 carbon atoms. Specifically, a methyl group, an ethyl group, an n-propyl group, an isopropyl group, an n-butyl group, an isobutyl group, a sec-butyl group, a tert-butyl group, an n-pentyl group, an isopentyl group, a neopentyl group, a tert-pentyl group, an n-hexyl group, an isohexyl group, a cyclopropyl group, a 1-methylcyclopropyl group, a cyclobutyl group, a cyclopentyl group, a cyclohexyl group, a cyclopropylmethyl group, a cyclopentylmethyl group, or the like may be mentioned. Among them, a methyl group, an ethyl group, or an n-propyl group is preferred, and a methyl group is particularly preferred.

These lower alkyl groups may be substituted with one, or two to three identical or different substituent groups or atoms selected from the following substituent group consisting of (a) to (g). These substituent groups or atoms may be substituted, insofar as substitution can be achieved, on the same carbon atom of the lower alkyl group, or may be substituted on different carbon atoms.
a) A carbamoyl group which may be substituted with one, or two identical or different lower alkyl groups: This carbamoyl group means an unsubstituted carbamoyl group, or a carbamoyl group substituted with one to two lower alkyl groups described above. Specifically, a methylcarbamoyl group, an ethylcarbamoyl group, a dimethylcarbamoyl group, an N-methyl-N-ethylcarbamoyl group, and the like may be mentioned. Among them, an unsubstituted carbamoyl group, a methylcarbamoyl group or a dimethylcarbamoyl group is preferred.

b) A lower alkanoyl group: The lower alkanoyl group means a straight-chained or branched alkanoyl group having 1 to 6 carbon atoms. Specifically, a formyl group, an acetyl group, an n-propionyl group, an n-butyryl group, an isobutyryl group, or the like may be mentioned.

c) An amino group which may be substituted with one, or two identical or different substituents selected from a lower alkyl group, a lower alkanoyl group, a lower alkylsulfonyl group, a lower alkoxycarbonyl group, and a carbamoyl group which may be substituted with a lower alkyl group: This amino group means an unsubstituted amino group, or an amino group substituted with one, or two identical or different substituents selected from a lower alkyl group, a lower alkanoyl group, a lower alkylsulfonyl group, and a carbamoyl group which may be substituted with a lower alkyl group. The lower alkyl group means the lower alkyl groups described above. The lower alkanoyl group means, as described in (b), a straight-chained or branched alkanoyl group having 1 to 6 carbon atoms, and may be exemplified by a formyl group, an acetyl group, an n-propionyl group, an n-butyryl group, an isobutyryl group, or the like. The lower alkylsulfonyl group means a sulfonyl group substituted with the above-mentioned lower alkyl groups. Specifically, a methylsulfonyl group, an ethylsulfonyl group, an n-propylsulfonyl group, an isopropylsulfonyl group, an n-butylsulfonyl group, an isobutylsulfonyl group, a tert-butylsulfonyl group, an n-pentylsulfonyl group, an isopentylsulfonyl group, a cyclopropylsulfonyl group, a cyclohexylsulfonyl group and the like may be mentioned. The lower alkoxycarbonyl group means a carbonyl group substituted with the above-mentioned lower alkoxy groups, and specifically, a methoxycarbonyl group, an ethoxycarbonyl group, a propoxycarbonyl group, an isopropoxycarbonyl group, a butoxycarbonyl group, an isobutoxycarbonyl group, a tert-butoxycarbonyl group, a pentoxycarbonyl group, a hexoxycarbonyl group, and the like may be mentioned. The carbamoyl group which may be substituted with a lower alkyl group means a carbamoyl group, or a carbamoyl group substituted with the above-mentioned lower alkyl groups. Specifically, a carbamoyl group, a methylcarbamoyl group, an ethylcarbamoyl group, an n-propylcarbamoyl group, an isopropylcarbamoyl group, an n-butylcarbamoyl group, an isobutylcarbamoyl group, a tert-butylcarbamoyl group, an n-pentylcarbamoyl group, an isopentylcarbamoyl group, a cyclopropylcarbamoyl group, a cyclohexylcarbamoyl group, a dimethylcarbamoyl group, a diethylcarbamoyl group, an N-methyl-N-ethylcarbamoyl group, and the like may be mentioned.

Therefore, the amino group substituted with one, or two identical or different substituents selected from the group consisting of a lower alkyl group, a lower alkanoyl group, a lower alkylsulfonyl group and a lower alkoxycarbonyl group, may be exemplified by a methylamino group, an ethylamino group, an n-propylamino group, an isopropylamino group, a cyclopropylamino group, an n-butylamino group, an isobutylamino group, a cyclopentylmethylamino group, a dimethylamino group, a diethylamino group, a di-n-propylamino group, a di-n-butylamino group, an N-methyl-N-ethylamino group, an N-ethyl-N-n-propylamino group, an N-methyl-N-cyclopentylmethylamino group, a formylamino group, an acetylamino group, an n-propionylamino group, an N-methyl-N-acetylamino group, an N-ethyl-N-acetylamino group, a methylsulfonylamino group, an ethylsulfonylamino group, isopropylsulfonylamino group, n-butylsulfonylamino group, a cyclopropylsulfonylamino group, a cyclobutanesulfonylamino group, an N-methyl-N-methylsulfonylamino group, an N-ethyl-N-methylsulfonylamino group, a methoxycarbonylamino group, an ethoxycarbonylamino group, a tert-butoxycarbonylamino group, a methylcarbamoylamino group, an ethylcarbamoylamino group, an n-propylcarbamoylamino group, an isopropylcarbamoylamino group, an n-butylcarbamoylamino group, an isobutylcarbamoylamino group, a dimethylcarbamoylamino group, a diethylcarbamoylamino group, an N-methyl-N-ethylcarbamoylamino group, or the like.

d) A hydroxyl group.

e) A lower alkoxy group: The lower alkoxy group means an alkoxy group containing the above-mentioned lower alkyl groups in the structure. Specifically, a methoxy group, an ethoxy group, an n-propoxy group, an isopropoxy group, an n-butoxy group, an isobutoxy group, an n-pentoxy group, a cyclopentyloxy group, or the like may be mentioned. Among them, a methoxy group or an ethoxy group is preferred, and a methoxy group is particularly preferred.

f) A lower alkylsulfonyl group: The lower alkylsulfonyl group means the same group as the lower alkylsulfonyl group mentioned as the substituent of the amino group in c) above, and may be exemplified by a methylsulfonyl group, an ethylsulfonyl group, an n-propylsulfonyl group, an isopropylsulfonyl group, an n-butylsulfonyl group, an isobutylsulfonyl group, a tert-butylsulfonyl group, an n-pentylsulfonyl group, an isopentylsulfonyl group, a cyclopropylsulfonyl group, a cyclohexylsulfonyl group, or the like.

g) A halogen atom: The halogen atom may be exemplified by fluorine, chlorine, bromine and iodine. Among them, fluorine or chlorine is preferred, and fluorine is particularly preferred.

2) A lower alkynyl group: The lower alkynyl group means a straight-chained, branched or cyclic alkynyl group having 2 to 6 carbon atoms. Specifically, an ethynyl group, a 1-propynyl group, a 2-propynyl group, a 1-butynyl group, a 2-butynyl group, a 1-pentynyl group, a 2-pentynyl group and the like may be mentioned. Among them, an ethynyl group, a 1-propynyl group, or a 2-propynyl group is preferred, and an ethynyl group is particularly preferred.

3) A carbamoyl group which may be substituted: The carbamoyl group which may be substituted means an unsubstituted carbamoyl group, or a carbamoyl group substituted with one, or two identical or different lower alkyl groups described above. Specifically, a methylcarbamoyl group, an ethylcarbamoyl group, an n-propylcarbamoyl group, a dimethylcarbamoyl group, a diethylcarbamoyl group, an N-methyl-N-ethylcarbamoyl group, and the like may be mentioned.

4) A cyano group.

5) An amino group which may be substituted: The amino group which may be substituted means the same group as the "c) amino group which may be substituted," mentioned as the substituent of the above-mentioned lower alkyl group, and means an unsubstituted amino group, or an amino group which may be substituted with one, or two identical or different substituents selected from a lower alkyl group, a lower alkanoyl group, a lower alkylsulfonyl group, a lower alkoxycarbonyl group, and a carbamoyl group which may be substituted with a lower alkyl group. Specifically, a methylamino group, an ethylamino group, an n-propylamino group, an isopropylamino group, a cyclopropylaminogroup, an n-butylamino group, an isobutylamino group, a cyclopentylmethylamino group, a dimethylamino group, a diethylamino group, a di-n-propylamino group, a di-n-butylamino group, an N-methyl-N-ethylamino group, an N-ethyl-N-n-propylamino group, an N-methyl-N-cyclopentylmethylamino group, a formylamino group, an acetylamino group, an n-propionylamino group, an N-methyl-N-acetylamino group, an N-ethyl-N-acetylamino group, a methylsulfonylamino group, an ethylsulfonylamino group, an isopropylsulfonylamino group, an n-butylsulfonylamino group, a cyclopropylsulfonylamino group, a cyclobutanesulfonylamino group, an N-methyl-N-methylsulfonylamino group, an N-ethyl-N-methylsulfonylamino group, a methoxycarbonylamino group, an N-methyl-N-methoxycarbonylamino group, an ethoxycarbonylamino group, an N-methyl-N-ethoxycarbonylamino group, a tert-butoxycarbonylamino group, an N-methyl-N-butoxycarbonylamino group, a methylcarbamoylamino group, an ethylcarbamoylamino group, an n-propylcarbamoylamino group, an isopropylcarbamoylamino group, an n-butylcarbamoylamino group, an isobutylcarbamoylamino group, a dimethylcarbamoylamino group, a diethylcarbamoylamino group, an N-methyl-N-ethylcarbamoylamino group, and the like may be mentioned. Among them, an unsubstituted amino group, an acetylamino group, a methylsulfonylamino group, and a tert-butoxycarbonylamino group are preferred.

6) A hydroxyl group.

7) A lower alkoxy group: The lower alkoxy group means an alkoxy group containing the above-mentioned lower alkyl group in the structure. Specifically, a methoxy group, an ethoxy group, an n-propoxy group, an isopropoxy group, an n-butoxy group, an isobutoxy group, a tert-butoxy group, an n-pentoxy group, a cyclopentyl oxy group, and the like may be mentioned. Among them, a methoxy group and an ethoxy group are preferred, and a methoxy group is particularly preferred.

8) A halogen atom: The halogen atom may be exemplified by fluorine, chlorine, bromine and iodine. Among them, fluorine or chlorine is preferred, and fluorine is particularly preferred.

Among these substituents of Ar₁ or Ar₂ listed in (1) to (8), a lower alkyl group which may be substituted, a carbamoyl group which may be substituted, a cyano group, an amino group which may be substituted, a lower alkoxy group, and a halogen atom are preferred.

Therefore, specific examples of Ar₁ and Ar₂ include a pyrrolyl group, a methylpyrrolyl group, a carbamoylpyrrolyl group, a dimethylcarbamoylpyrrolyl group, a cyanopyrrolyl group, a hydroxypyrrolyl group, a methoxypyrrolyl group, a fluoropyrrolyl group, a chloropyrrolyl group, an aminopyrrolyl group, a methylaminopyrrolyl group, a dimethylaminopyrrolyl group, a hydroxymethylpyrrolyl group, an aminomethylpyrrolyl group, a methylaminomethylpyrrolyl group, a dimethylaminomethylpyrrolyl group, a hydroxymethylpyrrolyl group;

a pyrazolyl group, a methylpyrazolyl group, a carbamoylpyrazolyl group, a dimethylcarbamoylpyrazolyl group, a cyanopyrazolyl group, a hydroxypyrazolyl group, a methoxypyrazolyl group, a fluoropyrazolyl group, a chloropyrazolyl group, an aminopyrazolyl group, a methylaminopyrazolyl group, a dimethylaminopyrazolyl group, a hydroxymethylpyrazolyl group, an aminomethylpyrazolyl group, a methylaminomethylpyrazolyl group, a dimethylaminomethylpyrazolyl group;

an imidazolyl group, a methylimidazolyl group, a carbamoylimidazolyl group, a dimethylcarbamoylimidazolyl group, a cyanoimidazolyl group, a hydroxyimidazolyl group, a methoxyimidazolyl group, a fluoroimidazolyl group, a chloroimidazolyl group, an aminoimidazolyl group, a methylaminoimidazolyl group, a dimethylaminoimidazolyl group, a hydroxymethylimidazolyl group, an aminomethylimidazolyl group, a methylaminomethylimidazolyl group, a dimethylaminomethylimidazolyl group;

an oxazolyl group, a methyloxazolyl group, a carbamoyloxazolyl group, a dimethylcarbamoyloxazolyl group, a cyanooxazolyl group, a hydroxyoxazolyl group, a methoxyoxazolyl group, a fluorooxazolyl group, a chlorooxazolyl group, an aminooxazolyl group, a methylaminooxazolyl group, a dimethylaminooxazolyl group, a hydroxymethyloxazolyl group, an aminomethyloxazolyl group, a methylaminomethyloxazolyl group, a dimethylaminomethyloxazolyl group;

a triazolyl group, a methyl triazolyl group, a carbamoyltriazolyl group, a dimethylcarbamoyltriazolyl group, a cyanotriazolyl group, a hydroxytriazolyl group, a methoxytriazolyl group, a fluorotriazolyl group, a chlorotriazolyl group, an aminotriazolyl group, a methylaminotriazolyl group, a dimethylaminotriazolyl group, a hydroxymethyltriazolyl group, an aminomethyltriazolyl group, a methylaminomethyltriazolyl group, a dimethylaminomethyltriazolyl group, a thiazolyl group, a methylthiazolyl group, an aminothiazolyl group, an aminomethylthiazolyl group;

a pyridyl group, a methyl pyridyl group, a carbamoylpyridyl group, a dimethylcarbamoylpyridyl group, a cyanopyridyl group, a hydroxypyridyl group, a methoxypyridyl group, a fluoropyridyl group, a chloropyridyl group, an aminopyridyl group, a methylaminopyridyl group, a dimethylaminopyridyl group, a methylsulfonylaminopyridyl group, an acetylaminopyridyl group, a methoxycarbonylaminopyridyl group, a tert-butoxycarbonylaminopyridyl group, a hydroxymethylpyridyl group, an aminomethylpyridyl group, a methylaminomethylpyridyl group, a dimethylaminomethylpyridyl group, a methylsulfonylaminomethylpyridyl group, an acetylaminomethylpyridyl group, a methoxycarbonylaminomethylpyridyl group, a tert-butoxycarbonylaminomethylpyridyl group;

a pyridazinyl group, a methylpyridazinyl group, a carbamoylpyridazinyl group, a dimethylcarbamoylpyridazinyl group, a cyanopyridazinyl group, a hydroxypyridazinyl group, a methoxypyridazinyl group, a fluoropyridazinyl group, a chloropyridazinyl group, an aminopyridazinyl group, a methyl aminopyridazinyl group, a dimethyl aminopyridazinyl group, a methylsulfonylaminopyridazinyl group, an acetylaminopyridazinyl group, a methoxycarbonylaminopyridazinyl group, a tert-butoxycarbonylaminopyridazinyl group, a hydroxymethylpyridazinyl group, an aminomethylpyridazinyl group, a methylaminomethylpyridazinyl group, a dimethylaminomethylpyridazinyl group, a methylsulfonylaminomethylpyridazinyl group, an acetylaminomethylpyridazinyl group, a methoxycarbonylaminomethylpyridazinyl group, a tert-butoxycarbonylaminomethylpyridazinyl group;

a pyrimidinyl group, a methylpyrimidinyl group, a carbamoylpyrimidinyl group, a dimethylcarbamoylpyrimidinyl group, a cyanopyrimidinyl group, a hydroxypyrimidinyl group, a methoxypyrimidinyl group, a fluoropyrimidinyl group, a chloropyrimidinyl group, an aminopyrimidinyl group, a methylaminopyrimidinylgroup, a dimethylaminopyrimidinyl group, a methylsulfonylaminopyrimidinyl group, an acetylaminopyrimidinyl group, a methoxycarbonylaminopyrimidinyl group, a tert-butoxycarbonylaminopyrimidinyl group, a hydroxymethylpyrimidinyl group, an aminomethylpyrimidinyl group, a methylaminomethylpyrimidinyl group, a dimethylaminomethylpyrimidinyl group, a methylsulfonylaminomethylpyrimidinyl group, an acetylaminomethylpyrimidinyl group;

a pyrazinyl group, a methylpyrazinyl group, a carbamoylpyrazinyl group, a dimethylcarbamoylpyrazinyl group, a cyanopyrazinyl group, a hydroxypyrazinyl group, a methoxypyrazinyl group, a fluoropyrazinyl group, a chloropyrazinyl group, an aminopyrazinyl group, a methylaminopyrazinyl group, a dimethylaminopyrazinyl group, a methylsulfonylaminopyrazinyl group, an acetylaminopyrazinyl group, a methoxycarbonylaminopyrazinyl group, a tert-butoxycarbonylaminopyrazinyl group, a hydroxymethylpyrazinyl group, an aminomethylpyrazinyl group, a methylaminomethylpyrazinyl group, a dimethylaminomethylpyrazinyl group, a methylsulfonylaminomethylpyrazinyl group, an acetylaminomethylpyrazinyl group, a methoxycarbonylaminomethylpyrazinyl group, a tert-butoxycarbonylaminomethylpyrazinyl group, and the like.

Among them, a pyrrolyl group, a methylpyrrolyl group, a hydroxymethylpyrrolyl group, a methoxypyrrolyl group, an imidazolyl group, a methylimidazolyl group, a methoxyimidazolyl group, a pyrazolyl group, a methylpyrazolyl group, a methoxypyrazolyl group, an oxazolyl group; a triazolyl group, a thiazolyl group, a pyridyl group, a methylpyridyl group, a carbamoylpyridyl group, a cyanopyridyl group, an aminopyridyl group, a methylsulfonylaminopyridyl group, an acetylaminopyridyl group, a butoxycarbonylaminopyridyl group, a hydroxypyridyl group, a methoxypyridyl group, a fluoropyridyl group, a chloropyridyl group, a hydroxymethylpyridyl group, an aminomethylpyridyl group, a methylsulfonylaminomethylpyridyl group, an acetylaminomethylpyridyl group, a pyrimidinyl group, a methylpyrimidinyl group, a methoxypyrimidinyl group, a pyrazinyl group, a methylpyrazinyl group, a methoxypyrazinyl group, a carbamoylpyrazinyl group, and an aminopyrazinyl group are preferred.

More specifically, a 1H-pyrrol-1-yl group, a 3-methyl-1H-pyrrol-1-yl group, a 3-hydroxymethyl-1H-pyrrol-1-yl group, a 3-aminomethyl-1H-pyrrol-1-yl group, a 3-methylaminomethyl-1H-pyrrol-1-yl group, a 3-dimethylaminomethyl-1H-pyrrol-1-yl group, a 3-carbamoyl-1H-pyrrol-1-yl group, a 3-hydroxy-1H-pyrrol-1-yl group, a 1H-pyrrol-2-ylgroup, a 1-methyl-1H-pyrrol-2-yl group, a 1H-pyrrol-3-yl group, a 1-methyl-1H-pyrrol-3-yl group;

a 1H-imidazol-2-yl group, a 1-methyl-1H-imidazol-2-yl group, a 1H-imidazol-4-yl group, a 1-methyl-1H-imidazol-4-yl group;

a 1H-pyrazol-3-yl group, a 1-methyl-1H-pyrazol-3-yl group;

an oxazol-2-yl group, an oxazol-4-yl group;

a 1H-1,2,4-triazol-3-yl group;

a thiazol-4-yl group;

a 2-pyridyl group, a 3-pyridyl group, a 4-pyridyl group, a 3-methoxy-2-pyridyl group, a 3-methyl-2-pyridyl group, a 3-fluoro-2-pyridyl group, a 4-methyl-2-pyridyl group, a 4-ethyl-2-pyridyl group, a 4-cyano-2-pyridyl group, a 4-carbamoyl-2-pyridyl group, a 4-methoxy-2-pyridyl group, a 4-ethoxy-2-pyridyl group, a 4-chloro-2-pyridyl group, a 4-fluoro-2-pyridyl group, a 4-amino-2-pyridyl group, a 4-methylamino-2-pyridyl group, a 4-dimethylamino-2-pyridyl group, a 4-methylsulfonylamino-2-pyridyl group, a 4-acetylamino-2-pyridyl group, a 4-hydroxy-2-pyridyl group, a 4-aminomethyl-2-pyridyl group, a 4-methylaminomethyl-2-pyridyl group, a 4-dimethylaminomethyl-2-pyridyl group, a 4-methylsulfonylamino-2-pyridyl group, a 4-acetylamino-2-pyridyl group, a 5-methyl-2-pyridyl group, a 5-ethynyl-2-pyridyl group, a 5-hydroxy-2-pyridyl group, a 5-methoxy-2-pyridyl group, a 5-cyano-2-pyridyl group, a 5-amino-2-pyridyl group, a 5-methylamino-2-pyridyl group, a 5-dimethylamino-2-pyridyl group, a 5-methylsulfonylamino-2-pyridyl group, a 5-acetylamino-2-pyridyl group, a 5-methoxycarbonylamino-2-pyridyl group, a 5-tert-butoxycarbonylamino-2-pyridyl group, a 5-carbamoyl-2-pyridyl group, a 5-methylcarbamoyl-2-pyridyl group, a 5-dimethylcarbamoyl-2-pyridyl group, a 5-chloro-2-pyridyl group, a 5-fluoro-2-pyridyl group, a 5-hydroxymethyl-2-pyridyl group, a 5-aminomethyl-2-pyridyl group, a 5-methylaminomethyl-2-pyridyl group, a 5-dimethylaminomethyl-2-pyridyl group, a 5-methylsulfonylaminomethyl-2-pyridyl group, a 5-acetylaminomethyl-2-pyridyl group, a 6-methyl-2-pyridyl group, a 6-methoxy-2-pyridylgroup, a 6-fluoro-2-pyridylgroup, a 3-pyridyl group, a 6-methyl-3-pyridyl group, a 6-methoxy-3-pyridyl group;

a 3-pyridazinyl group, a 6-methoxy-3-pyridazinyl group, a 6-methyl-3-pyridazinyl group, a 2-pyrimidinyl group;

a 5-methoxy-2-pyrimidinyl group, a 5-methyl-2-pyrimidinyl group, a 4-pyrimidinyl group;

a 2-pyrazinyl group, a 5-methoxy-2-pyrazinyl group, a 5-methyl-2-pyrazinyl group, a 5-amino-2-pyrazinyl group, a 5-methylsulfonylamino-2-pyrazinyl group, a 5-acetylamino-2-pyrazinyl group, a 5-aminomethyl-2-pyrazinyl group, a 5-methylsulfonylamino-2-pyrazinyl group, a 5-acetylaminomethyl-2-pyrazinyl group, and the like are preferred.

Among them, a 1H-pyrrol-1-yl group, a 3-methyl-1H-pyrrol-1-yl group, a 3-hydroxymethyl-1H-pyrrol-1-yl group, a 3-aminomethyl-1H-pyrrol-1-yl group, a 3-methylaminomethyl-1H-pyrrol-1-yl group, a 3-dimethylaminomethyl-1H-pyrrol-1-yl group, a 3-carbamoyl-1H-pyrrol-1-yl group, a 1H-pyrrol-2-yl group, a 3-methyl-1H-pyrrol-2-yl group, a 3-hydroxymethyl-1H-pyrrol-2-yl group, a 3-aminomethyl-1H-pyrrol-2-yl group, a 3-methylaminomethyl-1H-pyrrol-2-yl group, a 3-dimethylaminomethyl-1H-pyrrol-2-yl group, a 3-carbamoyl-1H-pyrrol-2-yl group, a 4-methyl-1H-pyrrol-2-yl group, a 4-hydroxymethyl-1H-pyrrol-2-yl group, a 4-aminomethyl-1H-pyrrol-2-yl group, a 4-methylaminomethyl-1H-pyrrol-2-yl group, a 4-dimethylaminomethyl-1H-pyrrol-2-yl group, a 3-carbamoyl-1H-pyrrol-2-yl group, a 1H-pyrrol-3-yl group, a 1-methyl-1H-pyrrol-3-yl group;

a 1H-imidazol-2-yl group, a 1-methyl-1H-imidazol-2-yl group, a 1H-imidazol-4-yl group, a 1-methyl-1H-imidazol-4-yl group;

a 1H-pyrazol-3-yl group, a 1-methyl-1H-pyrazol-3-yl group;

a 2-pyridyl group, a 3-pyridyl group, a 4-pyridyl group, a 3-methoxy-2-pyridyl group, a 3-methyl-2-pyridyl group, a 3-fluoro-2-pyridyl group, a 4-amino-2-pyridyl group, a 4-fluoro-2-pyridyl group, a 5-methyl-2-pyridyl group, a 5-ethynyl-2-pyridyl group, a 5-methoxy-2-pyridyl group, a 5-fluoro-2-pyridyl group, a 5-cyano-2-pyridyl group, a 5-carbamoyl-2-pyridyl group, a 5-amino-2-pyridyl group, a 5-methylsulfonylamino-2-pyridyl group, a 5-acetylamino-2-pyridyl group, a 5-tert-butoxycarbonylamino-2-pyridyl group, a 5-hydroxymethyl-2-pyridyl group, a 5-aminomethyl-2-pyridyl group, a 5-methylsulfonylaminomethyl-2-pyridyl group, a 5-acetylaminomethyl-2-pyridyl group, a 6-methyl-2-pyridyl group, a 6-methoxy-2-pyridyl group, a 6-fluoro-2-pyridyl group, a 6-methoxy-3-pyridyl group, a 6-methyl-3-pyridyl group;

a 6-methoxy-3-pyridazinyl group, a 6-methyl-3-pyridazinyl group;

a 2-pyrimidinyl group, a 5-methoxy-2-pyrimidinyl group, a 5-methyl-2-pyrimidinyl group, a 4-pyrimidinyl group;

a 2-pyrazinyl group, a 5-methoxy-2-pyrazinyl group, a 5-methyl-2-pyrazinylgroup,a 5-aminomethyl-2-pyrazinylgroup, a 5-methylamino-2-pyrazinyl group, a 5-dimethylamino-2-pyrazinyl group, a 5-methylsulfonylaminomethyl-2-pyrazinyl group, a 5-amino-2-pyrazinyl group, a 5-methylamino-2-pyrazinyl group, a 5-dimethylamino-2-pyrazinyl group, a 5-methylsulfonylamino-2-pyrazinyl group, a 5-acetylamino-2-pyrazinyl group, and the like are more preferred.

Among them, a 1-methyl-1H-imidazol-4-yl group, a 1-methyl-1H-pyrazol-3-yl group, a 2-pyridyl group, a 5-methyl-2-pyridyl group, a 5-ethynyl-2-pyridyl group, a 5-hydroxy-2-pyridyl group, a 5-methoxy-2-pyridyl group, a 5-cyano-2-pyridyl group, a 5-amino-2-pyridyl group, a 5-methylamino-2-pyridyl group, a 5-dimethylamino-2-pyridyl group, a 5-methylsulfonylamino-2-pyridyl group, a 5-acetylamino-2-pyridyl group, a 5-tert-butoxycarbonylamino-2-pyridyl group, a 5-carbamoyl-2-pyridyl group, a 5-methylcarbamoyl-2-pyridyl group, a 5-dimethylcarbamoyl-2-pyridyl group, a 5-chloro-2-pyridyl group, a 5-fluoro-2-pyridyl group, a 5-hydroxymethyl-2-pyridyl group, a 5-aminomethyl-2-pyridyl group, a 5-methylaminomethyl-2-pyridyl group, a 5-dimethylaminomethyl-2-pyridyl group, a 5-methylsulfonylaminomethyl-2-pyridyl group, a 5-acetylaminomethyl-2-pyridyl group, a 3-pyridyl group, a 6-methyl-3-pyridyl group, a 6-ethyl-3-pyridyl group, a 6-methoxy-3-pyridyl group, a 6-ethoxy-3-pyridyl group;

a 6-methyl-3-pyridazinyl group, a 6-ethyl-3-pyridazinyl group, a 6-methoxy-3-pyridazinyl group, a 6-ethoxy-3-pyridazinyl group, and the like are more preferred.

Among them, a 1-methyl-1H-imidazol-4-yl group, a 1-methyl-1H-pyrazol-3-yl group, a 2-pyridyl group, a 5-methyl-2-pyridyl group, a 5-amino-2-pyridyl group, a 5-methylsulfonylamino-2-pyridyl group, a 5-acetylamino-2-pyridyl group, a 5-tert-butoxycarbonylamino-2-pyridyl group, a 5-chloro-2-pyridyl group, a 5-fluoro-2-pyridyl group, a 5-carbamoyl-2-pyridylgroup, a 5-hydroxymethyl-2-pyridylgroup, a 5-aminomethyl-2-pyridyl group, a 3-pyridyl group, a 6-methyl-3-pyridyl group, a 6-methoxy-3-pyridyl group;

a 6-methyl-3-pyridazinyl group, a 6-methoxy-3-pyridazinyl group, and the like are particularly preferred.

R¹ and R² of the compound represented by Formula (I) each independently represent a lower alkyl group which may be substituted, an alicyclic heterocyclic group which may be substituted, a carbamoyl group which may be substituted, a hydroxyl group, a lower alkoxy group, or an amino group which may be substituted. Alternatively, R¹ and R² may form, together with the nitrogen atom substituted with R¹ and R², a 4- to 7-membered alicyclic heterocyclic group. Here, this 4- to 7-membered alicyclic heterocyclic group may have one nitrogen atom or oxygen atom, in addition to the nitrogen atom indicated in the formula, as the constituent atom. Furthermore, the 4-to 7-membered alicyclic heterocyclic group may be substituted with 1 to 4 identical or different substituent groups or atoms selected from the group consisting of a lower alkyl group which may be substituted, a carbamoyl group which may be substituted, an amino group which may be substituted, a hydroxyl group, a lower alkoxy group, an oxo group, a lower alkanoyl group, a lower alkylsulfonyl group and a halogen atom. These substituents may be substituted, insofar as substitution can be achieved, on the same atom, or may be substituted on different atoms.

The lower alkyl group which may be substituted means the same group as those defined in (1) of the above-mentioned substituents for Ar₁ and Ar₂. Among them, a methyl group, an ethyl group, an isopropyl group, a tert-butyl group, a 2-fluoro-1,1-dimethylethyl group, a neopentyl group, a cyclopropyl group, a 1-methylcyclopropyl group, a cyclopentyl group, and the like may be listed as preferred substituents.

An alicyclic heterocyclic group in an alicyclic heterocyclic group which may be substituted, means a saturated heterocyclic group, and specifically means a pyrrolidino group, a pyrazolidino group, an imidazolidino group, an isoxazolidino group, anisothiazolidinogroup, apiperidinogroup, apiperazino group, a tetrahydropyridazino group, a morpholino group, a thiomorpholino group, a tetrahydropyranyl group, or the like. These alicyclic heterocyclic groups may be substituted with one, or two to three identical or different substituents selected from the group consisting of a lower alkyl group and a lower alkoxy group. The lower alkyl group and lower alkoxy group in this case mean the same groups as those mentioned above.

The lower alkoxy group means, as described above, an alkoxy group containing a lower alkyl group as the constituent.

The carbamoyl group which may be substituted means, as in the case of (3) of the above-mentioned substituents for Ar₁ and Ar₂, an unsubstituted carbamoyl group, or a carbamoyl group substituted with one, or two identical or different lower alkyl groups described above. Specifically, a carbamoyl group, a methylcarbamoyl group, an ethylcarbamoyl group, an n-propylcarbamoyl group, a dimethylcarbamoyl group, a diethylcarbamoyl group, an N-methyl-N-ethylcarbamoyl group, and the like may be mentioned.

The amino group which may be substituted means, as in the case of (5) of the above-mentioned substituent's for Ar₁ and Ar₂, an amino group which may be substituted with one, or two identical or different substituents selected from a lower alkyl group, a lower alkanoyl group, a lower alkylsulfonyl group, a lower alkyloxycarbonyl group, and a carbamoyl group which may be substituted with a lower alkyl group. Specifically, in addition to an unsubstituted amino group, there may be mentioned a methylamino group, an ethylamino group, an n-propylamino group, an isopropylamino group, a cyclopropylamino group, an n-butylamino group, an isobutylamino group, a cyclopentylmethylamino group, a dimethylamino group, a diethylamino group, a di-n-propylamino group, a di-n-butylamino group, an N-methyl-N-ethylamino group, an N-ethyl-N-n-propylamino group, an N-methyl-N-cyclopentylmethylamino group, a formylamino group, an acetylamino group, an n-propionylamino group, an N-methyl-N-acetylamino group, an N-ethyl-N-acetylamino group, a methylsulfonylamino group, an ethylsulfonylamino group, an isopropylsulfonylamino group, an n-butylsulfonylamino group, a cyclopropylsulfonylamino group, a cyclobutanesulfonylamino group, an N-methyl-N-methylsulfonylamino group, an N-ethyl-N-methylsulfonylamino group, a methoxycarbonylamino group, an N-methyl-N-methoxycarbonylamino group, an ethoxycarbonylamino group, an N-methyl-N-ethoxycarbonylamino group, a tert-butoxycarbonylamino group, an N-methyl-N-butoxycarbonylamino group, a methylcarbamoylamino group, an ethylcarbamoylamino group, an n-propylcarbamoylamino group, an isopropylcarbamoylamino group, an n-butylcarbamoylamino group, an isobutylcarbamoylamino group, a dimethylcarbamoylamino group, a diethylcarbamoylamino group, an N-methyl-N-ethylcarbamoylamino group, and the like.

In addition, R¹ and R² may form, together with the nitrogen atom substituted with R¹ and R², a 4- to 7-membered alicyclic heterocyclic group. This 4- to 7-membered alicyclic heterocyclic group may further contain one nitrogen atom or oxygen atom as the constituent, and may be specifically exemplified by an azetidinyl group, a pyrrolidinyl group, a pyrazolidinyl group, a piperidinyl group, a piperazinyl group, a hexahydropyridazinyl group, a hexahydropyrimidinyl group, a pyrazolidinyl group, an imidazolidinyl group, a homopiperazinyl group, a morpholinyl group, or the like. Among them, an azetidinyl group, a pyrrolidinyl group, a pyrazolidinyl group, a piperidinyl group, a piperazinyl group, a hexahydropyrimidinyl group, a pyrazinyl group and a morpholinyl group are particularly preferred.

These alicyclic heterocyclic groups may be further substituted with 1 to 4 identical or different groups or atoms selected from the following substituents of (i) to (ix).
(i) A lower alkyl group which may be substituted: The lower alkyl group which may be substituted means the same group as the (1) lower alkyl group which may be substituted of the above-mentioned substituents for Ar₁ and Ar₂. That is, it indicates a lower alkyl group which may be substituted with one to three groups or atoms selected from the above-mentioned (a) to (g). Furthermore, the lower alkyl group may be substituted with an oxo group alone, or may be substituted with a group or atom selected from (a) to (g) in combination thereof. For the lower alkyl group, a methyl group is particularly preferred. Moreover, the group or atom substituted on the lower alkyl group is preferably a halogen atom or a hydroxyl group. For the lower alkyl group substituted on such alicyclic heterocyclic group, an unsubstituted lower alkyl group, a halogeno-lower alkyl group and a hydroxy-lower alkyl group are preferred. The halogeno-lower alkyl group means the lower alkyl group described above, which is substituted with the above-mentioned halogen atoms. Specifically, a fluoromethyl group, a difluoromethyl group, a trifluoromethyl group, a chloromethyl group, a dichloromethyl group, a trichloromethyl group, and the like may be mentioned, and among them, a methyl group, a fluoromethyl group, a difluoromethyl group or a trifluoromethyl group is preferred, and a fluoromethyl group is particularly preferred. The hydroxy-lower alkyl group means the lower alkyl group described above, which is substituted with a hydroxyl group, and specifically, ahydroxymethylgroup, a 1-hydroxyethyl group, a 2-hydroxyethyl group, a 1-hydroxypropyl group, a 2-hydroxypropyl group, a 3-hydroxypropyl group, and the like may mentioned.

(ii) A carbamoyl group which may be substituted: The carbamoyl group which may be substituted includes the same ones as those given in (3) of the above-mentioned substituents for Ar₁ and Ar₂. Among them, an unsubstituted carbamoyl group, a methylcarbamoyl group, or a dimethylcarbamoyl group is preferred, and an unsubstituted carbamoyl group is particularly preferred.

(iii) An amino group which may be substituted: The amino group which may be substituted includes the same ones as the amino groups listed in (5) of the above-mentioned substituents for Ar₁ and Ar₂. Among them, an unsubstituted amino group, a methylamino group, a dimethylamino group, an ethylamino group, or a diethylamino group is preferred, and an unsubstituted amino group or a dimethylamino group is particularly preferred.

(iv) A hydroxyl group.

(v) A lower alkoxy group: The lower alkoxy group includes the same ones as described above, a methoxy group or an ethoxy group is preferred, and a methoxy group is particularly preferred.

(vi) An oxo group.

(vii) A lower alkanoyl group: The lower alkanoyl group means, asdescrbedabove, a straight-chained orbranched alkanoyl group having 1 to 6 carbon atoms. Specifically, a formyl group, an acetyl group, an n-propionyl group, an n-butyryl group, an isobutyryl group, apivaloylgroup, and the like maybe mentioned, and among them, a formyl group is particularly preferred.

(viii) A lower alkylsulfonyl group: The lower alkylsulfonyl group includes the same ones as described above. That is, the lower alkylsulfonyl group means a sulfonyl group substituted with the above-mentioned lower alkyl group, and specifically, a methylsulfonyl group, an ethylsulfonyl group, an n-propylsulfonyl group, an isopropylsulfonyl group, an n-butylsulfonyl group, an isobutylsulfonyl group, a tert-butylsulfonyl group, an n-pentylsulfonyl group, an isopentylsulfonyl group, a cyclopropylsulfonyl group, a cyclohexylsulfonyl group, and the like may be mentioned. Among them, a methylsulfonyl group, an ethylsulfonyl group or an n-propylsulfonyl group is preferred.

(ix) A halogen atom: The same ones as described above may be mentioned. Among them, fluorine or chlorine is preferred.

The alicyclic heterocyclic group may be substituted with one or 2 to 4 identical or different groups selected from above (i) to (iX) at identical or different element of the alicyclic heterocyclic group, insofar as substitution can be achieved.

Specific examples of the 4- to 7-membered alicyclic heterocyclic group formed by R¹ and R² group together with the nitrogen atom on which they substitute, include an azetidin-1-yl group, a 3-oxoazetidin-1-yl group, a 2-oxoazetidin-1-yl group, a 3-aminoazetidin-1-yl group, a 3-methylaminoazetidin-1-yl group, a 3-dimethylaminoazetidin-1-yl group, a 2-methylazetidin-1-yl group, a 3-methylazetidin-1-yl group, a 2,2-dimethylazetidin-1-yl group, a 3,3-dimethylazetidin-1-yl group, a 2,2-dimethyl-3-dimethylaminoazetidin-1-yl group, a 2-hydroxymethylazetidin-1-yl group, a 3-hydroxymethylazetidin-1-yl group, a 2-fluoromethylazetidin-1-yl group, a 3-fluoromethylazetidin-1-yl group, a 3-methoxyazetidin-1-yl group, a 2-carbamoylazetidin-1-yl group, a 2-methylcarbamoylazetidin-1-yl group, a 2-dimethylcarbamoylazetidin-1-yl group, a 3-carbamoylazetidin-1-yl group, a 3-methylcarbamoylazetidin-1-yl group, a 3-dimethylcarbamoylazetidin-1-yl group, a 3,3-difluoroazetidin-1-yl group;

a pyrrolidino group, a 2-methylpyrrolidino group, a 2-ethylpyrrolidino group, a 2-aminomethylpyrrolidino group, a 2-methylaminomethylpyrrolidino group, a 2-dimethylaminomethylpyrrolidino group, a 2-hydroxymethylpyrrolidino group, a 2-methoxymethylpyrrolidino group, a 2-fluoromethylpyrrolidino group, a 2-trifluoromethylpyrrolidino group, a 2,2-dimethylpyrrolidino group,a2,3-dimethylpyrrolidino group, a 2,4-dimethylpyrrolidino group, a 2,5-dimethylpyrrolidino group, a 2-carbamoylpyrrolidino group, a 2-methylcarbamoylpyrrolidino group, a 2-dimethylcarbamoylpyrrolidino group, a 2-methoxypyrrolidino group, a 2-oxopyrrolidino group, a 2,5-dioxopyrrolidino group, a 2-methoxymethyl-5-methylpyrrolidino group, a 2,2-dimethyl-3-dimethylaminopyrrolidino group, a 3-methylpyrrolidino group, a 3-methoxymethylpyrrolidino group, a 3-fluoromethylpyrrolidino group, a 3-trifluoromethylpyrrolidino group, a 3-aminopyrrolidino group, a 3-methylaminopyrrolidino group, a 3-dimethylaminopyrrolidino group, a 3-oxopyrrolidino group, a 3,3-dimethylpyrrolidino group, a 3-hydroxymethylpyrrolidino group, a 3-carbamoylpyrrolidino group, a 3-methylcarbamoylpyrrolidino group, a 3-dimethylcarbamoylpyrrolidino group, a 3-methoxypyrrolidino group, a 3-fluoropyrrolidino group, a 3,3-difluoropyrrolidino group;

an imidazolidin-1-yl group, a 3-methylimidazolidin-1-yl group, a2-oxoimidazolidin-1-ylgroup, a 4-oxoimidazolidin-1-yl group, a 3-methyl-2-oxoimidazolidin-1-yl group, a 3-methyl-4-oxoimidazolidin-1-yl group, a 2,2-dimethylimidazolin-1-yl group; a pyrazolidin-1-yl group, a 2-methylpyrazolidin-1-yl group, a 3-oxopyrazolidin-1-yl group, a 3,5-dioxopyrazolidin-1-yl group, a 2-formylpyrazolidin-1-yl group, a 2-methylsulfonylpyrazolidin-1-yl group;

a piperidino group, a 2-oxopiperidino group, a 3-oxopiperidino group, a 4-oxopiperidino group, a 2-hydroxypiperidino group, a 3-hydroxypiperidino group, a 4-hydroxypiperidino group, a 2-methoxypiperidino group, a 3-methoxypiperidino group, a 4-methoxypiperidino group, a 3-aminopiperidino group, a 4-aminopiperidino group, a 3-methylaminopiperidino group, a 4-methylaminopiperidino group, a 3-dimethylaminopiperidino group, a 4-dimethylaminopiperidino group, a 2-methylpiperidino group, a 3-methylpiperidino group, a 4-methylpiperidino group, a 2, 2-dimethylpiperidino group, a 3,3-dimethylpiperidino group, a 4,4-dimethylpiperidino group, a 3-fluoropiperidino group, a 4-fluoropiperidino group, a 4-chloropiperidino group, a 3,3-difluoropiperidino group, a 4,4-difluoropiperidino group, a 3,3-dichloropiperidino group, a 4,4-dichloropiperidino group, a 4-fluoromethylpiperidino group, a 2-hydroxymethylpiperidino group, a 3-hydroxymethylpiperidino group, a 4-hydroxymethylpiperidino group,a2-carbamoylpiperidino group, a 3-carbamoylpiperidino group, a 4-carbamoylpiperidino group, a 2-methylcarbamoylpiperidino group, a 3-methylcarbamoylpiperidino group, a 4-methylcarbamoylpiperidino group, a 2-dimethylcarbamoylpiperidino group, a 3-dimethylcarbamoylpiperidino group, a 4-dimethylcarbamoylpiperidino group, a 2-methoxymethylpiperidino group, a 3-methoxymethylpiperidino group, a 4-methoxymethylpiperidino group, a 2-aminomethylpiperidino group, a 3-aminomethylpiperidino group, a 4-aminomethylpiperidino group, a 2-methylaminomethylpiperidino group, a 3-methylaminomethylpiperidino group, a 4-methylaminomethylpiperidino group, a 2-dimethylaminomethylpiperidino group, a 3-dimethylaminomethylpiperidino group, a 4-dimethylaminomethylpiperidino group, a 2-aminoethylpiperidino group, a 3-aminoethylpiperidino group, a4-aminoethylpiperidino group,a2-methylaminoethylpiperidino group, a 3-methylaminoethylpiperidino group, a 4-methylaminoethylpiperidino group, a 2-dimethylaminoethylpiperidino group, a 3-dimethylaminoethylpiperidino group, a 4-dimethylaminoethylpiperidino group;

a piperazino group, a 2-oxopiperazino group, a 3-oxopiperazino group, a 2-oxo-4-methylpiperazino group, a 3-oxo-4-methylpiperazino group, a 4-formylpiperazino group, a 2,3-dioxopiperazino group, a 3,5-dioxopiperazino group, a 2,6-dioxopiperazino group, a 2,3-dioxo-4-methylpiperazino group, a 3,5-dioxo-4-methylpiperazino group, a 2,6-dioxo-4-methylpiperazino group, a 2-methylpiperazino group, a 3-methylpiperazino group, a 4-methylpiperazino group, a 2-ethylpiperazino group, a 3-ethylpiperazino group, a 4-ethylpiperazino group, a 2-isopropylpiperazino group, a 3-isopropylpiperazino group, a 4-isopropylpiperazino group, a 2-cyclopropylpiperazino group,a3-cyclopropylpiperazino group, a 4-cyclopropylpiperazino group, a 4-cyclobutylpiperazino group, a 2,2-dimethylpiperazino group, a 3,3-dimethylpiperazino group, a 2,3-dimethylpiperazino group, a 2, 4-dimethylpiperazino group, a 3,4-dimethylpiperazino group, a 3, 5-dimethylpiperazino group, a 2, 6-dimethylpiperazino group, a 2-ethyl-4-methylpiperazino group, a 3-ethyl-4-methylpiperazino group, a 2-isopropyl-4-methylpiperazino group, a 3-isopropyl-4-methylpiperazino group, a 2-cyclopropyl-4-methylpiperazino group, a 3-cyclopropyl-4-methylpiperazino group, a 1,2,6-trimethylpiperazino group, a 3,4,5-trimethylpiperazino group, a 2,2,4-trimethylpiperazino group, a 3,3,4-trimethylpiperazino group, a 3,3,4-trimethyl-5-oxopiperazino group, a 2,2,4-trimethyl-3-oxopiperazino group, a 4-acetylpiperazino group, a 2-hydroxymethylpiperazino group, a 3-hydroxymethylpiperazino group, a 4-methoxypiperazino group, a 2-methoxymethylpiperazino group, a 3-methoxymethylpiperazino group, a 2-hydroxyethylpiperazino group, a 3-hydroxyethylpiperazino group, a 4-hydroxyethylpiperazino group, a 2-hydroxymethyl-4-methylpiperazino group, a 3-hydroxymethyl-4-methylpiperazino group, a 2-methoxymethyl-4-methylpiperazino group, a 3-methoxymethyl-4-methylpiperazino group, a 2-hydroxyethyl-4-methylpiperazino group, a 3-hydroxyethyl-4-methylpiperazino group, a 2-methoxyethyl-4-methylpiperazino group, a 3-methoxyethyl-4-methylpiperazino group, a 2-carbamoylpiperazino group, a 3-carbamoylpiperazino group, a 4-carbamoylpiperazino group, a 2-methylcarbamoylpiperazino group, a 3-methylcarbamoylpiperazino group, a 4-methylcarbamoylpiperazino group, a 2-dimethylcarbamoylpiperazino group, a 3-dimethylcarbamoylpiperazino group, a 4-dimethylcarbamoylpiperazino group, a 2-carbamoylmethylpiperazino group, a 3-carbamoylmethylpiperazino group, a 4-carbamoylmethylpiperazino group, a 2-methylcarbamoylmethylpiperazino group, a 3-methylcarbamoylmethylpiperazino group, a 4-methylcarbamoylpiperazino group, a 2-dimethylcarbamoylmethylpiperazino group, a 3-dimethylcarbamoylmethylpiperazino group, a 2-carbamoyl-4-methylpiperazino group, a 3-carbamoyl-4-methylpiperazino group, a4-carbamoylpiperazino group, a 2-methylcarbamoyl-4-methylpiperazino group, a 3-methylcarbamoyl-4-methylpiperazino group, a 4-methylcarbamoylpiperazino group, a 2-dimethylcarbamoyl-4-methylpiperazino group, a 3-dimethylcarbamoyl-4-methylpiperazino group, a 4-dimethylcarbamoylpiperazino group, a 2-carbamoylmethyl-4-methylpiperazino group, a 3-carbamoylmethyl-4-methylpiperazino group, a 4-carbamoylmethylpiperazino group, a 2-methylcarbamoylmethyl-4-methylpiperazino group, a 3-methylcarbamoylmethyl-4-methylpiperazino group, a 4-methylcarbamoylpiperazino group, a 2-dimethylcarbamoylmethyl-4-methylpiperazino group, a 3-dimethylcarbamoylmethyl-4-methylpiperazino group, a 2-aminomethylpiperazino group, a 3-aminomethylpiperazino group, a 2-methylaminomethylpiperazino group, a 3-methylaminomethylpiperazino group, a 2-dimethylaminomethylpiperazino group, a 3-dimethylaminomethylpiperazino group, a 2-aminoethylpiperazino group, a 3-aminoethylpiperazino group, a 4-aminoethylpiperazino group, a 2-methylaminoethylpiperazino group, a 3-methylaminoethylpiperazino group, a 4-methylaminoethylpiperazino group, a 2-dimethylaminoethylpiperazino group, a 3-dimethylaminoethylpiperazino group, a 4-dimethylaminoethylpiperazino group, a 2-aminomethyl-4-methylpiperazino group, a 3-aminomethyl-4-methylpiperazino group, a 2-methylaminomethyl-4-methylpiperazino group, a 3-methylaminomethyl-4-methylpiperazino group, a 2-dimethylaminomethyl-4-methylpiperazino group, a 3-dimethylaminomethyl-4-methylpiperazino group, a 2-aminoethyl-4-methylpiperazino group, a 3-aminoethyl-4-methylpiperazino group, a 2-methylaminoethyl-4-methylpiperazino group, a 3-methylaminoethyl-4-methylpiperazino group, a 2-dimethylaminoethyl-4-methylpiperazino group, a 3-dimethylaminoethyl-4-methylpiperazino group, a 4-methylsulfonylpiperazino group;

a morpholino group, a 2-methylmorpholino group, a 3-methylmorpholino group, a 2-ethylmorpholino group, a 3-ethylmorpholino group, a 2,2-dimethylmorpholino group, a 3,3-dimethylmorpholino group, a 2-hydroxymethylmorpholino group, a 3-hydroxymethylmorpholino group, a 2-methoxymethylmorpholino group, a 3-methoxymethylmorpholino group, a 2-hydroxyethylmorpholino group, a 3-hydroxyethylmorpholino group, a 2-methoxyethylmorpholino group, a 3-methoxyethylmorpholino group, a 2-carbamoylmorpholino group, a 3-carbamoylmorpholino group, a 2-methylcarbamoylmorpholino group, a 3-methylcarbamoylmorpholino group, a 2-dimethylcarbamoylmorpholino group, a 3-dimethylcarbamoylmorpholino group, a 2-carbamoylmethylmorpholino group, a 3-carbamoylmethylmorpholino group, a 2-methylcarbamoylmethylmorpholino group, a 3-methylcarbamoylmethylmorpholino group, a 2-dimethylcarbamoylmethylmorpholino group, a 3-dimethylcarbamoylmethylmorpholino group, a 2-carbamoylethylmorpholino group, a 3-carbamoylethylmorpholino group, a 2-methylcarbamoylethylmorpholino group, a 3-methylcarbamoylethylmorpholino group, a 2-dimethylcarbamoylethylmorpholino group, a 3-dimethylcarbamoylethylmorpholino group, a 2-aminomethylmorpholiho group, a 3-aminomethylmorpholino group, a 2-methylaminomethylmorpholino group, a 3-methylaminomethylmorpholino group, a 2-dimethylaminomethylmorpholino group, a 3-dimethylaminomethylmorpholino group, a 2-aminoethylmorpholino group, a 3-aminoethylmorpholino group, a 2-methylaminoethylmorpholino group, a 3-methylaminoethylmorpholino group, a 2-dimethylaminoethylmorpholino group, a 3-dimethylaminoethylmorpholino group;

a hexahydropyridazin-1-yl group, a 2-formylhexahydropyridazin-1-yl group, a 2-acetylhexahydropyridazin-1-yl group, a 3-oxohexahydropyridazin-1-yl group, a 6-oxohexahydropyridazin-1-yl group, a 4-aminohexahydropyridazin-1-yl group, a 4-methylaminohexahydropyridazin-1-yl group, a 4-dimethylaminohexahydropyridazin-1-yl group, a 2-methylhexahydropyridazin-1-yl group, a 3-methylhexahydropyridazin-1-yl group, a 4-methylhexahydropyridazin-1-yl group, a 2,3-dimethylhexahydropyridazin-1-yl group, a 3,3-dimethylhexahydropyridazin-1-yl group, a 4,4-dimethylhexahydropyridazin-1-yl group, a 3-hydroxymethylhexahydropyridazin-1-yl group, a 4-hydroxymethylhexahydropyridazin-1-yl group, a 5-hydroxymethylhexahydropyridazin-1-yl group, a 6-hydroxymethylhexahydropyridazin-1-yl group, a 2-carbamoylhexahydropyridazin-1-yl group, a 3-carbamoylhexahydropyridazin-1-yl group, a 4-carbamoylhexahydropyridazin-1-yl group, a 5-carbamoylhexahydropyridazin-1-yl group, a 6-carbamoylhexahydropyridazin-1-yl group, a 2-methylcarbamoylhexahydropyridazin-1-yl group, a 3-methylcarbamoylhexahydropyridazin-1-yl group, a 4-methylcarbamoylhexahydropyridazin-1-yl group, a 5-methylcarbamoylhexahydropyridazin-1-yl group, a 6-methylcarbamoylhexahydropyridazin-1-yl group, a 2-dimethylcarbamoylhexahydropyridazin-1-yl group, a 3-dimethylcarbamoylhexahydropyridazin-1-yl group, a 4-dimethylcarbamoylhexahydropyridazin-1-yl group, a 5-dimethylcarbamoylhexahydropyridazin-1-yl group, a 6-dimethylcarbamoylhexahydropyridazin-1-yl group, a 3-methoxymethylhexahydropyridazin-1-yl group, a 4-methoxymethylhexahydropyridazin-1-yl group, a 5-methoxymethylhexahydropyridazin-1-yl group, a 6-methoxymethylhexahydropyridazin-1-yl group, a 2-aminoethylhexahydropyridazin-1-yl group, a 3-aminoethylhexahydropyridazin-1-yl group, a 4-aminoethylhexahydropyridazin-1-yl group, a 5-aminoethylhexahydropyridazin-1-yl group, a 6-aminoethylhexahydropyridazin-1-yl group, a 2-methylaminoethylhexahydropyridazin-1-yl group, a 3-methylaminoethylhexahydropyridazin-1-yl group, a 4-methylaminoethylhexahydropyridazin-1-yl group, a 5-methylaminoethylhexahydropyridazin-1-yl group, a 6-methylaminoethylhexahydropyridazin-1-yl group, a 3-aminomethylhexahydropyridazin-1-yl group, a 4-aminomethylhexahydropyridazin-1-yl group, a 5-aminomethylhexahydropyridazin-1-yl group, a 6-aminomethylhexahydropyridazin-1-yl group, a 3-methylaminomethylhexahydropyridazin-1-yl group, a 4-methylaminomethylhexahydropyridazin-1-yl group, a 5-methylaminomethylhexahydropyridazin-1-yl group, a 6-methylaminomethylhexahydropyridazin-1-yl group, a 3-dimethylaminomethylhexahydropyridazin-1-yl group, a 4-dimethylaminomethylhexahydropyridazin-1-yl group, a 5-dimethylaminomethylhexahydropyridazin-1-yl group, a 6-dimethylaminomethylhexahydropyridazin-1-yl group, a 2-dimethylaminoethylhexahydropyridazin-1-yl group, a 3-dimethylaminoethylhexahydropyridazin-1-yl group, a 4-dimethylaminoethylhexahydropyridazin-1-yl group, a 5-dimethylaminoethylhexahydropyridazin-1-yl group, a 6-dimethylaminoethylhexahydropyridazin-1-yl group;

a hexahydropyrimidin-1-yl group, a 2-oxohexahydropyrimidin-1-yl group, a 4-oxohexahydropyrimidin-1-yl group, a 5-oxohexahydropyrimidin-1-yl group, a 6-oxohexahydropyrimidin-1-yl group, a 2-methylhexahydropyrimidin-1-yl group, a 3-methylhexahydropyrimidin-1-yl group, a 4-methylhexahydropyrimidin-1-yl group, a 4-methylhexahydropyrimidin-1-yl group, a 2,2-dimethylhexahydropyrimidin-1-yl group, a 4,4-dimethylhexahydropyrimidin-1-yl group, a 5,5-dimethylhexahydropyrimidin-1-yl group, a 6,6-dimethylhexahydropyrimidin-1-yl group, a 2-hydroxymethylhexahydropyrimidin-1-yl group, a 4-hydroxymethylhexahydropyrimidin-1-yl group, a 5-hydroxymethylhexahydropyrimidin-1-yl group, a 6-hydroxymethylhexahydropyrimidin-1-yl group, a 2-carbamoylhexahydropyrimidin-1-yl group, a 3-carbamoylhexahydropyrimidin-1-yl group, a 4-carbamoylhexahydropyrimidin-1-yl group, a 5-carbamoylhexahydropyrimidin-1-yl group, a 6-carbamoylhexahydropyrimidin-1-yl group, a 2-methylcarbamoylhexahydropyrimidin-1-yl group, a 3-methylcarbamoylhexahydropyrimidin-1-yl group, a 4-methylcarbamoylhexahydropyrimidin-1-yl group, a 5-methylcarbamoylhexahydropyrimidin-1-yl group, a 6-methylcarbamoylhexahydropyrimidin-1-yl group, a 2-dimethylcarbamoylhexahydropyrimidin-1-yl group, a 3-dimethylcarbamoylhexahydropyrimidin-1-yl group, a 4-dimethylcarbamoylhexahydropyrimidin-1-yl group, a 5-dimethylcarbamoylhexahydropyrimidin-1-yl group, a 6-dimethylcarbamoylhexahydropyrimidin-1-yl group, a 3-methoxymethylhexahydropyrimidin-1-yl group, a 4-methoxymethylhexahydropyrimidin-1-yl group, a 5-methoxymethylhexahydropyrimidin-1-yl group, a 6-methoxymethylhexahydropyrimidin-1-yl group, a 2-aminoethylhexahydropyrimidin-1-yl group, a 3-aminoethylhexahydropyrimidin-1-yl group, a 4-aminoethylhexahydropyrimidin-1-yl group, a 5-aminoethylhexahydropyrimidin-1-yl group, a 6-aminoethylhexahydropyrimidin-1-yl group, a 2-methylaminoethylhexahydropyrimidin-1-yl group, a 3-methylaminoethylhexahydropyrimidin-1-yl group, a 4-methylaminoethylhexahydropyrimidin-1-yl group, a 5-methylaminoethylhexahydropyrimidin-1-yl group, a 6-methylaminoethylhexahydropyrimidin-1-yl group, a 2-dimethylaminoethylhexahydropyrimidin-1-yl group, a 3-dimethylaminoethylhexahydropyrimidin-1-yl group, a 4-dimethylaminoethylhexahydropyrimidin-1-yl group, a 5-dimethylaminoethylhexahydropyrimidin-1-yl group, a 6-dimethylaminoethylhexahydropyrimidin-1-yl group;

a homopiperazino group, a 2-oxohomopiperazino group, a 3-oxohomopiperazino group, a 5-oxohomopiperazino group, a 6-oxohomopiperazino group, a 7-oxohomopiperazino group, a 2-oxo-4-methylhomopiperazino group, a 3-oxo-4-methylhomopiperazino group, a 5-oxo-4-methylhomopiperazino group, a 6-oxo-4-methylhomopiperazino group, a 7-oxo-4-methylhomopiperazino group,a2,3-dioxohomopiperazino group, a 2,7-dioxohomopiperazino group, a 3,5-dioxohomopiperazino group,a3,7-dioxohomopiperazino group, a 2,3-dioxo-4-methylhomopiperazino group, a 2,7-dioxo-4-methylhomopiperazino group, a 3,5-dioxo-4-methylhomopiperazino group, a 3,7-dioxo-4-methylhomopiperazino group, a 2-methylhomopiperazino group, a 3-methylhomopiperazino group, a 4-methylhomopiperazino group, a 5-methylhomopiperazino group, a 6-methylhomopiperazino group, a 7-methylhomopiperazino group, a 2-ethylhomopiperazino group, a 3-ethylhomopiperazino group, a 4-ethylhomopiperazino group, a 5-ethylhomopiperazino group, a 6-ethylhomopiperazino group, a 7-ethylhomopiperazino group, a 4-cyclopropylhomopiperazino group, a 2,2-dimethylhomopiperazino group, a 3,3-dimethylhomopiperazino group, a 5,5-dimethylhomopiperazino group, a 6,6-dimethylhomopiperazino group, a 7,7-dimethylhomopiperazino group, a 2,3-dimethylhomopiperazino group, a 2,4-dimethylhomopiperazino group, a 3,4-dimethylhomopiperazino group, a 3,5-dimethylhomopiperazino group, a 3,4,5-trimethylhomopiperazino group, a 2-hydroxymethylhomopiperazino group, a 3-hydroxymethylhomopiperazino group, a 5-hydroxymethylhomopiperazino group, a 6-hydroxymethylhomopiperazino group, a 7-hydroxymethylhomopiperazino group, a 2-hydroxymethyl-4-methylhomopiperazino group, a 3-hydroxymethyl-4-methylhomopiperazino group, a 5-hydroxymethyl-4-methylhomopiperazino group, a 6-hydroxymethyl-4-methylhomopiperazino group, a 7-hydroxymethyl-4-methylhomopiperazino group, a 2-methoxymethylhomopiperazino group, a 3-methoxymethylhomopiperazino group, a 5-methoxymethylhomopiperazino group, a 6-methoxymethylhomopiperazino group, a 7-methoxymethylhomopiperazino group, a 2-methoxymethyl-4-methylhomopiperazino group, a 3-methoxymethyl-4-methylhomopiperazino group, a 5-methoxymethyl-4-methylhomopiperazino group, a 6-methoxymethyl-4-methylhomopiperazino group, a 7-methoxymethyl-4-methylhomopiperazino group, a 2-hydroxyethylhomopiperazino group, a 3-hydroxyethylhomopiperazino group, a 4-hydroxyethylhomopiperazino group, a 5-hydroxyethylhomopiperazino group, a 6-hydroxyethylhomopiperazino group, a 7-hydroxyethylhomopiperazino group, a 2-hydroxyethyl-4-methylhomopiperazino group, a 3-hydroxyethyl-4-methylhomopiperazino group, a 5-hydroxyethyl-4-methylhomopiperazino group, a 6-hydroxyethyl-4-methylhomopiperazino group, a 7-hydroxyethyl-4-methylhomopiperazino group, a 2-methoxyethylhomopiperazino group, a 3-methoxyethylhomopiperazino group, a 4-methoxyethylhomopiperazino group, a 5-methoxyethylhomopiperazino group, a 6-methoxyethylhomopiperazino group, a 7-methoxyethylhomopiperazino group, a 2-methoxyethyl-4-methylhomopiperazino group, a 3-methoxyethyl-4-methylhomopiperazino group, a 5-methoxyethyl-4-methylhomopiperazino group, a 6-methoxyethyl-4-methylhomopiperazino group, a 7-methoxyethyl-4-methylhomopiperazino group, a 2-carbamoylhomopiperazino group, a 3-carbamoylhomopiperazino group, a 4-carbamoylhomopiperazino group, a 5-carbamoylhomopiperazino group, a 6-carbamoylhomopiperazino group, a 7-carbamoylhomopiperazino group, a 2-carbamoyl-4-methylhomopiperazino group, a 3-carbamoyl-4-methylhomopiperazino group, a 4-carbamoylhomopiperazino group, a 5-carbamoyl-4-methylhomopiperazino group, a 6-carbamoyl-4-methylhomopiperazino group, a 7-carbamoyl-4-methylhomopiperazino group, a 2-methylcarbamoylhomopiperazino group, a 3-methylcarbamoylhomopiperazino group, a 4-methylcarbamoylhomopiperazino group, a 5-methylcarbamoylhomopiperazino group, a 6-methylcarbamoylhomopiperazino group, a 7-methylcarbamoylhomopiperazino group, a 2-methylcarbamoyl-4-methylhomopiperazino group, a 3-methylcarbamoyl-4-methylhomopiperazino group, a 5-methylcarbamoyl-4-methylhomopiperazino group, a 6-methylcarbamoyl-4-methylhomopiperazino group, a 7-methylcarbamoyl-4-methylhomopiperazino group, a 2-dimethylcarbamoylhomopiperazino group, a 3-dimethylcarbamoylhomopiperazino group, a 4-dimethylcarbamoylhomopiperazino group, a 5-dimethylcarbamoylhomopiperazino group, a 6-dimethylcarbamoylhomopiperazino group, a 7-dimethylcarbamoylhomopiperazino group, 2-dimethylcarbamoyl-4-methylhomopiperazino group, a 3-dimethylcarbamoyl-4-methylhomopiperazino group, a 5-dimethylcarbamoyl-4-methylhomopiperazino group, a 6-dimethylcarbamoyl-4-methylhomopiperazino group, a 7-dimethylcarbamoyl-4-methylhomopiperazino group, a 2-carbamoylmethylhomopiperazino group, a 3-carbamoylmethylhomopiperazino group, a 4-carbamoylmethylhomopiperazino group, a 5-carbamoylmethylhomopiperazino group, a 6-carbamoylmethylhomopiperazino group, a 7-carbamoylmethylhomopiperazino group, a 2-carbamoylmethyl-4-methylhomopiperazino group, a 3-carbamoylmethyl-4-methylhomopiperazino group, a 5-carbamoylmethyl-4-methylhomopiperazino group, a 6-carbamoylmethyl-4-methylhomopiperazino group, a 7-carbamoylmethyl-4-methylhomopiperazino group, a 2-methylcarbamoylmethylhomopiperazino group, a 3-methylcarbamoylmethylhomopiperazino group, a 4-methylcarbamoylhomopiperazino group, a 5-methylcarbamoylhomopiperazino group, a 6-methylcarbamoylhomopiperazino group, a 7-methylcarbamoylhomopiperazino group, a 2-methylcarbamoylmethyl-4-methylhomopiperazino group, a 3-methylcarbamoylmethyl-4-methylhomopiperazino group, a 5-methylcarbamoyl-4-methylhomopiperazino group, a 6-methylcarbamoyl-4-methylhomopiperazino group, a 7-methylcarbamoyl-4-methylhomopiperazino group, a 2-dimethylcarbamoylmethylhomopiperazino group, a 3-dimethylcarbamoylmethylhomopiperazino group, a 4-dimethylcarbamoylmethylhomopiperazino group, a 5-dimethylcarbamoylmethylhomopiperazino group, a 6-dimethylcarbamoylmethylhomopiperazino group, a 7-dimethylcarbamoylmethylhomopiperazino group, a 2-dimethylcarbamoylmethyl-4-methylhomopiperazino group, a 3-dimethylcarbamoylmethyl-4-methylhomopiperazino group, a 5-dimethylcarbamoylmethyl-4-methylhomopiperazino group, a 6-dimethylcarbamoylmethyl-4-methylhomopiperazino group, a 7-dimethylcarbamoylmethyl-4-methylhomopiperazino group, a 2-aminomethylhomopiperazino group, a 3-aminomethylhomopiperazino group, a 5-aminomethylhomopiperazino group, a 6-aminomethylhomopiperazino group, a 7-aminomethylhomopiperazino group, a 2-aminomethyl-4-methylhomopiperazino group, a 3-aminomethyl-4-methylhomopiperazino group, a 5-aminomethyl-4-methylhomopiperazino group, a 6-aminomethyl-4-methylhomopiperazino group, a 7-aminomethyl-4-methylhomopiperazino group, a 2-methylaminomethylhomopiperazino group, a 3-methylaminomethylhomopiperazino group, a 4-methylaminomethylhomopiperazino group, a 5-methylaminomethylhomopiperazino group, a 6-methylaminomethylhomopiperazino group, a 7-methylaminomethylhomopiperazino group, a 2-methylaminomethyl-4-methylhomopiperazino group, a 3-methylaminomethyl-4-methylhomopiperazino group, a 5-methylaminomethyl-4-methylhomopiperazino group, a 6-methylaminomethyl-4-methylhomopiperazino group, a 7-methylaminomethyl-4-methylhomopiperazino group, a 2-dimethylaminomethylhomopiperazino group, a 3-dimethylaminomethylhomopiperazino group, a 4-dimethylaminomethylhomopiperazino group, a 5-dimethylaminomethylhomopiperazino group, a 6-dimethylaminomethylhomopiperazino group, a 7-dimethylaminomethylhomopiperazino group, a 2-dimethylaminomethyl-4-methylhomopiperazino group, a 3-dimethylaminomethyl-4-methylhomopiperazino group, a 5-dimethylaminomethyl-4-methylhomopiperazino group, a 6-dimethylaminomethyl-4-methylhomopiperazino group, a 7-dimethylaminomethyl-4-methylhomopiperazino group, a 2-aminoethylhomopiperazino group, a 3-aminoethylhomopiperazino group, a 4-aminoethylhomopiperazino group, a 5-aminoethylhomopiperazino group, a 6-aminoethylhomopiperazino group, a 7-aminoethylhomopiperazino group, a 2-aminoethyl-4-methylhomopiperazino group, a 3-aminoethyl-4-methylhomopiperazino group, a 5-aminoethyl-4-methylhomopiperazino group, a 6-aminoethyl-4-methylhomopiperazino group, a 7-aminoethyl-4-methylhomopiperazino group, a 2-methylaminoethylhomopiperazino group, a 3-methylaminoethylhomopiperazino group, a 4-methylaminoethylhomopiperazino group, a 5-methylaminoethylhomopiperazino group, a 6-methylaminoethylhomopiperazino group, a 7-methylaminoethylhomopiperazino group, a 2-methylaminoethyl-4-methylhomopiperazino group, a 3-methylaminoethyl-4-methylhomopiperazino group, a 5-methylaminoethyl-4-methylhomopiperazino group, a 6-methylaminoethyl-4-methylhomopiperazino group, a 7-methylaminoethyl-4-methylhomopiperazino group, a 2-dimethylaminoethylhomopiperazino group, a 3-dimethylaminoethylhomopiperazino group, a 4-dimethylaminoethylhomopiperazino group, a 5-dimethylaminoethylhomopiperazino group, a 6-dimethylaminoethylhomopiperazino group, a 7-dimethylaminoethylhomopiperazino group, a 2-dimethylaminoethyl-4-methylhomopiperazino group, a 3-dimethylaminoethyl-4-methylhomopiperazino group, a 5-dimethylaminoethyl-4-methylhomopiperazino group, a 6-dimethylaminoethyl-4-methylhomopiperazino group, a 7-dimethylaminoethyl-4-methylhomopiperazino group, a 4-methanesulfonylhomopiperazino group, a 4-methanesulfonylaminohomopiperazino group, a 4-(azetidin-1-yl)-homopiperazino group, a 4-pyrrolidinohomopiperazino group, a 4-piperidinohomopiperazino group;

a 1,4-oxazepan-4-yl group, and the like.

Among them, the following are more preferred. An azetidin-1-yl group, a 3-dimethylaminoazetidin-1-yl group, a 2-methylazetidin-1-yl group, a 3-methylazetidin-1-yl group, a 2,2-dimethylazetidin-1-yl group, a 3,3-dimethylazetidin-1-yl group, a 2,2-dimethyl-3-dimethylaminoazetidin-1-yl group, a 2-hydroxymethylazetidin-1-yl group, a 3-hydroxymethylazetidin-1-yl group, a 2-carbamoylazetidin-1-yl group, a 2-methylcarbamoylazetidin-1-yl group, a 2-dimethylcarbamoylazetidin-1-yl group, a 3,3-difluoroazetidin-1-yl group;

a pyrrolidino group, a 2-methylpyrrolidino group, a 2-aminomethylpyrrolidino group, a 2-hydroxymethylpyrrolidino group, a 2-methoxymethylpyrrolidino group, a 2-fluoromethylpyrrolidino group, a 2-trifluoromethylpyrrolidino group, a 2,2-dimethylpyrrolidino group, a 2,5-dimethylpyrrolidino group, a 2-carbamoylpyrrolidino group, a 2-methoxypyrrolidino group, a 2-oxopyrrolidino group, a 2-methoxymethyl-5-methylpyrrolidino group, a 3-methylpyrrolidino group, a 3-methoxymethylpyrrolidino group, a3-fluoromethylpyrrolidino group, a 3-trifluoromethylpyrrolidino group, a 3-aminopyrrolidino group, a 3,3-dimethylpyrrolidino group, a 3-hydroxymethylpyrrolidino group, a 3-carbamoylpyrrolidino group, a 3-methoxypyrrolidino group, a 3-fluoropyrrolidino group, a 3,3-difluoropyrrolidino group;

an imidazolidin-1-yl group, a 3-methylimidazolidin-1-yl group, a2-oxoimidazolidin-1-ylgroup, a4-oxoimidazolidin-1-yl group, a 3-methyl-2-oxoimidazolidin-1-yl group, a 3-methyl-4-oxoimidazolidin-1-yl group, a 2,2-dimethylimidazolin-1-yl group;

a pyrazolidin-1-yl group, a 2-methylpyrazolidin-1-yl group, a 3-oxopyrazolidin-1-ylgroup, a 2-formylpyrazolidin-1-yl group, a 2-methylsulfonylpyrazolidin-1-yl group;

a piperidino group, a 2-oxopiperidino group, a 3-oxopiperidino group, a 4-oxopiperidino group, a 2-hydroxymethylpiperidino group, a 3-hydroxymethylpiperidino group, a 4-hydroxymethylpiperidino group, a 2-methoxypiperidino group, a 3-methoxypiperidino group, a 4-methoxypiperidino group, a 2-methylpiperidino group, a 3-methylpiperidino group, a 4-methylpiperidino group, a 2,2-dimethylpiperidino group, a 3,3-dimethylpiperidino group, a 4,4-dimethylpiperidino group, a 3-fluoropiperidino group, a 4-fluoropiperidino group, a 4-chloropiperidino group, a 3,3-difluoropiperidino group, a 4,4-difluoropiperidinogroup, a2-fluoromethylpiperidinogroup, a 3-fluoromethylpiperidino group, a 4-fluoromethylpiperidino group, a 3,3-dichloropiperidino group, a 4,4-dichloropiperidino group, a 2-hydroxymethylpiperidino group, a 2-carbamoylpiperidino group, a 2-methylcarbamoylpiperidino group, a 2-dimethylcarbamoylpiperidino group, a 2-methoxymethylpiperidino group, a 4-methyl-4-methoxypiperidino group, a 2-aminomethylpiperidino group, a 2-methylaminomethylpiperidino group, a 2-dimethylaminomethylpiperidino group, a 2-aminoethylpiperidino group, a 2-methylaminoethylpiperidino group, a 2-dimethylaminoethylpiperidino group;

a piperazino group, a 2-oxo-4-methylpiperazino group, a 3-oxo-4-methylpiperazino group, a 4-formylpiperazino group, a 2,3-dioxo-4-methylpiperazino group, a 3,5-dioxo-4-methylpiperazino group, a 2, 6-dioxo-4-methylpiperazino group, a 4-methylpiperazino group, a 4-ethylpiperazino group, a 4-isopropylpiperazino group, a 4-cyclopropylpiperazino group, a2,4-dimethylpiperazino group, a 3,4-dimethylpiperazino group, a 2-ethyl-4-methylpiperazino group, a 3-ethyl-4-methylpiperazino group, a 2-isopropyl-4-methylpiperazino group, a 3-isopropyl-4-methylpiperazino group, a 2-cyclopropyl-4-methylpiperazino group, a 3-cyclopropyl-4-methylpiperazino group, a 3,4,5-trimethylpiperazino group, a 2,2,4-trimethylpiperazino group, a 3,3,4-trimethylpiperazino group, a 3,3,4-trimethyl-5-oxopiperazino group, a 2,2,4-trimethyl-3-oxopiperazino group, a 2-hydroxymethyl-4-methylpiperazino group, a 3-hydroxymethyl-4-methylpiperazino group, a 2-methoxymethyl-4-methylpiperazino group, a 3-methoxymethyl-4-methylpiperazino group, a 2-hydroxyethyl-4-methylpiperazino group, a 3-hydroxyethyl-4-methylpiperazino group, a 2-methoxyethyl-4-methylpiperazino group, a 3-methoxyethyl-4-methylpiperazino group, a 2-carbamoyl-4-methylpiperazino group, a 3-carbamoyl-4-methylpiperazino group, a4-carbamoylpiperazino group, a 2-methylcarbamoyl-4-methylpiperazino group, a 3-methylcarbamoyl-4-methylpiperazino group, a 4-methylcarbamoylpiperazino group, a 2-dimethylcarbamoyl-4-methylpiperazino group, a 3-dimethylcarbamoyl-4-methylpiperazino group, a 4-dimethylcarbamoylpiperazino group, a 2-carbamoylmethyl-4-methylpiperazino group, a 3-carbamoylmethyl-4-methylpiperazino group, a 4-carbamoylmethylpiperazino group, a 2-methylcarbamoylmethyl-4-methylpiperazino group, a 3-methylcarbamoylmethyl-4-methylpiperazino group, a 4-methylcarbamoylpiperazino group, a 2-dimethylcarbamoylmethyl-4-methylpiperazino group, a 3-dimethylcarbamoylmethyl-4-methylpiperazino group, a 2-aminomethyl-4-methylpiperazino group, a 2-methylaminomethyl-4-methylpiperazino group, a 2-dimethylaminomethyl-4-methylpiperazino group, a 2-aminoethyl-4-methylpiperazino group, a 2-methylaminoethyl-4-methylpiperazino group, a 2-dimethylaminoethyl-4-methylpiperazino group;

a morpholino group, a 2-methylmorpholino group, a 3-methylmorpholino group, a 2-ethylmorpholino group, a 3-ethylmorpholino group, a 2,2-dimethylmorpholino group, a 3,3-dimethylmorpholino group, a 3-hydroxymethylmorpholino group, a 3-methoxymethylmorpholino group, a 3-hydroxyethylmorpholino group, a 3-methoxyethylmorpholino group, a 3-carbamoylmorpholino group, a 3-methylcarbamoylmorpholino group, a 3-dimethylcarbamoylmorpholino group, a 3-carbamoylmethylmorpholino group, a 3-methylcarbamoylmethylmorpholino group, a 3-dimethylcarbamoylmethylmorpholino group, a 3-carbamoylethylmorpholino group, a 3-methylcarbamoylethylmorpholino group, a 3-dimethylcarbamoylethylmorpholino group, a 3-aminomethylmorpholino group, a 3-methylaminomethylmorpholino group, a 3-dimethylaminomethylmorpholino group, a 3-aminoethylmorpholino group, a 3-methylaminoethylmorpholino group, a 3-dimethylaminoethylmorpholino group;

a 2-acetylhexahydropyridazin-1-yl group, a 2-formylhexahydropyridazin-1-yl group, a 2-methylhexahydropyridazin-1-yl group, a 3-oxohexahydropyridazin-1-yl group, a 6-oxohexahydropyridazin-1-yl group, a 2,3-dimethylhexahydropyridazin-1-yl group, a 3-hydroxymethylhexahydropyridazin-1-yl group, a 5-hydroxymethylhexahydropyridazin-1-yl group, a 6-hydroxymethylhexahydropyridazin-1-yl group, a 2-carbamoylhexahydropyridazin-1-yl group, a 2-methylcarbamoylhexahydropyridazin-1-yl group, a 2-dimethylcarbamoylhexahydropyridazin-1-yl group;

a 2-oxohexahydropyrimidin-1-yl group, a 4-oxohexahydropyrimidin-1-yl group, a 6-oxohexahydropyrimidin-1-yl group, a 2-methylhexahydropyrimidin-1-yl group, a 3-methylhexahydropyrimidin-1-yl group, a 3-carbamoylhexahydropyrimidin-1-yl group, a 3-methylcarbamoylhexahydropyrimidin-1-yl group, a 3-dimethylcarbamoylhexahydropyrimidin-1-yl group, a 6-hydroxymethylpyrimidin-1-yl group;

a 2-oxo-4-methylhomopiperazino group, a 3-oxo-4-methylhomopiperazino group, a 5-oxo-4-methylhomopiperazino group, a 6-oxo-4-methylhomopiperazino group, a 7-oxo-4-methylhomopiperazino group, a 2,3-dioxohomopiperazino group, a 2,7-dioxohomopiperazino group, a 3,5-dioxohomopiperazino group,a 3, 7-dioxohomopiperazino group, a 2,3-dioxo-4-methylhomopiperazino group, a 2,7-dioxo-4-methylhomopiperazino group, a 3,5-dioxo-4-methylhomopiperazino group, a 3,7-dioxo-4-methylhomopiperazino group, a 4-methylhomopiperazino group, a 4-ethylhomopiperazino group, a 4-cyclopropylhomopiperazino group, a 2,4-dimethylhomopiperazino group, a 3,4-dimethylhomopiperazino group, a 3,4,5-trimethylhomopiperazino group, a 2-hydroxymethyl-4-methylhomopiperazino group, a 7-hydroxymethyl-4-methylhomopiperazino group, a 2-methoxymethyl-4-methylhomopiperazino group, a 3-methoxymethyl-4-methylhomopiperazino group, a 5-methoxymethyl-4-methylhomopiperazino group, a 6-methoxymethyl-4-methylhomopiperazino group, a 7-methoxymethyl-4-methylhomopiperazino group, a 2-hydroxyethyl 4-methylhomopiperazino group, a 7-hydroxyethyl-4-methylhomopiperazino group, a 2-methoxyethyl-4-methylhomopiperazino group, a 3-methoxyethyl-4-methylhomopiperazino group, a 5-methoxyethyl-4-methylhomopiperazino group, a 6-methoxyethyl-4-methylhomopiperazino group, a 7-methoxyethyl-4-methylhomopiperazino group, a 2-carbamoyl-4-methylhomopiperazino group, a 7-carbamoyl-4-methylhomopiperazino group, a 2-methylcarbamoyl-4-methylhomopiperazino group, a 7-methylcarbamoyl-4-methylhomopiperazino group, a 2-dimethylcarbamoylhomopiperazino group, a 7-dimethylcarbamoylhomopiperazino group; a 1,4-oxazepan-4-yl group;

a 3-methyl-4-oxoimidazolidin-1-yl group, and the like.

Among them, the following are more preferred. An azetidin-1-yl group, a 3-dimethylaminoazetidin-1-yl group, a 2,2-dimethyl-3-dimethylaminoazetidin-1-yl group, a 2-hydroxymethylazetidin-1-yl group, a 2-carbamoylazetidin-1-yl group, a 2-methylcarbamoylazetidin-1-yl group, a 2-dimethylcarbamoylazetidin-1-yl group, a 3,3-difluoroazetidin-1-yl group;

a pyrrolidino group, a 2-methylpyrrolidino group, a 2-aminomethylpyrrolidino group, a 2-hydroxymethylpyrrolidino group, a 2-methoxymethylpyrrolidino group, a 2-fluoromethylpyrrolidino group, a 2-trifluoromethylpyrrolidino group, a 2,2-dimethylpyrrolidino group, a 2,5-dimethylpyrrolidino group, a 2-carbamoylpyrrolidino group, a 2-methoxypyrrolidino group, a 2-oxopyrrolidino group, a 2-methoxymethyl-5-methylpyrrolidino group, a 3-methylpyrrolidino group, a 3-methoxymethylpyrrolidino group,a3-fluoromethylpyrrolidino group, a 3-trifluoromethylpyrrolidino group, a 3-aminopyrrolidino group, a 3-hydroxymethylpyrrolidino group, a 3-carbamoylpyrrolidino group, a 3-methoxypyrrolidino group, a 3-fluoropyrrolidino group, a 3,3-difluoropyrrolidino group;

an imidazolidin-1-yl group, a 3-methylimidazolidin-1-yl group, a 2-oxoimidazolidin-1-yl group, a 4-oxoimidazolidin-1-yl group, a 3-methyl-2-oxoimidazolidin-1-yl group, a 3-methyl-4-oxoimidazolidin-1-yl group, a 2,2-dimethylimidazolin-1-yl group;

a pyrazolidin-1-yl group, a 2-methylpyrazolidin-1-yl group, a 2-formylpyrazolidin-1-yl group, a 2-methylsulfonylpyrazolidin-1-yl group;

a piperidino group, a 2-oxopiperidino group, a 2-methoxypiperidino group, a 3-methoxypiperidino group, a 4-methoxypiperidino group, a 2-hydroxymethylpiperidino group, a 2-carbamoylpiperidino group, a 2-methylcarbamoylpiperidino group, a 2-dimethylcarbamoylpiperidino group, a 2-methoxymethylpiperidino group, a 2-aminomethylpiperidino group, a 2-methylaminomethylpiperidino group, a 2-dimethylaminomethylpiperidino group, a 2-aminoethylpiperidino group, a 2-methylaminoethylpiperidino group, a 2-dimethylaminoethylpiperidino group, a 3-fluoropiperidino group, a 4-fluoropiperidino group, a 4-methylpiperidino group, a 4-methoxypiperidino group, a 3,3-difluoropiperidino group, a 4, 4-dif luoropiperidino group, a 3-fluoromethylpiperidino group, a 4-fluoromethylpiperidino group, a 4-methyl-4-methoxypiperidino group;

a piperazino group, a 2-oxo-4-methylpiperazino group, a 3-oxo-4-methylpiperazino group, a 4-formylpiperazino group, a 2,3-dioxo-4-methylpiperazino group, a 3,5-dioxo-4-methylpiperazino group, a 2,6-dioxo-4-methylpiperazino group, a 4-methylpiperazino group, a 4-ethylpiperazino group, a 4-isopropylpiperazino group, a 4-cyclopropylpiperazino group,a2,4-dimethylpiperazino group, a 3,4-dimethylpiperazino group, a 2-ethyl-4-methylpiperazino group, a 3-ethyl-4-methylpiperazino group, a 3,4,5-trimethylpiperazino group, a 2,2,4-trimethylpiperazino group, a 3,3,4-trimethylpiperazino group, a 3,3,4-trimethyl-5-oxopiperazino group, a 2,2,4-trimethyl-3-oxopiperazino group, a 2-hydroxymethyl-4-methylpiperazino group, a 3-hydroxymethyl-4-methylpiperazino group, a 2-methoxymethyl-4-methylpiperazino group, a 3-methoxymethyl-4-methylpiperazino group, a 2-hydroxyethyl-4-methylpiperazino group, a 3-hydroxyethyl-4-methylpiperazino group, a 2-methoxyethyl-4-methylpiperazino group, a 3-methoxyethyl-4-methylpiperazino group, a 2-carbamoyl-4-methylpiperazino group, a 2-methylcarbamoyl-4-methylpiperazino group, a 2-dimethylcarbamoyl-4-methylpiperazino group, a 2-carbamoylmethyl-4-methylpiperazino group, a 2-methylcarbamoylmethyl-4-methylpiperazino group, a 2-dimethylcarbamoylmethyl-4-methylpiperazino group, a 2-aminomethyl-4-methylpiperazino group, a 2-methylaminomethyl-4-methylpiperazino group, a 2-dimethylaminomethyl-4-methylpiperazino group, a 2-aminoethyl-4-methylpiperazino group, a 2-methylaminoethyl-4-methylpiperazino group, a 2-dimethylaminoethyl-4-methylpiperazino group;

a morpholino group, a 2,2-dimethylmorpholino group, a 3,3-dimethylmorpholino group, a 3-hydroxymethylmorpholino group, a 3-methoxymethylmorpholino group, a 3-hydroxyethylmorpholino group, a 3-methoxyethylmorpholino group, a 3-carbamoylmorpholino group, a 3-methylcarbamoylmorpholino group, a 3-dimethylcarbamoylmorpholino group, a 3-aminomethylmorpholino group, a 3-methylaminomethylmorpholino group, a 3-dimethylaminomethylmorpholino group, a 3-aminoethylmorpholino group, a 3-methylaminoethylmorpholino group, a 3-dimethylaminoethylmorpholino group;

a 2-acetylhexahydropyridazin-1-yl group, a 2-formylhexahydropyridazin-1-yl group, a 3-oxohexahydropyridazin-1-yl group, a 2-methylhexahydropyridazin-1-yl group, a 2-carbamoylhexahydropyridazin-1-yl group;

a 2-oxohexahydropyrimidin-1-yl group, a 4-oxohexahydropyrimidin-1-yl group, a 3-methylhexahydropyrimidin-1-yl group, a 6-hydroxymethylhexahydropyrimidin-1-yl group;

a 2-oxo-4-methylhomopiperazino group, a 3-oxo-4-methylhomopiperazino group, a 5-oxo-4-methylhomopiperazino group, a 7-oxo-4-methylhomopiperazino group, a 2,3-dioxo-4-methylhomopiperazino group, a 2,7-dioxo-4-methylhomopiperazino group, a 3,5-dioxo-4-methylhomopiperazino group, a 3,7-dioxo-4-methylhomopiperazino group, a 4-methylhomopiperazino group, a 4-ethylhomopiperazino group, a 4-cyclopropylhomopiperazino group;

a 1,4-oxazepan-4-yl group; a 3-methyl-4-oxoimidazolidin-1-yl group, and the like.

Among them, the following are more preferred. A 3-dimethylaminoazetidin-1-yl group, a 2,2-dimethyl-3-dimethylaminoazetidin-1-yl group, a 2-hydroxymethylazetidin-1-yl group, a 2-carbamoylazetidin-1-yl group, a 3,3-difluoroazetidin-1-yl group;

a pyrrolidino group, a 2-methylpyrrolidino group, a 2-fluoromethylpyrrolidino group, a 2-trifluoromethylpyrrolidino group, a 2-hydroxymethylpyrrolidino group, a 2,5-dimethylpyrrolidino group, a 2-carbamoylpyrrolidino group, a 3-fluoropyrrolidino group, a 3,3-difluoropyrrolidino group;

a pyrazolidino group, a 2-methyl-pyrazolidin-1-yl group, a 2-formyl-pyrazolidin-1-yl group, a 2-methylsulfonyl-pyrazolidin-1-yl group;

a piperidino group, a 2-hydroxymethylpiperidino group, a 2-carbamoylpiperidino group, a 2-methylcarbamoylpiperidino group, a 2-dimethylcarbamoylpiperidino group, a 3-methoxypiperidino group, a 3-fluoropiperidino group, a 4-fluoropiperidino group, a 4-methylpiperidino group, a 4-methoxypiperidino group, a 3,3-difluoropiperidino group, a 4,4-difluoropiperidino group, a 4-fluoromethylpiperidino group, a 4-methyl-4-methoxypiperidino group;

a 3-oxo-4-methylpiperazino group, a 4-methylpiperazino group, a 4-ethylpiperazino group, a 4-isopropylpiperazino group, a 4-cyclopropylpiperazino group, a 2, 4-dimethylpiperazino group, a 3,4-dimethylpiperazino group, a 3,4,5-trimethylpiperazino group, a 2,2,4-trimethylpiperazino group, a 3,3,4-trimethylpiperazino group;

a morpholino group, a 3-carbamoylmorpholino group; a thiomorpholino group, a 1,1-dioxothiomorpholino group;

a 2-methylhexahydropyridazin-1-yl group, a 3-methylhexahydropyridazin-1-yl group;

a 3-oxo-4-methylhomopiperazino group, a 5-oxo-4-methylhomopiperazino group, a 4-methylhomopiperazino group, a 4-ethylhomopiperazino group, a 4-cyclopropylhomopiperazino group;

a 1,4-oxazepan-4-yl group; a 3-methyl-4-oxoimidazolidin-1-yl group;

a 2-acetylhexahydropyridazin-1-yl group, a 2-carbamoylhexahydropyridazin-1-yl group, a tetrahydropyran-4-yl group, and the like.

Among them, a 3,3-difluoroazetidin-1-yl group, a piperidino group, a 4-methoxypiperidino group, a 4,4-difluoropiperidino group, a 4-methylpiperazino group, a 3-oxo-4-methylpiperazino group, a 2-fluoromethylpyrrolidino group, and the like are particularly preferred.

With regard to the compound (I) of the present invention, it is preferable that Ar₁, Ar₂, R¹ and R² represent the groups described above, and it is preferable to have combinations of the particularly preferable groups. The particularly preferred compound (I)includes the compounds described below.
Namely,
N-tert-butyl-1-(6-methoxy-3-pyridyl)-5-(2-pyridyl)-1H-1,2,4 -triazole-3-carboxamide,
N-cyclopentyl-1-(6-methoxy-3-pyridyl)-5-(2-pyridyl)-1H-1,2, 4-triazole-3-carboxamide,
N-tert-butyl-5-(5-cyano-2-pyridyl)-1-(6-methoxy-3-pyridyl)-1H-1,2,4-triazole-3-carboxamide,
5-(5-cyano-2-pyridyl)-1-(6-methoxy-3-pyridyl)-1H-1,2,4-tria zole-3-carboxylic acid N-neopentylamide,
1-(6-methoxy-3-pyridyl)-5-(2-pyridyl)-1H-1,2,4-triazole-3-c arboxylic acid N-neopentylamide,
N-tert-butyl-1-(6-methoxy-3-pyridyl)-5-(5-methyl-2-pyraziny 1)-1H-1,2,4-triazole-3-carboxamide,
1-(6-methoxy-3-pyridyl)-5-(5-methyl-2-pyrazinyl)-1H-1,2,4-t riazole-3-carboxylic acid N-neopentylamide,
N-tert-butyl-1-(6-methoxy-3-pyridyl)-5-(2-pyrazinyl)-1H-1,2 ,4-triazole-3-carboxamide,
N-tert-butyl-1-(6-methoxy-3-pyridyl)-5-(5-methyl-2-pyridyl) -1H-1,2,4-triazole-3-carboxamide,
1-(6-methoxy-3-pyridyl)-5-(5-methyl-2-pyridyl)-1H-1,2,4-tri azole-3-carboxylic acid N-(tetrahydropyran-4-yl)amide,
N-tert-butyl-5-(5-chloro-2-pyridyl)-1-(6-methoxy-3-pyridyl) -1H-1,2,4-triazole-3-carboxamide,
N-tert-butyl-5-(5-fluoro-2-pyridyl)-1-(6-methoxy-3-pyridyl) -1H-1,2,4-triazole-3-carboxamide,
5-(5-tert-butoxycarbonylamino-2-pyridyl)-1-(6-methoxy-3-pyr idyl)-1H-1,2,4-triazole-3-carboxylic acid tert-butylamide,
5-(5-amino-2-pyridyl)-1-(6-methoxy-3-pyridyl)-1H-1,2,4-tria zole-3-carboxylic acid N-tert-butylamide,
N-tert-butyl-5-(5-methanesulfonylamino-2-pyridyl)-1-(6-meth oxy-3-pyridyl)-1H-1,2,4-triazole-3-carboxamide,
5-(5-acetylamino-2-pyridyl)-1-(6-methoxy-3-pyridyl)-1H-1,2, 4-triazole-3-carboxylic acid N-tert-butylamide,
N-tert-butyl-1-(3-pyridyl)-5-(2-pyridyl)-1H-1,2,4-triazole-3-carboxamide,
1-(6-methoxy-3-pyridyl)-5-(5-methyl-2-pyridyl)-1H-1,2,4-tri azole-3-carboxylic acid N-neopentylamide,
N-(2-fluoro-1,1-dimethylethyl)-1-(6-methoxy-3-pyridyl)-5-(2 -pyridyl)-1H-1,2,4-triazole-3-carboxamide,
N-tert-butyl-5-(5-methyl-2-pyridyl)-1-(3-pyridyl)-1H-1,2,4-triazole-3-carboxamide,
N-methoxy-N-methyl-1-(6-methoxy-3-pyridyl)-5-(2-pyridyl)-1H -1,2,4-triazole-3-carboxamide,
N-isopropyl-1-(6-methoxy-3-pyridyl)-5-(2-pyridyl)-1H-1,2,4-triazole-3-carboxamide,
1-[1-(6-methoxy-3-pyridyl)-5-(5-methyl-2-pyrazinyl)-1H-1,2, 4-triazole-3-carbonyl]-4,4-difluoropiperidine,
1-[1-(6-methoxy-3-pyridyl)-5-(5-methyl-2-pyrazinyl)-1H-1,2, 4-triazole-3-carbonyl]-4-methoxypiperidine,
1-[5-(5-fluoro-2-pyridyl)-1-(6-methoxy-3-pyridyl)-1H-1,2,4-triazole-3-carbonyl]-4-methoxypiperidine,
1-[1-(6-methoxy-3-pyridyl)-5-(2-pyridyl)-1H-1, 2, 4-triazole-3-carbonyl]-4,4-difluoropiperidine,
1-[1-(6-methoxy-3-pyridyl)-5-(2-pyridyl)-1H-1,2,4-triazole-3-carbonyl]-4-methoxypiperidine,
1-[1-(6-methoxy-3-pyridyl)-5-(2-pyridyl)-1H-1,2,4-triazole-3-carbonyl]-3,3-difluoroazetidine,
1-[1-(6-methoxy-3-pyridyl)-5-(5-methyl-2-pyridyl)-1H-1,2,4-triazole-3-carbonyl]-4-methylpiperazine,
N-tert-butyl-1-(6-methoxy-3-pyridyl)-5-(1-methyl-1H-pyrazol -3-yl)-1H-1,2,4-triazole-3-carboxamide,
N-tert-butyl-1-(6-methoxy-3-pyridyl)-5-(1-methyl-1H-imidazo 1-4-yl)-1H-1,2,4-triazole-3-carboxamide,
1-[1-(6-methoxy-3-pyridyl)-5-(1-methyl-1H-imidazol-4-yl)-1H -1,2,4-triazole-3-carbonyl]-4,4-difluoropiperidine,
N-methoxy-N-methyl-1-(6-methoxy-3-pyridyl)-5-(5-methyl-2-py ridyl)-1H-1,2,4-triazole-3-carboxamide,
N,N-dimethyl-1-(6-methoxy-3-pyridyl)-5-(5-methyl-2-pyridyl) -1H-1,2,4-triazole-3-carboxamide,
N-tert-butyl-1-(6-methyl-3-pyridyl)-5-(5-methyl-2-pyridyl)-1H-1,2,4-triazole-3-carboxamide,
1-[1-(6-methoxy-3-pyridyl)-5-(5-methyl-2-pyridyl)-1H-1,2,4-triazole-3-carbonyl]-4-methyl-3-oxopiperazine,
(2S)-1-[1-(6-methoxy-3-pyridyl)-5-(5-methyl-2-pyridyl)-1H-1 ,2,4-triazole-3-carbonyl]-2-fluoromethylpyrrolidine,
4-[1-(6-methoxy-3-pyridyl)-5-(5-methyl-2-pyridyl)-1H-1,2,4-triazole-3-carbonyl]morpholine,
1-[5-(5-carbamoyl-2-pyridyl)-1-(6-methoxy-3-pyridyl)-1H-1,2 ,4-triazole-3-carbonyl]-4,4-difluoropiperidine,
1-[5-(5-aminomethyl-2-pyridyl)-1-(6-methoxy-3-pyridyl)-1H-1 ,2,4-triazole-3-carbonyl]-4,4-difluoropiperidine,
1-[5-(5-hydroxymethyl-2-pyridyl)-1-(6-methoxy-3-pyridyl)-1H -1,2,4-triazole-3-carbonyl]-4,4-difluoropiperidine,
N-tert-butyl-1-(6-methyl-3-pyridyl)-5-(2-pyridyl)-1H-1,2,9-triazole-3-carboxamide,
N-tert-butyl-5-(5-amino-2-pyrazinyl)-1-(6-methoxy-3-pyridyl )-1H-1,2,4-triazole-3-carboxamide,
N-(1-methylcyclopropyl)-1-(6-methoxy-3-pyridyl)-5-(5-methyl -2-pyridyl)-1H-1,2,4-triazole-3-carboxamide.

With regard to the salt of the compound (I) of the present invention, it cannot be said that all of the compounds of the present invention form salts, but the compounds having a carbonyl group, an amino group and the like, or the compounds having a pyridine ring and the like for Ar₁ or Ar₂, form salts. Moreover, the salts may form solvates. The salt mentioned herein includes salts of inorganic acids such as hydrochloric acid, hydrobromic acid, sulfuric acid, nitric acid and the like; salts of organic acids such as methanesulfonic acid, p-toluenesulfonic acid, fumaric acid, trifluoroacetic acid and the like; and salts with the ions of alkali metals or alkaline earth metals such as sodium, potassium, calcium and the like.

The solvate as used in the phrase "the solvate of the compound (I) of the present invention or the salt" includes, in addition to the solvates formed by adding the solvent used in crystallization and the like, those formed by absorbing moisture in the air. Examples of the solvent include, for example, water, lower alcohols such as methanol, ethanol and the like, organic solvents such as acetone, acetonitrile and the like.

The compound (I) of the present invention can be produced by the methods described below. Hereinafter, representative methods for producing the compound (I) of the present invention will be described.

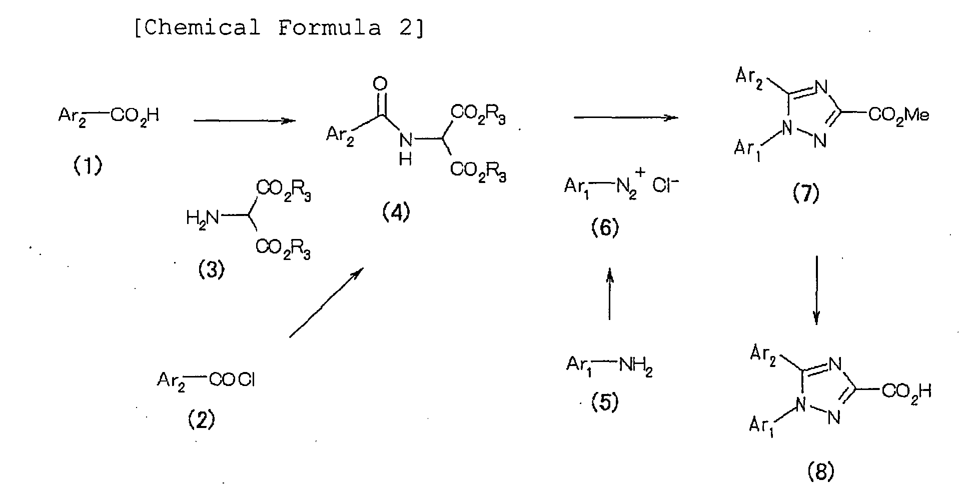

wherein Ar₁ and Ar₂ represent the same ones as those described above; and R₃ represents a lower alkyl group.

Amide (4) can be produced by condensing carboxylic acid (1) with amine (3). The amide (4) can also be produced by acylating the amine (3) with acid chloride (2).

The above-described reaction may be performed by applying correspondingly those methods generally used as the method of peptide synthesis. Examples of the method of peptide synthesis generally used include, for instance, an azide method, an acid chloride method, an acid anhydride method, a DCC (dicyclocarbodiimide) method, an active ester method, a carbodiimidazole method, a DCC/HOBT (1-hydroxybenzotriazole) method, a method of using a water-soluble carbodiimide, a method of using diethylcyanophosphate, and the like, and these methods are described in M. Bodanszky, Y.S. Klausner and M.A. Ondetti, "Peptide Synthesis" (A Wiley-interscience publication, New York, 1976); G.R. Pettit, "Synthetic Peptides" (Elsevier Scientific Publication Company, New York, 1976); The Chemical Society of Japan, "Lectures on Experimental Chemistry, 4th Ed., Vol. 22, Organic Synthesis IV" (Maruzen Co., Ltd., 1992), and the like. The solvent used in this condensation reaction includes solvents such as N,N-dimethylformamide, pyridine, chloroform, methylene chloride, tetrahydrofuran, dioxane, acetonitrile and the like, and solvent mixtures thereof. The reaction temperature is preferably -20 to 50°C, and more preferably -10 to 30°C. For the carboxylic acid (1) and the acid chloride (2), commercially available compounds may be used, or those produced according to the methods described in Reference Examples or methods equivalent thereto, may be used.

Furthermore, the amide (4) can be converted to various derivatives by modifying the Ar₂ moiety on the basis of conventional knowledge in organic chemistry. For example, a benzyl ester derivative (4a) as shown below may be converted to various derivatives (4b to 4e) by modifying the benzyl ester moiety to carboxylic acid, urethane, amine and halogen, respectively.

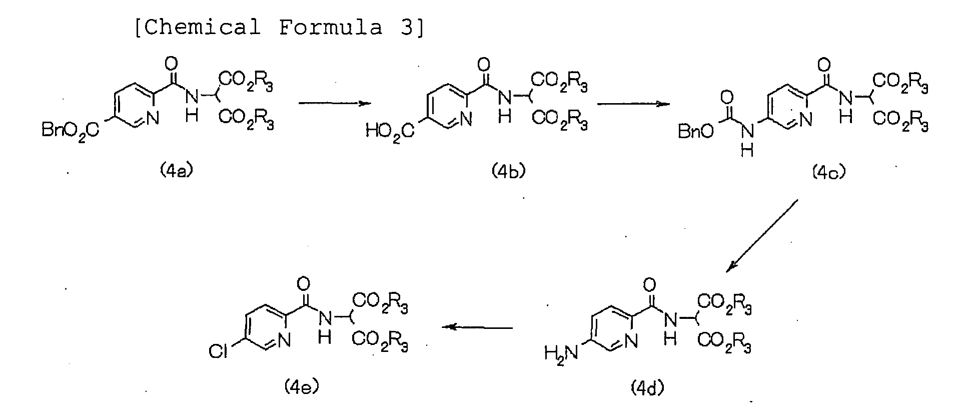

wherein R₃ represents the same ones as those described above; and Bn represents a benzyl group.

Specifically, carboxylic acid (4b) can be produced by dissolving the benzyl ester derivative (4a) in 1,4-dioxane or the like, and catalytically reducing the resultant using 10% palladium on carbon as a catalyst. The carboxylic acid (4b) can be derived to a urethane (4c) by adding benzyl alcohol, triethylamine and diphenylphosphorylazide to the carboxylic acid in a solvent such as 1,4-dioxane or the like at room temperature, and then heating to reflux. The urethane (4c) can be derived to an amine (4d) by catalytically reducing the urethane in a solvent such as 1,4-dioxane or the like, using 10% palladium on carbon as a catalyst. The amine (4d) can be derived to a chloroderivative (4e) by adding tert-butyl nitrite and anhydrous copper (II) chloride in a solvent such as acetonitrile or the like at room temperature, and then stirring the resultant at 65°C. The reaction conditions for the reactions described above may be appropriately selected on the basis of conventional knowledge in organic chemistry.

Triazole ester (7) can be produced as follows. The triazole ester (7) can be obtained by dissolving amine (5) in acetic acid and concentrated hydrochloric acid, treating the resultant solution with sodium nitrite to prepare a diazonium salt (6), adding an acetone solution of the amide (4) and an aqueous solution of potassium carbonate to the diazonium salt, extracting the resultant product with a ethyl acetate or the like, concentrating the resultant under reduced pressure, dissolving the residue into a solution in anhydrous methanol without purifying, and then reacting the solution with sodium methoxide at roomtemperature. For the reaction of the diazonium salt (6) and the amide (4), the reaction solution may be adjusted to around pH 6. The reaction temperature in this case is preferably-30 to 20°C. Also, for the production of the diazonium salt (6), a reaction temperature around 0°C is preferred.
The triazole ester (7) can be converted to triazole carboxylic acid (8) by dissolving the triazole ester in methanol, and treating the resultant with an aqueous solution of 1 mol/L sodium hydroxide.
The reaction of forming the triazole ring can also be performed by applying the method described in Helv. Chim. Acta., Vol. 73, p. 1701 (1990) correspondingly.

With regard to the amine (5), those commercially available may be used, or those produced according to the methods described in Reference Examples or methods equivalent thereto may also be used.

Furthermore, the triazole ester (7) can be converted to various derivatives by further modifying the ester on the basis of conventional knowledge in organic chemistry. For example, triazole ester (7a) can be converted to various derivatives (7b to 7d) of alcohol, triflate and nitrile.

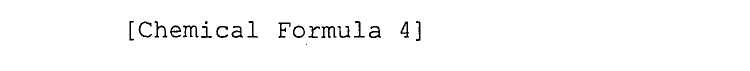

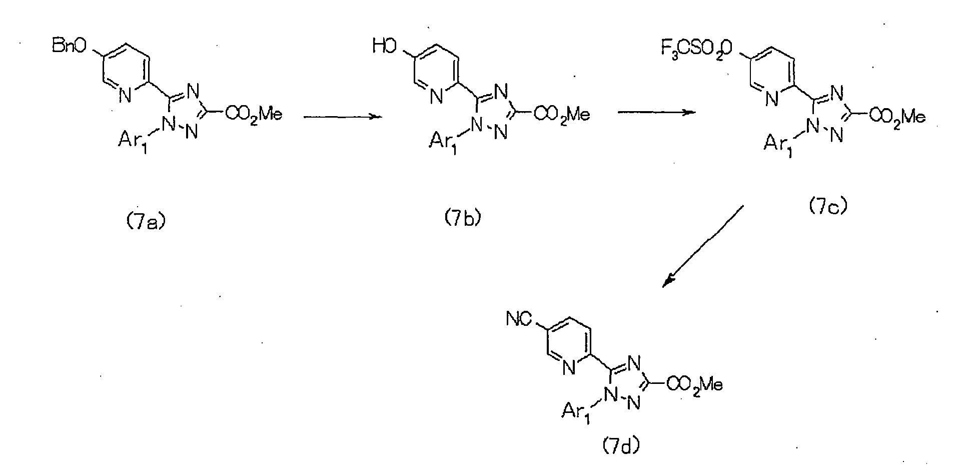

wherein Ar₁ represents the same ones as those described above; and Bn represents a benzyl group.

Specifically, a hydroxyl derivative (7b) can be produced by dissolving the benzyloxy derivative (7a) in a mixed solvent of methanol/ethyl acetate/acetic acid or the like, and catalytically reducing the resultant solution using 10% palladium on carbon as a catalyst. A triflate derivative (7c) can be produced by dissolving the hydroxyl derivative (7b) in methylene chloride or the like, and reacting the resultant solution with trifluoromethanesulfonic anhydride at -50 to 50°C in the presence of a base such as pyridine or the like. Furthermore, a cyano derivative (7d) can be produced by dissolving the triflate derivative (7c) in 1,2-dichloroethane or the like, and reacting the resultant solution with tri-n-butyltin cyanide and tetrakis(triphenylphosphine)palladium (0). The reaction temperature is preferably 10 to 100°C. The reaction conditions for the above-described reactions may be appropriately selected on the basis of conventional knowledge in organic chemistry.

The triazole ester (7) can be derived to carboxylic acid (8) by hydrolysis according to a conventional method. This hydrolysis reaction can be performed in the presence of a base or a Lewis acid. The base includes hydroxides of alkali metals (for example, lithium, sodium, potassium, or the like). The Lewis acid includes, for example, boron tribromide. The reaction temperature is preferably -20 to 100°C, and more preferably -5 to 50°C.

The triazole compound (I) of the present invention can be produced by condensing carboxylic acid (8) and amine (9).

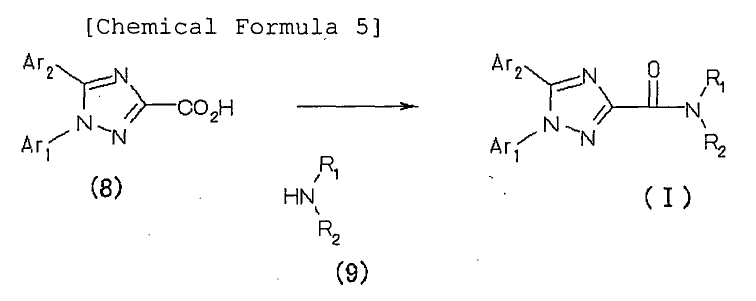

wherein Ar₁, Ar₂, R¹ and R² represent the same ones as those described above.

The condensation reaction described above may be performed by applying correspondingly the methods generally used as the method of peptide synthesis. Examples of the method of peptide synthesis generally used include an azide method, an acid chloride method, an acid anhydride method, a DCC (dicyclocarbodiimide) method, an active ester method, a carbodiimidazole method, a DCC/HOBT (1-hydroxybenzotriazole) method, a method of using a water-soluble carbodiimide, a method of using diethylcyanophosphate, and the like, and these methods are described in M. Bodanszky, Y.S. Klausner and M.A. Ondetti, "Peptide Synthesis" (AWiley -interscience publication, New York, 1976); G.R. Pettit, "Synthetic Peptides" (Elsevier Scientific Publication Company, New York, 1976); The Chemical Society of Japan, "Lectures on Experimental Chemistry, 4th Ed., Vol. 22, Organic Synthesis IV" (Maruzen Co., Ltd., 1992), and the like. The solvent used in this condensation reaction includes solvents such as N, N-dimethylformamide, pyridine, chloroform, methylene chloride, tetrahydrofuran, dioxane, acetonitrile and the like, or solvent mixtures thereof. The reaction temperature is preferably -20 to 50°C, and more preferably -10 to 30°C. For the amine (9), commercially available compounds may be used, or those produced according to the methods described in Reference Examples or methods equivalent thereto, may be used.

With respect to the above-described condensation reaction, in case where the amine (9) has a functional group such as a hydroxyl group, an amino group, a carboxyl group or the like, it may be necessary to protect the functional group in advance, using an appropriate protective group. The protective group for the hydroxyl group includes a tert-butyl group, a benzyl group or the like, while the protective group for the amino group includes a trifluoroacetyl group, a tert-butoxycarbonyl group, a benzyloxycarbonyl group, or the like. If the functional group is a carboxyl group, the amine may be used in the condensation reaction after being derived to a methyl ester or a tert-butyl ester. These protective groups can be cleaved under the conditions that are suitable for the respective protective groups.

Also, on the basis of conventional knowledge in organic chemistry, the compound (I) of the present invention produced according to the methods described above can be derived to other compounds (I) of the present invention by further applying modifying.

The compound (I) of the present invention, a salt thereof, or a solvate of the compound or the salt has a potent platelet aggregation suppressing action, that is, an antiplatelet effect, and potently inhibited thrombus formation in high shear stress-induced thrombosis models. Moreover, the compound exhibited very good bioavailability in cynomolgus. Therefore, the compound (I) of the present invention, a salt thereof, or a solvate of the compound or the salt are useful in mammals including humans, as a prophylactic and/or therapeutic agent for ischemic diseases caused by thrombi and emboli, such as myocardial infarction, angina pectoris (chronic stable angina, unstable angina, and the like), ischemic cerebrovascular disorder (transient ischemic attack (TIA), cerebral infarction, and the like), peripheral vascular disorder, occlusion after replacement with an artificial vessel, thrombotic occlusion after coronary artery intervention (coronary artery bypass grafting (CABG), percutaneous transluminal coronary angioplasty (PTCA), stent placement, and the like), diabetic retinopathy and nephropathy, occlusion after replacement with an artificial heart valve, and the like. They are also useful for the prevention and/or treatment of thrombi and emboli associated with vascular surgery, blood extracorporeal circulation and the like. Furthermore, they are useful for an improvement in ischemic symptoms associated with chronic arterial occlusion, such as ulcer, pain, cold sensation and the like.

In the case of using the compound (I) of the present invention, a salt thereof, or a solvate of the compound or the salt as a medicine, dosage may vary depending on the age, gender, symptoms and the like of the patient, but a daily dose for an adult is preferably 0.1 mg to 1 g, and particularly preferably 0.5 mg to 500 mg. In this case, it is possible to administer the daily dose in several divided portions, and if necessary, it is also possible to administer an amount exceeding the daily dose.

A medicine containing the compound (I) of the present invention, a salt thereof, or a solvate of the compound or the salt as an active ingredient, can be used by applying appropriate administration methods and formulations according to the need. The preparation may be selected from formulations which are prepared according to the preparation methods for various conventionally used preparations, by blending in a pharmaceutically acceptable carrier if necessary, and which comply with the administration method, and the administration method and the formulation are not particularly limited.

Examples of oral preparations include solid preparations such as tablets, powders, granules, pills, capsules and the like, as well as liquid preparations such as liquid, syrup, elixir, suspension, emulsion and the like.
To prepare an injection, the compound (I), a salt thereof, or a solvate of the compound or the salt may be dissolved and filled in a container, or may be prepared into a solid ready-to-use preparation by lyophilization or the like.

In the case of preparing these preparations, pharmaceutically acceptable additives such as, for example, a binder, a disintegrant, a dissolution promoter, a gliding agent, a filler, an excipient and the like may be selected and used according to the need.

### EXAMPLES

Hereinafter, the present invention will be described in detail by making reference to Examples and Reference Examples, but the present invention is not intended to be limited to these examples.

### [Reference Example 1]

1-(6-Methoxy-3-pyridyl)-5-(2-pyridyl)-1H-1,2,4-triazole-3-c arboxylic acid

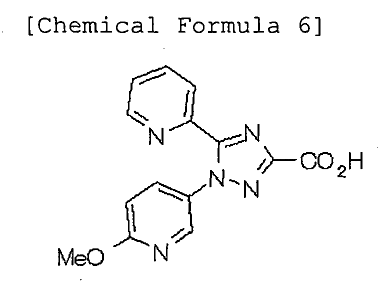

### 1) 2-[(Pyridine-2-carbonyl)amino]malonic acid dimethyl ester

Picolinoyl chloride hydrochloride (15.0 g) was added to a solution of aminomalonic acid dimethyl ester hydrochloride (18.56 g) and triethylamine (35.2 mL) in dichloromethane (210 mL) at 0°C, and the mixture was stirred at room temperature for 4.5 hours. A saturated aqueous solution of sodium hydrogen carbonate and dichloromethane were added to the reaction solution, and the mixture was partitioned. The organic layer was washed with saturated brine, and then was dried over anhydrous sodium sulfate. After separating the organic layer by filtration, the solvent was evaporated under reduced pressure, and a residue thus obtained was purified by silica gel column chromatography (ethyl acetate-hexane), to obtain 2-[(pyridine-2-carbonyl)amino]malonic acid dimethyl ester (17.9 g, 84%) as a solid.

¹H-NMR (400MHz, CDCl₃) δ: 3.87 (6H, s), 5.43 (1H, d, J=9.2Hz), 7.43-7.53 (1H, m), 7.86 (1H, td, J=7.7, 1.7Hz), 8.16-8.19 (1H, m), 8.94-9.00 (1H, m), 8.58-8.63 (1H, m).
MS (ESI) m/z: 253 (M+H)⁺.

### 2) 1-(6-Methoxy-3-pyridyl)-5-(2-pyridyl)-1H-1,2,4-triazole-3-c arboxylic acid methyl ester

A solution of sodium nitrite (3.1 g) in water (20 mL) was slowly added dropwise to a mixed solution of 5-amino-2-methoxypyridine (5.4 g) in acetic acid (26 mL) and concentrated hydrochloric acid (6.5 mL) at 0°C, and the mixture was stirred for 15 minutes. The reaction solution was cooled to -15°C, a solution of the 2-[(pyridine-2-carbonyl)amino]malonic acid dimethyl ester (10.0 g) obtained above in acetone (90 mL) and a solution of potassium carbonate (54.7 g) in water (80 mL) were slowly added to the reaction solution, and then the resultant mixture was stirred at 0°C for 30 minutes. Ethyl acetate was added to the reactionsolution,andthe mixture waspartitioned. The organic layer was washed sequentially with water, a saturated aqueous solution of sodium hydrogen carbonate, water, and saturated brine, and then was dried over anhydrous sodium sulfate. After filtration, the solvent was evaporated under reduced pressure, and a residue thus obtained was dissolved in methanol (200 mL). Sodium methoxide (356 mg) was added to the solution at room temprature, and the mixture was stirred for 19 hours. The reaction solvent was evaporated under reduced pressure, and the solid thus obtained was collected by filtration and washed with methanol, to obtain 1-(6-methoxy-3-pyridyl)-5-(2-pyridyl)-1H-1,2,4-triazole-3-c arboxylic acid methyl ester (2.6 g, 21%) as a solid.

¹H-NMR (400MHz, CDCl₃) δ: 3.99 (3H, s), 4.04 (3H, s), 6.82 (1H, d, 8.8Hz), 7.33 (1H, ddd, J=7.6, 4.8, 1.1Hz), 7.71 (1H, dd, J=8. 8, 2.7Hz), 7.83 (1H, td, J=7.8, 1.8Hz), 8.22-8.24 (2H, m), 8.43 (1H, dq, J=4.7, 0.9Hz).

### 3) Title compound

A 1 mol/L aqueous solution of sodium hydroxide (10 mL) was added to a solution of the 1-(6-methoxy-3-pyridyl)-5-(2-pyridyl)-1H-1,2,4-triazole-3-c arboxylic acid methyl ester (2.4 g) obtained above in methanol (20 mL), and the mixture was stirred at room temperature for 1 hour. A 1 mol/L aqueous solution of hydrochloric acid was added to the reaction solution to acidify the solution, and a solid precipitated therefrom was collected by filtration and dried, to obtain the title compound (1.84 g, 81%) as a solid.

¹H-NMR (400MHz, DMSO-d₆) δ: 3.91 (3H, s), 6.95 (1H, d, J=8.5Hz), 7.48 (1H, ddd, J=7.5, 4.8, 1.2Hz), 7.88 (1H, dd, J=8.6, 2.7Hz), 8.00 (1H, td, J=7.7, 1.7Hz), 8.11-8.14 (1H, m), 8.30-8.34 (1H, m), 8.41-8.43 (1H, m).
MS (ESI) m/z: 298 (M+H)⁺.

### [Reference Example 2]

5-(5-Cyano-2-pyridyl)-1-(6-methoxy-3-pyridyl)-1H-1,2,4-tria zole-3-carboxylic acid

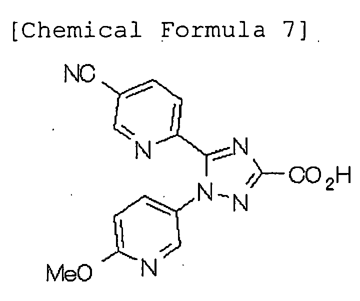

### 1) 5-Benzyloxy-2-methylpyridine

Benzyl bromide (10.9 mL) was added to a suspension of 5-hydroxy-2-methylpyridine (10.0 g) and potassium carbonate (38.0 g) in acetonitrile (200 mL) at room temperature, and the mixture was stirred for 12 hours. Water and ethyl acetate were added to the reaction solution, and the mixture was partitioned. The organic layer was dried over anhydrous sodium sulfate. After separating the organic layer by filtration, the solvent was evaporated under reduced pressure, and a residue thus obtained was purified by silica gel column chromatography (ethyl acetate-hexane), to obtain 5-benzyloxy-2-methylpyridine (4.14 g, 23%) as an oily product.

¹H-NMR (400MHz, CDCl₃) δ: 2.48 (3H, s), 5.08 (2H, s), 7.05 (1H, d, J=8.5Hz), 7.16 (1H, dd, J=8.5, 2.9Hz), 7.31-7.43 (5H, m), 8.26 (1H, d, J=2.9Hz).
EI-MS m/z: 199 (M⁺).

### 2) 2-[(5-Benzyloxypyridine-2-carbonyl)amino]malonic acid dimethyl ester

Selenium dioxide (40 g) was added to a solution of the 5-benzyloxy-2-methylpyridine (40 g) obtained above in pyridine (200 mL), and the mixture was heated to reflux for 24 hours. After air cooling, water and chloroform were added to the reaction solution, and the mixture was partitioned. The organic layer was dried over anhydrous sodium sulfate. After filtration, the solvent was evaporated under reduced pressure, toluene was added to the residue thus obtained, and the mixture was azeotropically evaporated under reduced pressure. The residue obtained was dissolved in N,N-dimethylformamide (1 L), and triethylamine (83.94 mL), 1-ethyl-3-(3-dimethylaminopropyl)carbodiimide hydrochloride (42.33 g), 1-hydroxybenzotriazole (29.8 g), and aminomalonic acid dimethyl ester hydrochloride (37 g) were added to the solution, which was then stirred at room temperature for 164 hours. Water and a mixed solvent of dichloromethane-methanol (10:1) were added to the reaction solution, and the mixture was partitioned. The organic layer was dried over anhydrous sodium sulfate. After filtration, the solvent was evaporated under reduced pressure, a residue thus obtained was purified by silica gel column chromatography (hexane-ethyl acetate), and the crude crystals were recrystallized from dichloromethane (methylene chloride)-hexane, to obtain 2-[(5-benzyloxypyridine-2-carbonyl)amino]malonic acid dimethyl ester (14.7 g).

¹H-NMR (400MHz, CDCl₃) δ: 3.85 (6H, s), 5.17 (2H, s), 5.42 (1H, d, J=7.4Hz), 7.33-7.44 (6H, m), 8.11 (1H, d, J=9.1Hz), 8.32 (1H, d, J=6.1Hz), 8.72 (1H, d, J=7.4Hz).

### 3) 5-(5-Benzyloxy-2-pyridyl)-1-(6-methoxy-3-pyridyl)-1H-1,2,4-triazole-3-carboxylic acid methyl ester

A solution of sodium nitrite (4.77 g) in water (17 mL) was added dropwise to a solution of 5-amino-2-methoxypyridine (5.9 g) in acetic acid (27 mL) and concentrated hydrochloric acid (6.75 mL) at 0°C, and the mixture was stirred for 15 minutes. At -15°C, a solution of the 2-[(5-benzyloxypyridine-2-carbonyl)amino]malonic acid dimethyl ester (14.7g) in acetone (68 mL) was added to the reaction solution, and a saturated aqueous solution of potassium carbonate was also added slowly until the reaction solution reached pH 6. After stirring the mixture at 0°C for 30 minutes, ethyl acetate was added to the reaction solution, and the resultant mixture waspartitioned. The organic layer was washed sequentially with water, a saturated aqueous solution of sodiumhydrogen carbonate, water, and saturated brine, and was dried over anhydrous sodium sulfate. After separating the organic layer by filtration, the solvent was evaporated under reduced pressure, and a residue thus obtained was dissolved in methanol (200 mL). Sodium methoxide (356 mg) was added to the solution at room temperature, and the resultant mixture was stirred for 19 hours. The reaction solvent was evaporated under reduced pressure, and the crude crystals thus obtained were collected by filtration and washed with cold methanol, to obtain 5-(5-benzyloxy-2-pyridyl)-1-(6-methoxy-3-pyridyl)-1H-1,2,4-triazole-3-carboxylic acid methyl ester (5.30 g, 31%).

¹H-NMR (400MHz, CDCl₃) δ: 4.01 (3H, s), 4.06 (3H, s), 5.13 (2H, s), 6.82 (1H, d, J=8.8Hz), 7.39-7.35 (6H, m), 7.70-7.68 (1H, m), 8.20-8.16 (3H, m).

### 4) 5-(5-Hydroxy-2-pyridyl)-1-(6-methoxy-3-pyridyl)-1H-1,2,4-tr iazole-3-carboxylic acid methyl ester

10% Palladium-carbon (5 g) was added to a mixed solution of the 5-(5-benzyloxy-2-pyridyl)-1-(6-methoxy-3-pyridyl)-1H-1,2,4-triazole-3-carboxylic acid methyl ester (5.3 g) obtained above in methanol (200 mL), acetic acid (50 mL), and ethyl acetate (300 mL), and the mixture was stirred under a hydrogen atmosphere at room temperature for 24 hours. The reaction solution was filtered, and the filtrate solvent was evaporated under reduced pressure. The solid thus obtained was recrystallized from ethyl acetate-hexane, to obtain 5-(5-hydroxy-2-pyridyl)-1-(6-methoxy-3-pyridyl)-1H-1,2,4-tr iazole-3-carboxylic acid methyl ester (3.4 g, 81%).

¹H-NMR (400MHz, CDCl₃) δ: 3.98 (3H, dd, J=19.6, 10.8Hz), 4.03 (3H, t, J=8.8Hz), 6.84 (1H, d, J=8.8Hz), 7.19 (1H, dd, J=8.3, 2.5Hz), 7.74 (1H, dd, J=8.8, 2.5Hz), 7.94 (1H, d, J=8.8Hz), 8.03 (1H, d, J=2.5Hz), 8.19 (1H, d, J=2.5Hz).

### 5) 5-(5-Cyano-2-pyridyl)-1-(6-methoxy-3-pyridyl)-1H-1,2,4-tria zole-3-carboxylic acid methyl ester

Pyridine (1.63 mL) and trifluoromethanesulfonic anhydride (2.04 mL) were added to a solution of the 5-(5-hydroxy-2-pyridyl)-1-(6-methoxy-3-pyridyl)-1H-1,2,4-tr iazole-3-carboxylic acid methyl ester (3.3 g) thus obtained in dichloromethane (50 mL) at room temperature, and the resultant mixture was stirred for 2.5 hours. A saturated aqueous solution of sodium hydrogen carbonate and dichloromethane were added to the reaction solution, and the mixture was partitioned. The organic layer was dried over anhydrous sodium sulfate. After separating the organic layer by filtration, the solvent was evaporated under reduced pressure, a residue thus obtained was dissolved in 1,2-dichloroethane (240 mL), and tetrakis(triphenylphosphine)palladium (15.85 g) and tri-n-butyltin cyanide (2.89 g) were added to the solution, which was then stirred at 80°C for 23 hours. After air cooling, an excess of potassium fluoride and methanol were added to the reaction solution, and the mixture was stirred at room temperature for 5 hours. A saturated aqueous solution of sodium hydrogen carbonate was added to the reaction solution, and the reaction solution was filtered through Celite. Then, chloroform was added to the filtrate, and the mixture was partitioned. The organic layer was dried over anhydrous sodium sulfate. After separating the organic layer by filtration, the solvent was evaporated under reduced pressure, and a residue thus obtained was purified by silica gel column chromatography (ethyl acetate-chloroform), to obtain 5-(5-cyano-2-pyridyl)-1-(6-methoxy-3-pyridyl)-1H-1,2,4-tria zole-3-carboxylic acid methyl ester (1.73 g, 56%) as a solid.

¹H-NMR (400MHz, CDCl₃) δ: 4.00 (3H, s), 4.06 (3H, s), 6.84 (1H, d, J=6.4Hz), 7.67 (1H, dd, J=8.8, 2.9Hz), 8.12 (1H, dd, J=8.3, 2.0Hz), 8.20 (1H, d, J=2.0Hz), 8.46 (1H, dd, J=8.3, 1.0Hz), 8.65-8.67 (1H, m).

### 6) Title compound

Lithium hydroxide monohydrate (290 mg) was added to a solution of the 5-(5-cyano-2-pyridyl)-1-(6-methoxy-3-pyridyl)-1H-1,2,4-tria zole-3-carboxylic acid methyl ester (1. 6 g) in tetrahydrofuran (30 mL) and water (15 mL) at room temperature, and the resultant mixture was stirred for 5.5 hours. A1 mol/L aqueous solution of hydrochloric acid was added to the reaction solution, and precipitated crystals were collected by filtration, to obtain the title compound (1.07 g, 95%) as a solid.

¹H-NMR (400MHz, DMSO-d₆) δ: 3.91 (3H, s), 6.96 (1H, d, J=8.8Hz), 7.90 (1H, dd, J=8.8, 2.5Hz), 8.29 (1H, dd, J=8.3, 1.0Hz), 8.34 (1H, d, J=2.5Hz), 8.51 (1H, dd, J=8.3, 2.0Hz), 8.86-8.88 (1H, m).

[Reference Example 3] 4-Methoxypiperidine hydrochloride

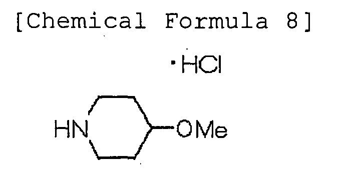

### 1) 4-Methoxypiperidine-1-carboxylic acid tert-butyl ester

Under an argon atmosphere, a solution of 4-hydroxypiperidine-1-carboxylic acid tert-butyl ester (2.00 g) in N,N-dimethylformamide (20 mL) was added dropwise to a suspension of 60% sodium hydride (0.477 g) in N,N-dimethylformamide (20 mL) at room temperature. After stirring the resultant mixture for 15 minutes, methyl iodide (0. 742 mL) was added dropwise thereto, and the mixture was stirred for 2 hours. Water and ethyl acetate were added to the reaction solution, and the resultant mixture was partitioned. The organic layer was dried over anhydrous sodium sulfate. After separating the organic layer by filtration, the solvent was evaporated under reduced pressure, and a residue thus obtained was purified by silica gel column chromatography (hexane-ethyl acetate), to obtain 4-methoxypiperidine-1-carboxylic acid tert-butyl ester (1.43 g, 67%) as an oily product.

¹H-NMR (400MHz, CDCl₃) δ: 1.39-1.54 (2H, m), 1.46 (9H, s), 1.81-1.84 (2H, m), 3.05-3.12 (2H, m), 3.31-3.39 (1H, m), 3.35 (3H, s), 3.74-3.77 (2H, m).

### 2) Title compound

A 4 mol/L hydrochloric acid-dioxane solution (10 mL) was added to a solution of the 4-methoxypiperidine-1-carboxylic acid tert-butyl ester (5.34 g) thus obtained in 1,4-dioxane (10 mL) at room temperature, and the resultant mixture was stirred for 30 minutes. A 4 mol/L hydrochloric acid-dioxane solution (20 mL) was further added thereto, and the mixture was stirred for 30 minutes. The reaction solvent was evaporated under reduced pressure, and the solid thus obtained was washed with ethyl acetate, to obtain the title compound (3.55 g).

¹H-NMR (400MHz, DMSO-d₆) δ: 1.68 (2H, m), 1.93 (2H, m), 2.91 (2H, m), 3.08 (2H, m), 3.23 (3H, s), 3.42 (1H, q, J=3.90Hz).

[Reference Example 4] 4,4-Difluoropiperidine hydrochloride

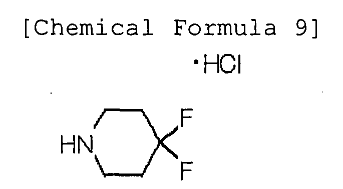

### 1) N-Benzyl-4,4-difluoropiperidine

Under an argon atmosphere, diethylaminosulfur trifluoride (8.38 mL) was added dropwise to a solution of 1-benzyl-4-piperidone (5.00 g) in benzene (200 mL) at 0°C, and the resultant mixture was stirred for 30 minutes and then heated to reflux for 18 hours. Under cooling to 0°C, a saturated aqueous solution of sodium hydrogen carbonate and ethyl acetate were added to the mixture, and the resultant mixture was partitioned. The organic layer was dried over anhydrous sodium sulfate. After separating the organic layer by filtration, the solvent was evaporated under reduced pressure, and a residue thus obtained was purified by silica gel column chromatography (hexane-ethyl acetate), to obtain N-benzyl-4,4-difluoropiperidine (4.67 g, 84%) as an oily product.

¹H-NMR (400MHz, CDCl₃) 5: 1.93-2.04 (4H, m), 2.53-2.55 (4H, m), 3.54 (2H, s), 7.24-7.34 (5H, m).
EI-MSm/z: 211 (M⁺).

### 2) Title compound

Under an argon atmosphere, 1-chloroethyl chloroformate (2.62 mL) was added dropwise to a solution of the N-benzyl-4,4-difluoropiperidine (4.66 g) in dichloromethane (93 mL) at 0°C, and then the resultant mixture was stirred at 55°C for 2 hours. After air cooling, the reaction solvent was evaporated under reduced pressure, and a solution of a residue thus obtained in methanol (93 mL) was heated to reflux for 4 hours. After air cooling, the reaction solvent was evaporated under reduced pressure, to obtain the title compound (3.03 g, 87%) as a solid.

FAB-MSm/z: 122 (M+H)+.

### [Reference Example 5]

5-(5-Fluoro-2-pyridyl)-1-(6-methoxy-3-pyridyl)-1H-1,2,4-tri azole-3-carboxylic acid sodium salt

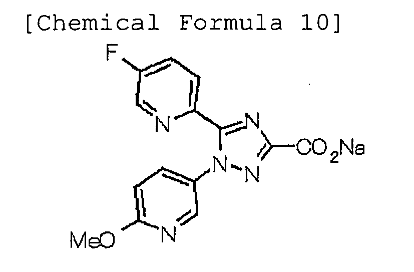

### 1) 5-Fluoropyridine-2-carbonitrile

5-Amino-2-cyanopyridine (24.5 g) was added to hydrogen fluoride-pyridine (100 mL) under ice cooling, and the resultant mixture was stirred for 10 minutes. Sodium nitrite (15.6 g) was added to the reaction solution, and the mixture was stirred at room temperature for 10 minutes, and then stirred at 50°C for 2 hours. After air cooling, a 20% aqueous solution of sodium hydroxide and diethyl ether were added to the reaction solution, and the resultant mixture was partitioned. The organic layer was dried over anhydrous sodium sulfate. After filtration, the solvent was evaporated under reduced pressure, and a residue thus obtained was purified by silica gel column chromatography (hexane-ethyl acetate), to obtain 5-fluoropyridine-2-carbonitrile (16.0 g, 64%) as a solid.

¹H-NMR (400MHz, CDCl₃) δ: 7.57 (1H, ddd, J=8.6, 8.6, 3.1Hz), 7.77 (1H, dd, J=8.6, 4.4Hz), 8.60 (1H, d, J=3.1Hz).
MS (EI) m/z: 122 (M⁺).

### 2) 2-[(5-Fluoropyridine-2-carbonyl)amino]malonic acid diethyl ester

A 6 N aqueous solution of hydrochloric acid (100 mL) containing the 5-fluoropyridine-2-carbonitrile (11.8 g) was heated to reflux for 4 hours. After air cooling, Nacl was added to the reaction solution for saturation, and ethyl acetate was further added thereto. The resultant mixture was partitioned, and the organic layer was dried over anhydrous sodium sulfate. After separating the organic layer by filtration, the solvent was evaporated under reduced pressure, and a residue thus obtained was dissolved in N,N-dimethylformamide (140mL). Then, 1-ethyl-3-(3-dimethylaminopropyl)carbodiimide hydrochloride (6.0 g), 1-hydroxybenzotriazole (770mg), and aminomalonic acid diethyl ester hydrochloride (7.2 g) were added to the solution, and the resultant mixture was stirred at room temperature for 19.5 hours. Water and ethyl acetate were added to the reaction solution, and the mixture was partitioned. The organic layer was washed with saturated brine, and then dried over anhydrous sodium sulfate. After separating the organic layer by filtration, the solvent was evaporated under reduced pressure, and a residue thus obtained was purified by silica gel column chromatography (hexane-ethyl acetate), to obtain 2-[(5-fluoropyridine-2-carbonyl)amino]malonic acid diethyl ester (9.4 g, 53%) as a solid.

¹H-NMR (400MHz, CDCl₃) δ: 1.33 (6H, t, J=7.1Hz), 4.27-4.38 (4H, m), 5.36 (1H, d, J=7.4Hz), 7.51-7.56 (1H, m), 8.20-8.21 (1H, m), 8.46 (1H, d, J=2.7Hz), 8.74 (1H, d, J=10.0Hz).

### 3) Title compound

A solution of sodium nitrite (3.63 g) in water (7.5 mL) was added dropwise to a mixed solution of 5-amino-2-methoxypyridine (3.23 g) in acetic acid (21 mL) and concentrated hydrochloric acid (5.2mL) at 0°C, and the resultant mixture was stirred for 15 minutes. Under cooling the reaction solution to -15°C, a solution of the 2-[(5-fluoropyridine-2-carbonyl)amino]malonic acid diethyl ester (9.3 g) obtained above in acetone (30 mL), and a solution of potassium carbonate (17.2 g) in water (30 mL) were added slowly to the reaction solution, and the resultant mixture was stirred for 2.5 hours Under cooling to 0°C. Ethyl acetate was added to the reaction solution, and the mixture was partitioned. The organic layer was washed sequentially with water, a saturated aqueous solution of sodium hydrogen carbonate, water and saturated brine, and then was dried over anhydrous sodium sulfate. After separating the organic layer by filtration, the solvent was evaporated under reduced pressure, and a residue thus obtained was dissolved in methanol (150 mL). Sodium methoxide (170 mg) was added to this reaction solution, and the mixture was stirred at room temperature for 60 hours. The reaction solvent was evaporated under reduced pressure, and the solid thus obtained was washed with diethyl ether, to obtain the title compound (9.5 g, 93%).

¹H-NMR (400MHz, DMSO-d₆) δ: 3.83 (3H, s), 7.07 (1H, d, J=8.8Hz), 7.63-7.67 (1H, m), 7.75-7.79 (3H, m), 8.03-8.05 (2H, m).

### [Reference Example 6]

1-(6-Methoxy-3-pyridyl)-5-(5-methyl-2-pyridyl)-1H-1,2,4-tri azole-3-carboxylic acid

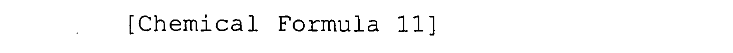

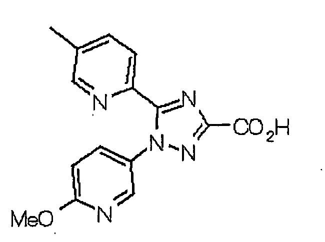

### 1) 2-[(5-Methylpyridine-2-carbonyl)amino]malonic acid diethyl ester

1-Ethyl-3-(3-dimethylaminopropyl)carbodiimide hydrochloride (10.15 g), 1-hydroxybenzotriazole (650 mg), aminomalonic acid diethyl ester hydrochloride (12.22 g), and triethylamine (20.1 mL) were added to a solution of 5-methylpyridine-2-carboxylic acid (6.6 g) in N,N-dimethylformamide (240 mL), and the resultant mixture was stirred at room temperature for 30.5 hours. Water and chloroform were added to the reaction solution, and the mixture was partitioned. The organic layer was dried over anhydrous sodium sulfate. After separating the organic layer by filtration, the solvent was evaporated under reduced pressure, and a residue thus obtained was purified by silica gel column chromatography (hexane-ethyl acetate), to obtain 2-[(5-methylpyridine-2-carbonyl)amino]malonic acid diethyl ester (7.0 g, 49%) as an oily product.

¹H-NMR (400MHz, CDCl₃) δ: 1.32 (6H, t, J=7.1Hz), 2.41 (3H, s), 4.29-4.34 (4H, m), 5.38 (1H, d, J=7.4Hz), 7.64 (1H, d, J=7.8Hz), 8.02-8.04 (8H, m), 8.43 (1H, s), 8.85 (1H, d, J=7.4Hz).

### 2) Title compound

A solution of sodium nitrite (2.77 g) in water (9.8 mL) was added dropwise to a mixed solution of 5-amino-2-methoxypyridine (3.42 g) in acetic acid (16 mL) and concentrated hydrochloric acid (4 mL) at 0°C, and the resultant mixture was stirred for 15 minutes. Under cooling the reaction solution to -15°C, a solution of 2-[(5-methylpyridine-2-carbonyl)amino]malonic acid diethyl ester (10 g) in acetone (90 mL), and a solution of potassium carbonate (54.7 g) in water (80 mL) were slowly added to the reaction solution, and the resultant mixture was stirred for 3.5 hours at 0°C. Ethyl acetate was added to the reaction solution, and the mixture was partitioned. The organic layer was washed sequentially with water, a saturated aqueous solution of sodium hydrogen carbonate, water and saturated brine, and then was dried over anhydrous sodium sulfate. After separating the organic layer by filtration, the solvent was evaporated under reduced pressure, then sodium methoxide (130 mg) was added to a solution of a residue thus obtained in methanol (100 mL) at roomtemperature, and the mixture was stirred at room temperature for 13 hours. The reaction solvent was evaporated under reduced pressure, then lithium hydroxide monohydrate (1.03 g) was added to a solution of the solid thus obtained in tetrahydrofuran (120 mL) and water (120 mL), and the resultant mixture was stirred at room temperature for 1.5 hours. A 1 mol/L aqueous solution of hydrochloric acid was added to the reaction solution to acidify the solution, and the resultant mixture was extracted with a mixed solvent of chloroform-methanol (10:1). The organic layer was dried over anhydrous sodium sulfate. After separating the organic layer by filtration, the solvent was evaporated under reduced pressure, and the solid thus obtained was washed with diethyl ether, to obtain the title compound (1.58 g, 21%).

¹H-NMR (400MHz, DMSO-d₆) δ: 2.31 (3H, s), 3.91 (3H, s), 6.93 (1H, d, J=8.8Hz), 7.80-7.86 (2H, m), 7.99 (1H, d, J=7.8Hz), 8.26-8.29 (2H, m).

### [Reference Example 7]

5-(5-Chloro-2-pyridyl)-1-(6-methoxy-3-pyridyl)-1H-1,2,4-tri azole-3-carboxylic acid

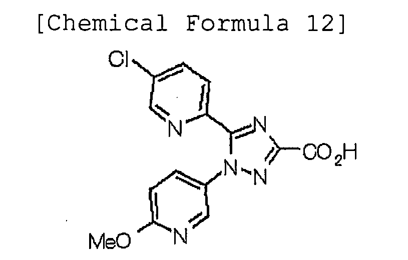

### 1) Pyridine-2,5-dicarboxylic acid dibenzyl ester

Thionyl chloride (250 mL) and N, N-dimethylformamide (10 mL) were added to a solution of 2, 5-pyridinedicarboxylic acid (60 g) in dichloromethane (360 mL), and the resultant mixture was heated to reflux for 5 hours. After air cooling, the solvent of the reaction solution was evaporated under reduced pressure, and toluene was added to the residue thus obtained. The resultant mixture was further azeotropically evaporated under reduced pressure, and a residue thus obtained was dissolved in dichloromethane (500 mL). A solution of benzyl alcohol (81.7 mL) in dichloromethane (200 mL) was added dropwise to the solution at 0°C, and the mixture was stirred at room temperature for 4 hours. Water was added to the reaction solution, and the mixture was partitioned. The organic layer was washed with a saturated aqueous solution of sodiumhydrogen carbonate, and then was dried over anhydrous sodium sulfate. After separating the organic layer by filtration, the solvent was evaporated under reduced pressure, to obtain pyridine-2,5-dicarboxylic acid dibenzyl ester (65 g, 52%) as a solid.

¹H-NMR (400MHz, CDCl₃) δ: 5.42 (2H, s), 5.47 (2H, s), 7.38-7.46 (10H, m), 8.19 (1H, d, J=8.3Hz), 8.44 (1H, dd, J=8.2, 2.1Hz), 9.35-9.36 (1H, m).

### 2) Pyridine-2,5-dicarboxylic acid 5-benzyl ester

Copper (II) sulfatepentahydrate (46. 7 g) was added to a suspension of pyridine-2, 5-dicarboxylic acid dibenzyl ester (65 g) in methanol (500 mL), and the resultant mixture was heated to reflux for 1 hour. After air cooling, a precipitate was collected by filtration. This solid was suspended in dioxane, and hydrogen sulfide gas was blown into the suspension at room temperature. The reaction solution was filtered, and the filtrate solvent was evaporatedunder reducedpressure, to obtain pyridine-2, 5-dicarboxylic acid 5-benzyl ester (48.1 g, 74%) as a solid.

¹H-NMR (400MHz, DMSO-d₆) δ: 5.40 (2H, s), 7.29-7.42 (3H, m), 7.49-7.50 (2H, m), 8.20 (1H, br s), 8.47 (1H, d, J=7.8Hz), 9.19 (1H, br s), 10.64 (1H, br s).

### 3) 2-[(5-Benzyloxycarbonylpyridine-2-carbonyl)amino]malonic acid diethyl ester

Thionyl chloride (50 mL) and N,N-dimethylformamide (12 mL) were added to a solution of pyridine-2, 5-dicarboxylic acid 5-benzyl ester(48.1 g) in dichloromethane (360 mL), and the resultant mixture was heated to reflux for 3 hours. After air cooling, the reaction solvent was evaporated under reduced pressure, then toluene was added to the residue thus obtained, and the mixture was further azeotropically evaporated under reduced pressure. To a solution of a residue thus obtained in dichloromethane (500 mL), aminomalonic acid diethyl ester hydrochloride (47.49 g) was added at 0°C, and the mixture was stirred at room temperature for 39 hours. Water was added to the reaction solution, and the mixture was partitioned. The organic layer was washed with a saturated aqueous solution of sodium hydrogen carbonate, and then was dried over anhydrous sodium sulfate. After separating the organic layer by filtration, the solvent was evaporated under reduced pressure, and a residue thus obtained was purified by silica gel column chromatography (hexane-ethyl acetate), to obtain 2-[(5-benzyloxycarbonylpyridine-2-carbonyl)amino]malonic acid diethyl ester (22.5 g, 29%) as an oily product.

¹H-NMR (400MHz, CDCl₃) δ: 1.32 (6H, t, J=7.1Hz), 4.30-4.33 (4H, m), 5. 37 (1H, d, J=7.4Hz), 5. 42 (2H, s), 7.37-7.47 (5H, m), 8.23 (1H, d, J=8.1Hz), 8.47 (1H, dd, J=8.1, 1.7Hz), 8.92 (1H, d, J=10.0Hz), 9.24 (1H, d, J=2.0Hz).

### 4) 2-[(5-Carboxypyridine-2-carbonyl)amino]malonic acid diethyl ester

10% Palladium on carbon (2.2 g) was added to a solution of 2-[(5-benzyloxycarbonylpyridine-2-carbonyl)amino]malonic acid diethyl ester (22. 0 g) in dioxane (250 mL), and the resultant mixture was stirred under a hydrogen atmosphere at room temperature for 76 hours. The catalyst was separated by filtration, and the filtrate solvent was evaporated under reduced pressure, to obtain 2-[(5-carboxypyridine-2-carbonyl)amino]malonic acid diethyl ester (17.0 g, quantitative).

¹H-NMR (400MHz, CDCl₃) δ: 1.33 (6H, t, J=7.1Hz), 4.31-4.35 (4H, m), 5.40 (1H, d, J=7.6Hz), 8.27 (1H, d, J=8.1Hz), 8.49 (1H, dd, J=8.1, 2.0Hz), 8.96 (1H, d, J=7.6Hz), 9.23-9.24 (1H, m).

### 5) 2-[(5-Benzyloxycarbonylaminopyridine-2-carbonyl)amino]malon ic acid diethyl ester

Triethylamine (5.43 mL), diphenylphosphorylazide (8.4 mL) and benzyl alcohol (7.68 mL) were added to a solution of 2-[(5-carboxypyridine-2-carbonyl)amino]malonic acid diethyl ester (12 g) in dioxane (70 mL), and the resultant mixture was heated to reflux for 14.5 hours. After air cooling, the reaction solvent was evaporated under reduced pressure, then water and chloroform were added to the residue thus obtained, and the mixture was partitioned. The organic layer was dried over anhydrous sodium sulfate. After separating the organic layer by filtration, the solvent was evaporated under reduced pressure, and a residue thus obtained was purified by silica gel column chromatography (hexane-ethyl acetate), to obtain 2-[(5-benzyloxycarbonylaminopyridine-2-carbonyl)amino]malon ic acid diethyl ester (14.4 g, 91%) as a solid.

¹H-NMR (400MHz, CDCl₃) δ: 1.32 (6H, t, J=7.1Hz), 4.26-4.34 (4H, m), 5.23 (2H, s), 5.37 (1H, d, J=7.6Hz), 7.27-7.40 (5H, m), 8.10 (2H, s), 8.53 (1H, s), 8.73 (1H, d, J=7.6Hz).

### 6) 2-[(5-Aminopyridine-2-carbonyl)amino]malonic acid diethyl ester

10% Palladium on carbon (1.4 g) was added to a solution of 2-[(5-benzyloxycarbonylaminopyridine-2-carbonyl)amino]malon ic acid diethyl ester (14.4 g) in dioxane (200 mL), and the resultant mixture was stirred under a hydrogen atmosphere at room temperature for 14 hours. After separating the organic layer by filtration, the filtrate solvent was evaporated under reduced pressure, and a residue thus obtained was purified by silica gel column chromatography (hexane-ethyl acetate), to obtain 2-[(5-aminopyridine-2-carbonyl)amino]malonic acid diethyl ester (11.5 g, 80%) as an oily product.

¹H-NMR (400MHz, CDCl₃) 5: 1.31 (6H, t, J=7.1Hz), 4.04 (2H, s), 4.26-4.34 (4H, m), 5.37 (1H, d, J=7.6Hz), 7.00 (1H, dd, J=8.3, 2.7Hz), 7.95 (1H, d, J=8.6Hz), 8.01 (1H, d, J=2.7Hz), 8.64 (1H, d, J=10.0Hz).

### 7) Title compound

tert-Butyl nitrite (930µL) and copper (II) chloride (750 mg) were added to a solution of 2-[(5-aminopyridine-2-carbonyl)amino]malonic acid diethyl ester (2. 0 g) in acetonitrile (65 mL), and the resultant mixture was stirred at 65°C for 20 minutes. After air cooling, a 1 mol/L aqueous solution of hydrochloric acid and chloroform were added thereto, and the mixture was partitioned. The organic layer was dried over anhydrous sodium sulfate. After separating the organic layer by filtration, the solvent was evaporated under reduced pressure, and a residue thus obtained was purified by silica gel column chromatography (hexane-ethyl acetate), to obtain 2-[(5-chloropyridine-2-carbonyl)amino]malonic acid diethyl ester. A solution of sodium nitrite (780 mg) in water (2.5 mL) was added dropwise to a solution of 5-amino-2-methoxypyridine (690 mg) in acetic acid (4.4 mL) and concentrated hydrochloric acid (1.1mL) at 0°C, and the resultant mixture was stirred for 15 minutes. Under cooling to -15°C, a solution of 2-[(5-chloropyridine-2-carbonyl)amino]malonic acid diethyl ester (2.1 g) in acetone (10 mL), and a solution of potassium carbonate (3.69 g) in water (10 mL) were slowly added to the reaction solution, and the mixture was stirred at 0°C for 1.5 hours. Ethyl acetate was added to the reaction solution, and the mixture was partitioned. The organic layer was washed sequentially with water, a saturated aqueous solution of sodium hydrogen carbonate, water and saturated brine, and then dried over anhydrous sodium sulfate. After separating the organic layer by filtration, the solvent was evaporated under reduced pressure, and the residue was dissolved in methanol (50 mL). Sodium methoxide (40 mg) was added to the solution at room temperature, and the mixture was stirred for 13 hours. The reaction solvent was evaporated under reduced pressure, and 1 mol/L hydrochloric acid was added to a residue thus obtained to acidify the residue. Ethyl acetate and Nacl were added to the residue, and the mixture was partitioned. The organic layer was dried over anhydrous sodium sulfate. After separating the organic layer by filtration, the solvent was evaporated under reduced pressure, and the solid thus obtained was washed with diethyl ether, to obtain the title compound (429 mg, 15%).

¹H-NMR (400MHz, DMSO-d₆) δ: 3.92 (3H, s), 6.96 (1H, d, J=8.8Hz), 7.89 (1H, dd, J=8.8, 2.7Hz), 8.15 (3H, t, J=1.7Hz), 8.33 (1H, d, J=2.2Hz), 8.52 (1H, dd, J=1.1, 0.5Hz).

### [Reference Example 8]

1-(6-Methoxy-3-pyridyl)-5-(5-methyl-2-pyrazinyl)-1H-1,2,4-t riazole-3-carboxylic acid

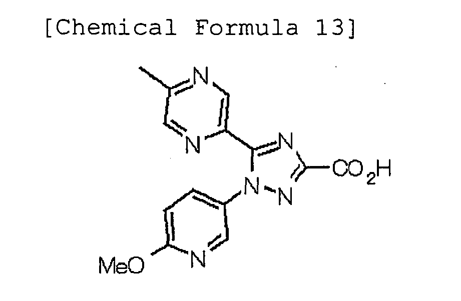

### 1) 2-[(5-Methylpyrazine-2-carbonyl)amino]malonic acid diethyl ester

2-[(5-Methylpyrazine-2-carbonyl)amino]malonic acid diethyl ester (76 g, 86%) was obtained as an oily product by the same method as in Reference Example 6-(1), using 5-methylpyrazine-2-carboxylic acid (41.44 g) and aminomalonic acid diethyl ester hydrochloride (920 mg).

¹H-NMR (400MHz, CDCl₃) 5: 1.33 (6H, t, J=7.1Hz), 2.67 (3H, s), 4.27-4.36 (4H, m), 5.37 (1H, d, J=7.4Hz), 8.45-8.47 (1H, m), 8.63 (1H, d, J=7.1Hz), 9.24 (1H, d, J=1.5Hz).

### 2) Title compound

A sodium nitrite (6.1 g) in water (22 mL) was slowly added dropwise to a mixed solution of 5-amino-2-methoxypyridine (7.53 g) in acetic acid (35 mL) and concentrated hydrochloric acid (8.7 mL) at 0°C, and the resultant mixture was stirred for 15 minutes. Under cooling the reaction solution to -15°C, a solution of 2-[(5-methylpyrazine-2-carbonyl)amino]malonic acid diethyl ester (14 g) in acetone (88 mL), and a solution of potassium carbonate (28.9 g) in water (88 mL) were slowly added to the reaction solution, and the mixture was stirred at 0°C for 3.5 hours. Water and ethyl acetate were added to the reaction solution, and the mixture was partitioned. The organic layer was washed sequentially with a saturated aqueous solution of sodium hydrogen carbonate, water and saturated brine, and then was dried over anhydrous sodium sulfate. After separating the organic layer by filtration, the solvent was evaporated under reduced pressure, and a residue thus obtained was dissolved in methanol (200 mL). To this solution, sodium methoxide (356 mg) was added at room temperature, and the mixture was stirred for 14 hours. The reaction solvent was evaporated under reduced pressure, and to the residue thus obtained, ethyl acetate, a 1 mol/L aqueous solution of hydrochloric acid, and Nacl were added, and the resultant mixture was partitioned. The organic layer was dried over anhydrous sodium sulfate. After separating the organic layer by filtration, the solvent was evaporated under reduced pressure, and the crude solid thus obtained was washed with diethyl ether, to obtain a mixture of 1-(6-methoxy-3-pyridyl)-5-(5-methyl-2-pyrazinyl)-1H-1,2,4-t riazole-3-carboxylic acid ethyl ester and 1-(6-methoxy-3-pyridyl)-5-(5-methyl-2-pyrazinyl)-1H-1,2,4-t riazole-3-carboxylic acid (3.26g). This mixture was dissolved in 1,4-dioxane (100mL) and water (100mL), then lithium hydroxide monohydrate (840 mg) was added to the reaction solution, and the mixture was stirred at room temperature for 1 hour. A 1 mol/L aqueous solution of hydrochloric acid was added to the reaction solution at 0°C to acidify the solution, and a solid precipitated therefrom was collected by filtration, to obtain the title compound (2.78 g, 89%).

¹H-NMR (400MHz, DMSO-d₆) δ: 2.49 (3H, s), 3.87 (3H, s), 6.91 (1H, d, J=8.8Hz), 7.86 (1H, dd, J=8.8, 2.9Hz), 8.30 (1H, d, J=2.0Hz), 8.39 (1H, s), 9.10 (1H, s).

### [Reference Example 9]

5-(5-tert-Butoxycarbonylamino-2-pyridyl)-1-(6-methoxy-3-pyr idyl)-1H-1,2,4-triazole-3-carboxylic acid

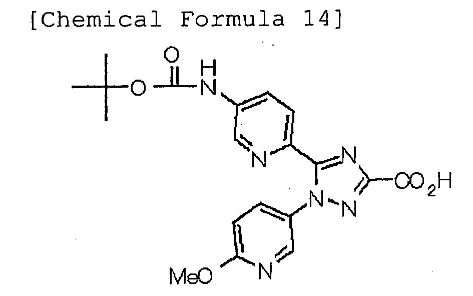

### 1) 2-[(5-tert-Butoxycarbonylaminopyridine-2-carbonyl)amino]mal onic acid diethyl ester

To a solution of 2-[(5-benzyloxycarbonylpyridine-2-carbonyl)amino]malonic acid diethyl ester (7. 0 g) of Reference Example 7-(3) in 1,4-dioxane (40 mL), triethylamine (3.17 mL), diphenylphosphorylazide (4.9mL) and tert-butanol (4.25 mL) were added, and the resultant mixture was heated to reflux for 14.5 hours. After air cooling, the reaction solvent was evaporated under reduced pressure, then water and chloroform were added to the residue thus obtained, and the mixture was partitioned. The organic layer was dried over anhydrous sodium sulfate. After separating the organic layer by filtration, the solvent was evaporated under reduced pressure, and a residue thus obtained was purified by silica gel column chromatography(ethyl acetate-hexane), to obtain 2-[(5-tert-butoxycarbonylaminopyridine-2-carbonyl)amino]mal onic acid diethyl ester (3.0 g, 35%) as a solid.

¹H-NMR (400MHz, CDCl₃) 5: 1.32 (6H, t, J=7.1Hz), 1.54 (9H, s), 4.25-4.35 (4H, m), 5.38 (1H, d, J=7.4Hz), 6.74 (1H, s), 8.10 (2H, s), 8.43 (1H, t, J=1.6Hz), 8.72 (1H, d, J=7.4Hz).

### 2) Title compound

A solution of sodium nitrite (710 mg) in water (2.5 mL) was added dropwise to a mixed solution of 3-amino-6-methoxypyridine (630 mg) in acetic acid (4 mL) and concentrated hydrochloric acid (1 mL) at 0°C, and the resultant mixture was stirred for 15 minutes. Under cooling the reaction solution to -15°C, a solution of 2-[(5-tert-butoxycarbonylaminopyridine-2-carbonyl)amino]mal onic acid diethyl ester (2.4 g) in acetone (10 mL), and a solution of potassium carbonate (3.36 g) in water (10 mL) were slowly added to the reaction solution, and the mixture was stirred at 0°C for 3 hours. Ethyl acetate was added to the reaction solution, and the mixture was partitioned. The organic layer was washed with water, a saturated aqueous solution of sodium hydrogen carbonate, water and saturated brine, and then was dried over anhydrous sodium sulfate. After separating the organic layer by filtration, the solvent was evaporated under reduced pressure, and a residue thus obtained was dissolved in methanol (50 mL). To this reaction solution, sodium methoxide (34 mg) was added at room temperature, and the mixture was stirred for 13.5 hours. The reaction solvent was evaporated under reduced pressure, then methanol (20mL) anda 1 mol/L aqueous solution of sodium hydroxide (20 mL) were added to the residue thus obtained, and the mixture was stirred at room temperature for 1 hour. A 1 mol/L aqueous solution of hydrochloric acid was added to the reaction solution to acidify the solution, and a solid precipitated therefrom was collected by filtration, to obtain the title compound (1.27 g, 51%) as a solid.

¹H-NMR (400MHz, DMSO-d₆) δ: 1.48 (9H, s), 3.93 (3H, s), 6.95 (1H, d, J=8.8Hz), 7.86 (1H, dd, J=8.8, 2.7Hz), 8.04-8.11 (2H, m), 8.30 (1H, d, J=2.7Hz), 8.39 (1H, d, J=2.2Hz), 9.87 (1H, s).

### [Reference Example 10]

1-(3-Pyridyl)-5-(2-pyridyl)-1H-1,2,4-triazole-3-carboxylic acid

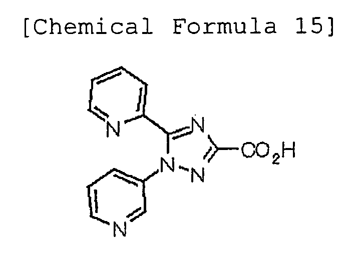

A solution of sodium nitrite (1.38 g) in water (5 mL) was slowly added dropwise to a mixed solution of 3-aminopyridine (1.23 g) in acetic acid (7.9 mL) and concentrated hydrochloric acid (2 mL) at 0°C, and then the resultant mixture was stirred for 15 minutes. Under cooling the reaction solution to -15°C, a solution of 2-[(pyridine-2-carbonyl)amino]malonic acid dimethyl ester (3.0 g) of Reference Example 1-(1) in acetone (20 mL), and a solution of potassium carbonate (4.93 g) in water (20 mL) were slowly added to the reaction solution, and the mixture was stirred at 0°C for 3.5 hours. Ethyl acetate was added to the reaction solution, and the mixture was partitioned. The organic layer was washed with water, a saturated aqueous solution of sodium hydrogen carbonate, water and saturated brine, and was dried over anhydrous sodium sulfate. After separating the organic layer by filtration, the solvent was evaporated under reduced pressure, and a residue thus obtained was dissolved in methanol (50 mL). Sodium methoxide (71 mg) was added to this reaction solution at room temperature, and the mixture was stirred for 13 hours. The reaction solvent was evaporated under reduced pressure, and a 1 mol/L aqueous solution of hydrochloric acid and Nacl were added to the residue thus obtained. The resultant mixture was extracted with ethyl acetate, and the organic layer was dried over anhydrous sodium sulfate. After separating the organic layer by filtration, the solvent was evaporated under reduced pressure, to obtain the title compound (3.05 g, 96%) as a solid.

¹H-NMR (400MHz, DMSO-d₆) δ: 7.94-7.47 (1H, m), 7.53 (1H, dd, J=8.3, 4.7Hz), 7.88-7.90 (1H, m), 7.99-8.04 (1H, m), 8.25 (1H, d, J=8.1Hz), 8.36 (1H, d, J=4.2Hz), 8.61-8.63 (2H, m).

[Reference Example 11] 2-Amino-1-fluoro-2-methylpropane hydrochloride

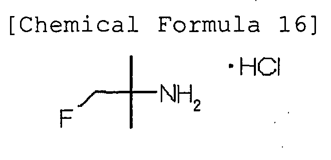

### 1) N-Benzyl-2-amino-2-methyl-1-propanol

A solution of 2-amino-2-methyl-1-propanol (10.0 g), benzaldehyde (11.98 mL) and p-toluenesulfonic acid (10 mg) in benzene (300mL) was heated to reflux for 4 hours using a Dean-Stark dehydrating apparatus. After air cooling, the reaction solvent was evaporated under reduced pressure, and a residue thus obtained was dissolved in methanol (200 mL). Under ice cooling, sodium cyanoborohydride (8. 89 g) was added to the solution, and the mixture was stirred for 1. 5 hours. The reaction solvent was evaporated under reduced pressure, then a saturated aqueous solution of sodium hydrogen carbonate and ethyl acetate were added to the residue thus obtained, and the resultant mixture was partitioned. The organic layer was washed with saturated brine, and then was dried over anhydrous magnesium sulfate. After separating the organic layer by filtration, the solvent was evaporated under reduced pressure, and a residue thus obtained was purified by silica gel column chromatography (dichloromethane-methanol-aqueous ammonia), to obtain N-benzyl-2-amino-2-methyl-1-propanol (10. 36 g, 52%) as a solid.

¹H-NMR (400MHz, CDCl₃) δ: 1.15 (6H, s), 1.86 (2H, br s), 3.35 (2H, s), 3.68 (2H, s), 7.30 (5H, s).

### 2) 3-Benzyl-4,4-dimethyl-1,2,3-oxathiazole-2-oxide

Under cooling to -20°C, to a solution of N-benzyl-2-amino-2-methyl-1-propanol (3.32 g) and diisopropylethylamine (12.6 mL) in dichloromethane (50 mL), a solution of thionyl chloride (1.49 mL) in dichloromethane (5 mL) was added dropwise over 7 minutes, and then the resultant mixture was stirred for 45 minutes. The reaction solvent was evaporated under reduced pressure, and a residue thus obtained was purified by silica gel column chromatography (hexane-ethyl acetate), to obtain 3-benzyl-4,4-dimethyl-1,2,3-oxathiazole-2-oxide (3.91g, 52%) as a solid.

¹H-NMR (400MHz, CDCl₃) δ: 1.24 (3H, s), 1.45 (3H, s), 4.15 (1H, d, J=14.6Hz), 4.20 (1H, d, J=8.1Hz), 4.27 (1H, d, J=14.6Hz), 4.64 (1H, d, J=8.3Hz), 7.26-7.42 (5H, m).

### 3) 3-Benzyl-4,4-dimethyl-1,2,3-oxathiazole-2-dioxide

Sodium periodate (2. 73 g) was added to a mixed solution of 3-benzyl-4,4-dimethyl-1,2,3-oxathiazole-2-oxide (1.92 g) and ruthenium chloride hydrate (5 mg) in acetonitrile (30 mL) and water (30 mL) at room temperature, and the resultant mixture was stirred for 3 days. Water and diethyl ether were added to the reaction solution, and the mixture was partitioned. The organic layer was washed with saturated brine, and then was dried over anhydrous sodium sulfate. After separating the organic layer by filtration, the solvent was evaporated under reduced pressure, and a residue thus obtained was purified by silica gel column chromatography (hexane-ethyl acetate), to obtain 3-benzyl-4,4-dimethyl-1,2,3-oxathiazole-2-dioxide (1.934 g, 94%) as an oily product.

¹H-NMR (400MHz, CDCl₃) δ: 1.30 (6H, s), 4.26 (4H, d, J=1.2Hz), 7.26-7.44 (5H, m).

### 4) N-Benzyl-2-amino-1-fluoro-2-methylpropane

Tetrabutylammonium fluoride (a 1.0 mol/L tetrahydrofuran solution, 15.8 mL) was added to a solution of 3-benzyl-4,4-dimethyl-1,2,3-oxathiazole-2-dioxide (1.91g)in tetrahydrofuran (10 mL) at room temperature, and the resultant mixture was stirred for 3 hours. The reaction solution was evaporated under reduced pressure, and a residue thus obtained was dissolved in diethyl ether (30 mL). To this reaction solution, a 20% aqueous solution (10 mL) of sulfuric acid was added at room temperature, and the mixture was stirred overnight. Under cooling the reaction solution with ice, sodium hydrogen carbonate was added in small portions to the reaction solution to neutralize the reaction solution, and the resultant mixture was extracted with diethyl ether. The organic layer was washed with saturated brine, and then was dried over anhydrous sodium sulfate. After separating the organic layer by filtration, the solvent was evaporated under reduced pressure, and a residue thus obtained was purified by silica gel column chromatography (hexane-ethyl acetate), to obtain N-benzyl-2-amino-1-fluoro-2-methylpropane (700 mg, 49%) as an oily product.

¹H-NMR (400MH, CDCl₃) δ: 1.16 (6H, d, J=2.0Hz), 1.39 (1H, s), 3.73 (2H, s), 4.26 (2H, d, J=47.9Hz), 7.21-7.37 (5H, m).

### 5) Title compound

10% Palladium on carbon (50 mg) and concentrated hydrochloric acid (1 mL) were added to a solution of N-benzyl-2-amino-1-fluoro-2-methylpropane (690mg) in methanol (20 mL), and the resultant mixture was stirred overnight under a hydrogen atmosphere (3.5 atmospheres) at room temperature. To the reaction solution, 10% palladium-carbon (100 mg) was further added, and the mixture was stirred for 6.5 hours under a hydrogen atmosphere (4.0 atmospheres) at 50°C. After air cooling, the reaction solution was filtered using Celite, and the filtrate solvent was evaporated under reduced pressure, to obtain the title compound (506 mg, quantitative) as a solid.

¹H-NMR (400MHz, CD₃OD) δ: 1.37 (6H, s), 4.42 (2H, d, J=37.2Hz).

### [Reference Example 12]

5-(5-Methyl-2-pyridyl)-1-(3-pyridyl)-1H-1,2,4-triazole-3-ca rboxylic acid

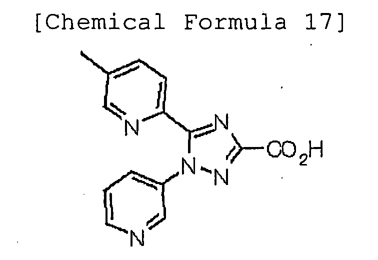

A solution of sodium nitrite (2.09 g) in water (4.5 mL) was added dropwise to a mixed solution of 3-aminopyridine (1.86 g) in acetic acid (12 mL) and concentrated hydrochloric acid (3mL) at 0°C, and the resultant mixture was stirred for 15 minutes. Under cooling the reaction solution to -15°C, a solution of 2-[(5-methylpyridine-2-carbonyl)amino]malonic acid diethyl ester (5.3 g) of Reference Example 6-(1) in acetone (18 mL), and a solution of potassium carbonate (7.46 g) in water (18 mL) were slowly added to the reaction solution, and the mixture was stirred at 0°C for 3 hours. Ethyl acetate was added to the reaction solution, and the mixture was partitioned. The organic layer was washed sequentially with water, a saturated aqueous solution of sodium hydrogen carbonate, water and saturated brine, and then was dried over anhydrous sodium sulfate. After separating the organic layer by filtration, the solvent was evaporated under reduced pressure, and a residue thus obtained was dissolved in methanol (50 mL). To this reaction solution, sodium methoxide (100 mg) was added, then a 1 mol/L aqueous solution of sodium hydroxide was further added, and the mixture was stirred overnight. A 1 mol/L aqueous solution of hydrochloric acid was added to the reaction solution at 0°C to acidify the solution, then chloroform-methanol (10/1) was added, and the mixture was partitioned. The organic layer was dried over anhydrous sodium sulfate. After separating the organic layer by filtration, the solvent was evaporated under reduced pressure, to obtain the title compound (1.04 g, 21%) as a solid.

¹H-NMR (400MHz, DMSO-d₆) 5: 2.30 (3H, s), 7.54-7.57 (1H, m), 7.82 (1H, dd, J=8.3, 1.7Hz), 7.95 (1H, dq, J=8.1, 1.3Hz), 8.02 (1H, d, J=8.1Hz), 8.20 (1H, d, J=2.0Hz), 8.66 (2H, dt, J=7.8, 2.8Hz).

### [Reference Example 13]

1-(6-Methoxy-3-pyridyl)-5-(2-pyrazinyl)-1H-1,2,4-triazole-3 -carboxylic acid

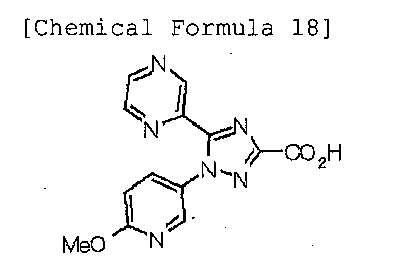

### 1) 2-[(Pyrazine-2-carbonyl)amino]malonic acid diethyl ester

To a solution of pyrazine-2-carboxylic acid (24.82 g) in N,N-dimethylformamide (400 mL), triethylamine (83.6 mL), 1-ethyl-3-(3-dimethylaminopropyl)carbodiimide hydrochloride (42.2 g), 1-hydroxybenzotriazole (2.7 g) and aminomalonic acid diethyl ester hydrochloride (50.8 g) were added, and the resultant mixture was stirred at room temperature for 15.5 hours. Water and ethyl acetate were added to the reaction solution, and the mixture was partitioned. The organic layer was washed with saturated brine, and then was dried over anhydrous sodium sulfate. After separating the organic layer by filtration, the solvent was evaporated under reduced pressure, and a residue thus obtained was purified by silica gel column chromatography (hexane-ethyl acetate), to obtain 2-[(pyrazine-2-carbonyl)amino]malonic acid diethylester(51.2 g, 91%) as an oily product.

### 2) 1-(6-Methoxy-3-pyridyl)-5-(2-pyrazinyl)-1H-1,2,4-triazole-3 -carboxylic acid methyl ester

A solution of sodium nitrite (21.2 g) in water (70 mL) was added dropwise to a mixed solution of 3-amino-5-methoxypyridine (26.2 g) in acetic acid (121 mL) and concentrated hydrochloric acid (30 mL) at 0°C, and the resultant mixture was stirred for 15 minutes. Under cooling the reaction solution to -15°C, a solution of the 2-[(pyrazine-2-carbonyl)amino]malonic acid diethyl ester (51. 2 g) obtained above in acetone (280mL), and a solution of potassium carbonate (100. 64 g) in water (280 mL) were slowly added to the reaction solution, and the mixture was stirred at 0°C for 4 hours. Ethyl acetate was added to the reaction solution to extract the reaction solution, and the organic layer was washed sequentially with water, a saturated aqueous solution of sodium hydrogen carbonate, water and saturated brine, and then was dried over anhydrous sodium sulfate. After separating the organic layer by filtration, the solvent was evaporated under reduced pressure, and a residue thus obtained was dissolved in methanol (500 mL). Sodium methoxide was added to the reaction solution at room temperature, and the mixture was stirred for 60.5 hours. The solvent of the reaction solution was evaporated under reduced pressure, to obtain 1-(6-methoxy-3-pyridyl)-5-(2-pyrazinyl)-1H-1,2,4-triazole-3 -carboxylic acid methyl ester (28.5 g, 50%) as a solid.

¹H-NMR (400MHz, CDCl₃) δ: 4.00 (3H, s), 4.08 (3H, s), 6.84 (1H, dd, J=8.8, 0.5Hz), 7.69 (1H, dd, J=8.8, 2.9Hz), 8.23 (1H, dd, J=2.7, 0.5Hz), 8.41 (1H, q, J=1.3Hz), 8.64 (1H, d, J=2.5Hz), 9.49 (1H, d, J=1.5Hz).

### 3) Title compound

Lithium hydroxide monohydrate (7.61 g) was added to a solution of 1-(6-methoxy-3-pyridyl)-5-(2-pyrazinyl)-1H-1,2,4-triazole-3 -carboxylic acid methyl ester (28.3 g) in 1,4-dioxane (180 mL) and water (180 mL) at room temperature, and the resultant mixture was stirred for 2.5 hours. A 1 mol/L aqueous solution of hydrochloric acid was added to the reaction solution to acidify the solution, and a solid precipitated therefrom was collected by filtration, to obtain the title compound (5.3 g, 20%).

¹H-NMR (400MHz, DMSO-d₆) δ: 3.91 (3H, s), 6.96 (1H, d, J=8.8Hz), 7.92 (1H, dd, J=8.8, 2.7Hz), 8.36 (1H, dd, J=2.7, 0.5Hz), 8.53 (1H, q, J=1.3Hz), 8.74 (1H, d, J=2.7Hz), 9.28 (1H, d, J=1.5Hz).

### [Reference Example 14]

1-(6-Methoxy-3-pyridyl)-5-(1-methyl-1H-pyrazol-3-yl)-1H-1,2 ,4-triazole-3-carboxylic acid

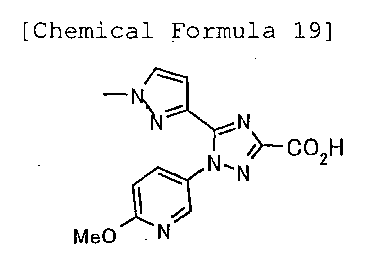

### 1) 2-[(1-Methyl-1H-pyrazole-3-carbonyl)amino]malonic acid diethyl ester

2-[(1-Methyl-1H-pyrazole-3-carbonyl)amino]malonic acid diethyl ester (6.9 g, quantitative) was obtained as an oily product by the same method as in Reference Example 13-(1), using 2-[(1-methyl-1H-pyrazole-3-carbonyl)amino]malonic acid (3.0 g) and aminomalonic acid diethyl ester hydrochloride (6.0 g).

¹H-NMR (400MHz, CDCl₃) δ: 1.31 (6H, t, J=7.1Hz), 3.94 (3H, s), 4.22-4.33 (4H, m). 5.36 (1H, d, J=7.4Hz), 6.78 (1H, m), 7.37 (1H, d, J=2.2Hz), 7.76 (1H, d, J=7.1Hz).

### 2) Title compound

A solution of sodium nitrite (3.5 g) in water (12.5 mL) was added dropwise to a mixed solution of 3-amino-5-methoxypyridine (4.9 g) in acetic acid (20 mL) and concentrated hydrochloric acid (5.0mL) at 0°C, and the resultant mixture was stirred for 15 minutes. Under cooling to -15°C, a solution of 2-[(1-methyl-1H-pyrazole-3-carbonyl)amino]malonic acid diethyl ester (8.5 g) in acetone (50 mL), and a solution of potassium carbonate (16. 6 g) in water (50 mL) were slowly added to the reaction solution, and the mixture was stirred at 0°C for 2 hours. Ethyl acetate was added to the reaction solution, and the mixture was partitioned. The organic layer was washed sequentially with water, a saturated sodium hydrogen carbonate solution, water and saturated brine, and then was dried over anhydrous sodium sulfate. After separating the organic layer by filtration, the solvent was evaporated under reduced pressure, and the residue obtained was dissolved in methanol (250 mL). Sodium methoxide (160 mg) was added to the solution at room temperature, and the mixture was stirred for 10 hours. A 1 mol/L aqueous solution of sodium hydroxide was added to the reaction solution, and the mixture was stirred for 1 hour. A 1 mol/L aqueous solution of hydrochloric acid was added to the reaction solution to acidify the solution, then a mixed solvent of methanol-dichloromethane (1:10) was added to thereto, and the resultant mixture was partitioned. The organic layer was dried over anhydrous sodium sulfate. After separating the organic layer by filtration, the solvent was evaporated under reduced pressure, to obtain the title compound (1.26 g, 14%) as a solid.

¹H-NMR (400MHz, DMSO-d₆) δ: 3.75 (3H, s), 3.91 (3H, s), 6.56 (1H, d, J=2.2Hz), 6.96 (1H, d, J=8.8Hz), 7.78 (1H, d, J=2.5Hz), 7.89 (1H, dd, J=8.8, 2.9Hz), 8.34 (1H, d, J=2.9Hz).

### [Reference Example 15]

1-(6-Methoxy-3-pyridyl)-5-(1-methyl-1H-imidazol-4-yl)-1H-1, 2,4-triazole-3-carboxylic acid

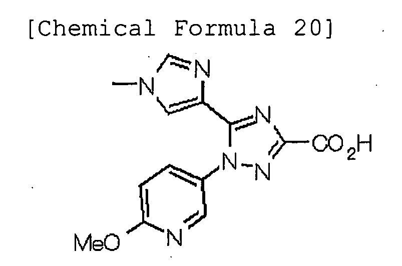

### 1) 2-[(1-Methyl-1H-imidazole-4-carbonyl)amino]malonic acid diethyl ester

2-[(1-Methyl-1H-imidazole-4-carbonyl)amino]malonic acid diethyl ester (6.45 g, 57%) was obtained as a solid by the same method as in Reference Example 13-(1), using 2-[(1-methyl-1H-imidazole-4-carbonyl)amino]malonic acid (5.0 g) and aminomalonic acid diethyl ester hydrochloride (10.1 g).

¹H-NMR (400MHz, CDCl₃) δ: 1.30 (6H, t, J=7.1Hz), 3.74 (3H, s), 4.25-4.33 (4H, m), 5.36 (1H, d, J=7.6Hz), 7.42 (1H, d, J=1.0Hz), 7.53 (1H, d, J=1.2Hz), 7.94 (1H, d, J=7.1Hz).

### 2) Title compound

A solution of sodium nitrite (3.5 g) in water (12.5 mL) was added dropwise to a mixed solution of 3-amino-5-methoxypyridine (4.9 g) in acetic acid (20 mL) and concentrated hydrochloric acid (5.0mL) at 0°C, and the resultant mixture was stirred for 15 minutes. Under cooling to -15°C, a solution of 2-[(1-methyl-1H-imidazole-4-carbonyl)amino]malonic acid diethyl ester (8.5 g) in acetone (50 mL), and a solution of potassium carbonate (16.6 g) in water (50 mL) were slowly added to the reaction solution, and the mixture was stirred at 0°C for 2 hours. Ethyl acetate was added to the reaction solution, and the mixture was partitioned. The organic layer was washed sequentially with water, a saturated sodium hydrogen carbonate solution, water and saturated brine, and then was dried over anhydrous sodium sulfate. After separating the organic layer by filtration, the solvent was evaporated under reduced pressure, and a residue thus obtained was dissolved in methanol (250 mL). Sodium methoxide (160 mg) was added thereto at room temperature, and the mixture was stirred for 10 hours. A 1 mol/L aqueous solution of sodium hydroxide was added to the reaction solution, and the mixture was stirred for 1 hour. A 1 mol/L aqueous solution of hydrochloric acid was added to the reaction solution to acidify the solution, then a mixed solvent of methanol-dichloromethane (1:10) was added to the reaction solution, and the mixture was partitioned. The organic layer was dried over anhydrous sodium sulfate. After separating the organic layer by filtration, the solvent was evaporated under reduced pressure, to obtain the title compound (2.19 g, 24%) as a solid.

¹H-NMR (400MHz, DMSO-d₆) δ: 3.67 (3H, s), 3.91 (3H, s), 6.94 (1H, dd, J=8.8, 0.7Hz), 7.62 (1H, d, J=1.0Hz), 7.73 (1H, d, J=1.2Hz), 7.85 (1H, dd, J=8.8, 2.7Hz), 8.31 (1H, dd, J=2.7, 0.5 Hz).

### [Reference Example 16]

1-(6-Methyl-3-pyridyl)-5-(5-methyl-2-pyridyl)-1H-1,2,4-tria zole-3-carboxylic acid

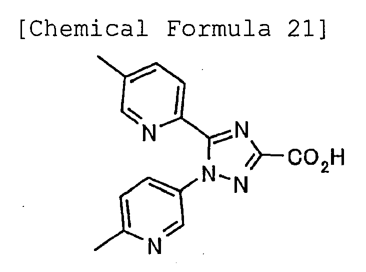

A solution of sodium nitrite (5.24 g) in water (2.5 ml) was added dropwise to a mixed solution of 3-amino-6-methylpyridine (7.81 g) in acetic acid (30 ml) and concentrated hydrochloric acid (7.5 ml) at 0°C, and then the resultant mixture was stirred for 15 minutes. Under cooling to -15°C, a solution of 2-[(5-methylpyridine-2-carbonyl)amino]malonic acid diethyl ester (13.24 g) of Reference Example 6-(1) in acetone (10 ml), and a solution of potassium carbonate (24.88 g) in water (10 ml) were slowly added to the reaction solution. The reaction solution was warmed to 0°C, and was stirred for 18 hours. Ethyl acetate was added to the reaction solution, and the mixture was partitioned. The organic layer was washed sequentially with water, a saturated sodium hydrogen carbonate solution, water and saturated brine, and was dried over anhydrous sodium sulfate. After separating the organic layer by filtration, the solvent was evaporated under reduced pressure, then to a solution of a residue thus obtained in methanol (250 ml), sodium methoxide (240mg) was added at room temperature, and the mixture was stirred for 1.5 hours. The solvent of the reaction solution was evaporated under reduced pressure, then a 1 M aqueous solution of sodium hydroxide was added to the residue thus obtained, and the mixture was stirred at room temperature for 40 minutes. A 1 M aqueous solution of hydrochloric acid was added to the reaction solution at 0°C to acidify the solution, then chloroform-methanol (10:1) was added thereto, and the mixture was partitioned. The organic layer was dried over anhydrous sodium sulfate. After separating the organic layer by filtration, the solvent was evaporated under reduced pressure, and a residue thus obtained was crystallized frommethanol-diethyl ether, to obtain the title compound (3.8 g, 29%).

¹H-NMR (400MHz, DMSO-d₆) δ: 2.32 (3H, s), 2.60 (3H, s), 7.50 (1H, d, J=8.3Hz), 7.84 (1H, dd, J=8.1, 2.2Hz), 7.95 (1H, dd, J=8.3, 2.5Hz), 8.03 (1H, d, J=8.1Hz), 8.26-8.26 (1H, m), 8.62 (1H, d, J=2.5Hz).

### [Reference Example 17]

1-(6-Methyl-3-pyridyl)-5-(2-pyridyl)-1H-1,2,4-triazole-3-ca rboxylic acid

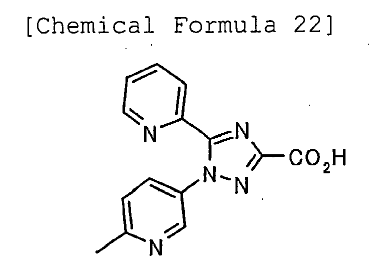

The title compound (770 mg, 9%) was obtained as a solid by the same method as in Reference Example 16, using 3-amino-6-methylpyridine (4.87 g) and 2-[(pyridine-2-carbonyl)amino]malonicacid diethyl ester (8.41 g) of Reference Example 1-(1).

¹H-NMR (400MHz, DMSO-d₆) δ: 7.41 (1H, d, J=8.3Hz), 7.48 (1H, ddd, J=7.5, 4.8, 1.2Hz), 7.84 (1H, dd, J=8.3, 2.7Hz), 8.01 (1H, td, J=7.7, 1.7Hz), 8.10-8.12 (1H, m), 8.39 (1H, d, J=4.2Hz), 8.53 (1H, d, J=2.5Hz).

[Reference Example 18] 1-Methylpiperazin-2-one hydrochloride

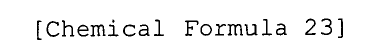

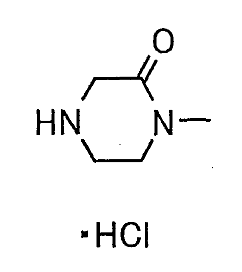

### 1) 3-oxopiperazine-1-carboxylic acid tert-butyl ester

Triethylamine (3.83 ml) and di-tert-butoxydicarbonate (6.32 ml) were added to a mixed solution of piperazin-2-one (2.5 g) in tetrahydrofuran (50 ml) and methanol (50 ml) at room temperature, and the resultant mixture was stirred for 4 hours. The reaction solvent was evaporated under reduced pressure, then water and ethyl acetate were added to the residue thus obtained, and the mixture was partitioned. The organic layer was washed sequentially with water and saturated brine, and then the washing water layers were combined and extracted again with ethyl acetate. The combined organic layers were dried over anhydrous magnesium sulfate. After separating the organic layer by filtration, the solvent was evaporated under reduced pressure, and a residue thus obtained was solidified from ethyl acetate-hexane, to obtain 3-oxopiperazine-1-carboxylic acid tert-butyl ester (3.6 g, 72%).

¹H-NMR (400MHz, CDCl₃) δ: 1.48 (9H, s), 3.37-3.40 (2H, m), 3.62-3.65 (2H, m), 4.01 (2H, s), 6.32 (1H, br s).

### 2) 4-Methyl-3-oxopiperazine-1-carboxylic acid tert-butyl ester

Sodium hydride (60%, 960 mg) was added to a solution of the 3-oxopiperazine-1-carboxylic acid tert-butyl ester (3.0 g) obtained above in N,N-dimethylformamide (50 ml) at 0°C, then methyl iodide (2.33 ml) was added to the reaction solution; and the resultant mixture was stirred at room temperature for 15 hours. Water and ethyl acetate were added to the reaction solution, and the mixture was partitioned. The organic layer was washed sequentially with water and saturated brine, and then the washing water layers were combined and further extracted with ethyl acetate. The combined organic layers were dried over anhydrous magnesium sulfate. After separating the organic layer by filtration, the solvent was evaporated under reduced pressure, to obtain 4-methyl-3-oxopiperazine-1-carboxylicacid tert-butyl ester (2.32 g, 72%) as an oily product.

¹H-NMR (400MHz, CDCl₃) δ: 1.47 (9H, s), 3.01 (3H, s), 3.34 (2H, t, J=5.6Hz), 3.65 (2H, t, J=5.6Hz), 4.07 (2H, s).

### 3) Title compound

A solution of 4 M hydrochloric acid-dioxane solution (20 ml) was added to the 4-methyl-3-oxopiperazine-1-carboxylicacid tert-butyl ester (2.06 g) obtained above, and the resultant mixture was stirred at roomtemperature for 1 hour. The reaction solvent was evaporated under reduced pressure, and toluene was added to the residue thus obtained. The solvent of the mixture was azeotropically evaporated under reduced pressure, and a residue thus obtained was dried, to obtain the title compound (1.44 g, 99%) as an oily product.

¹H-NMR (400MHz, DMSO-d₆) δ: 2.86 (3H, s), 3.34 (2H, br m), 3.50 (2H, m), 3.64 (2H, s).
ESI-MSm/z: 115 (M+H)⁺.

[Reference Example 19] (2S)-2-Fluoromethylpyrrolidine hydrochloride

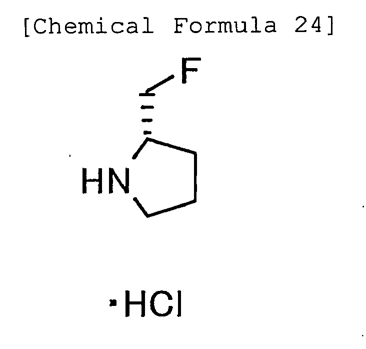

### 1) (2S)-1-Benzoyl-2-hydroxymethylpyrrolidine

Sodium hydrogen carbonate (7.51 g) was added to a mixed solution of (2S)-hydroxymethylpyrrolidine (3.00ml) and benzoyl chloride (6. 92 ml) in dichloromethane (100 ml) and water (100 ml) at room temperature, and the resultant mixture was stirred for 1.5 hours. Dichloromethane was added to the reaction solution, and the mixture was partitioned. The organic layer was dried over anhydrous sodium sulfate. After separating the organic layer by filtration, the solvent was evaporated under reduced pressure, and a residue thus obtained was dissolved in tetrahydrofuran (120 ml) and water (60 ml). Lithium hydroxide monohydrate (6.25 g) was added to the reaction solution at room temperature, and the mixture was stirred overnight. The reaction solution was partitioned, and the organic layer was dried over anhydrous sodium sulfate. After separating the organic layer by filtration, the solvent was evaporated under reduced pressure, to obtain (2S)-1-benzoyl-2-hydroxymethylpyrrolidine (5.79 g, 98%) as an oily product.

¹H-NMR (400MHz, CDCl₃) δ: 1.60-2.27 (4H, m), 3.43-3.52 (2H, m), 3.71-3.82 (2H, m), 4.40 (1H, d, J=7.3Hz), 4.92 (1H, s), 7.40-7.52 (5H, m).

### 2) (2S)-1-Benzoyl-2-fluoromethylpyrrolidine

Diethylaminosulfur trifluoride (1. 93 ml) was added to a solution of (2S)-1-benzoyl-2-hydroxymethylpyrrolidine (1.50g) in dichloromethane (50 ml) under ice cooling, and the resultant mixture was stirred overnight. A saturated aqueous solution of sodium hydrogen carbonate and chloroform were added to the reaction solution, and the mixture was partitioned. The organic layer was dried over anhydrous sodium sulfate. After separating the organic layer by filtration, the solvent was evaporated under reduced pressure, and a residue thus obtained was purified by silica gel column chromatography (hexane-ethyl acetate), to obtain (2S)-1-benzoyl-2-fluoromethylpyrrolidine (772 mg, 51%) as an oily product.

¹H-NMR (400MHz, CDCl₃) δ: 1.74-2.18 (4H, m), 3.49 (2H, br s), 4.44-4.90 (3H, m), 7.38-7.54 (5H, m).

### 3) (2S)-1-Benzyl-2-fluoromethylpyrrolidine

Under a nitrogen atmosphere, lithium aluminum hydride (128 mg) was added to a solution of (2S)-1-benzoyl-2-fluoromethylpyrrolidine (350 mg) in tetrahydrofuran (20 ml) at room temperature, and the resultant mixture was heated to reflux for 1 hour. After air cooling, ice was added to the reaction solution for treating with an excess of lithium aluminum hydride. Subsequently, diethyl ether and water were added to the reaction solution, and the mixture was partitioned. The organic layer was dried over anhydrous sodium sulfate. After separating the organic layer by filtration, the solvent was evaporated under reduced pressure, and a residue thus obtained was purified by silica gel column chromatography (ethyl acetate-hexane), to obtain (2S)-1-benzyl-2-fluoromethylpyrrolidine (142 mg, 44%) as an oily product.

¹H-NMR (400MHz, CDCl₃) δ: 1. 62-1. 76 (3H, m), 1.88-1.97 (1H, m), 2.25-2.31 (1H, m), 2.84-2.97 (2H, m), 3.48 (1H, d, J=12.9Hz), 4.04 (1H, d, J=13.2Hz), 4.21-4.42 (2H, m), 7.22-7.34 (5H, m).

### 4) Title compound

1-Chloroethyl chloroformate (289 µl) was added to a solution of (2S)-1-benzyl-2-fluoromethylpyrrolidine (466 mg) in dichloromethane (20ml) at room temperature, and the resultant mixture was heated to reflux for 1 hour. After air cooling, the reaction solvent was evaporated under reduced pressure, then a residue thus obtained was dissolved in methanol (20 ml), and the solution was heated to reflux for 1 hour. After air cooling, the reaction solvent was distilled under reduced pressure, and a solid obtained therefrom was collected by filtration using diethyl ether, to obtain the title compound (292 mg, 87%).

¹H-NMR (400MHz, DMSO-d₆) δ: 1.56-2.06 (4H, m), 3.15 (2H, t, J=7.2Hz), 3.75-3.85 (1H, m), 4.55-4.76 (2H, m), 9.66 (2H, s).

### [Reference Example 20]

1-[5-(5-Cyano-2-pyridyl)-1-(6-methoxy-3-pyridyl)-1H-1,2,4-t riazole-3-carbonyl]-4,4-difluoropiperidine

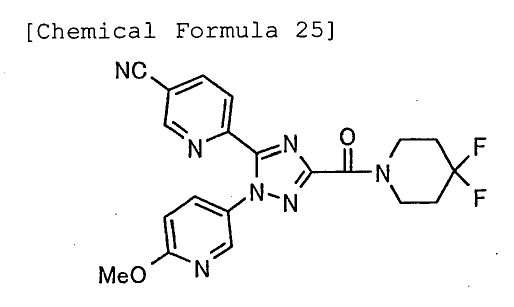

### 1) 5-Benzyloxy-2-methylpyridine

Benzyl bromide (10.9 ml) was added to a solution of 3-hydroxy-6-methylpyridine (10.0 g) and potassium carbonate (38.0) in acetonitrile (200 ml) at room temperature, and the resultant mixture was stirred for 12 hours. Water and ethyl acetate were added to the reaction solution, and the mixture was partitioned. The organic layer was dried over anhydrous sodium sulfate. After separating the organic layer by filtration, the solvent was evaporated under reduced pressure, and a residue thus obtained was purified by silica gel column chromatography (ethyl acetate-hexane), to obtain 5-benzyloxy-2-methylpyridine (4.14 g, 23%) as an oily product.

¹H-NMR (400MHz, CDCl₃) δ: 2.48 (3H, s), 5.08 (2H, s), 7.05 (1H, d, J=8.5Hz), 7.16 (1H, dd, J=8.5, 2.9Hz), 7.31-7.43 (5H, m), 8.26 (1H, d, J=2.9Hz).
EI-MS m/z: 199 (M⁺).

### 2) 2-[(5-Benzyloxypyridine-2-carbonyl)amino]malonic acid dimethyl ester

Selenium dioxide (40 g) was added to a solution of the 5-benzyloxy-2-methylpyridine (40 g) obtained above in pyridine (200 ml), and the resultant mixture was heated to reflux for 24 hours. After air cooling, water and chloroform were added to the reaction solution, and the mixture was partitioned. The organic layer was dried over anhydrous sodium sulfate. After filtration, the solvent was evaporated under reduced pressure, then toluene was added to the residue thus obtained, and the solvent of the mixture was azeotropically evaporated under reduced pressure. A residue thus obtained was dissolved in N,N-dimethylformamide (1 1), then triethylamine (83.94 ml), 1-ethyl-3-(3-dimethylaminopropyl)carbodiimide hydrochloride (42.33 g), 1-hydroxybenzotriazole (29.8 g), and aminomalonic acid dimethyl ester hydrochloride (37 g) were added thereto, and the resultant mixture was stirred at room temperature for 164 hours. Water and a mixed solvent of methylene chloride-methanol (10:1) were added to the reaction solution, and the mixture was partitioned. The organic layer was dried over anhydrous sodium sulfate. After filtration, the solvent was evaporated under reduced pressure, and a residue thus obtained was purified by silica gel column chromatography (hexane-ethyl acetate). Crude crystals thus obtained were recrystallized from methylene chloride-hexane, to obtain 2-(5-benzyloxypyridine-2-carbonyl)aminomalonic acid dimethyl ester (14.7 g).

¹H-NMR (400MHz, CDCl₃) δ: 3.85 (6H, s), 5.17 (2H, s), 5.42 (1H, d, J=7.4Hz), 7.33-7.49 (6H, m), 8.11 (1H, d, J=9.1Hz), 8.32 (1H, d, J=6.1Hz), 8.72 (1H, d, J=7.4Hz).

### 3) 5-(5-Benzyloxy-2-pyridyl)-1-(6-methoxy-3-pyridyl)-1H-1,2,4-triazole-3-carboxylic acid methyl ester

A solution of sodium nitrite (4.77 g) in water (17 ml) was added dropwise to a solution of 5-amino-2-methoxypyridine (5.9 g) in acetic acid (27 ml) and concentrated hydrochloric acid (6.75 ml) at 0°C, and the resultant mixture was stirred for 15 minutes. Under cooling to -15°C, a solution of the 2-[(5-benzyloxypyridine-2-carbonyl)amino]malonic acid dimethyl ester (14.7 g) obtained above in acetone (68 ml) was added to the reaction solution, and a saturated aqueous solution of potassium carbonate was slowly added thereto until the reaction solution reached pH 6. After stirring the reaction solution at 0°C for 30 minutes, ethyl acetate was added to the reaction solution,and the mixture was partitioned. The organic layer was washed sequentially with water, a saturated sodium hydrogen carbonate solution, water and saturated brine, and was dried over anhydrous sodium sulfate. After filtration, the solvent was evaporated under reduced pressure, and a residue thus obtained was dissolved in methanol (200 ml). Then, sodium methoxide (356 mg) was added to the solution at room temperature, and the mixture was stirred for 19 hours. The reaction solvent was evaporated under reduced pressure, and crude crystals thus obtained were collected by filtration and washed with cold methanol, to obtain 5-(5-benzyloxy-2-pyridyl)-1-(6-methoxy-3-pyridyl)-1H-1,2,4-triazole-3-carboxylic acid methyl ester (5.3 g, 31%).

¹H-NMR (400MHz, CDCl₃) δ: 4.01 (3H, s), 4.06 (3H, s), 5.13 (2H, s), 6.82 (1H, d, J=8.8Hz), 7.39-7.35 (6H, m), 7.70-7.68 (1H, m), 8.20-8.16 (3H, m).

### 4) 5-(5-Hydroxy-2-pyridyl)-1-(6-methoxy-3-pyridyl)-1H-1,2,4-tr iazole-3-carboxylic acid methyl ester

10% Palladium on carbon (5 g) was added to a mixed solution of the 5-(5-benzyloxy-2-pyridyl)-1-(6-methoxy-3-pyridyl)-1H-1,2,4-triazole-3-carboxylic acid methyl ester (5.3 g) obtained above in methanol (200 ml), acetic acid (50 ml), and ethyl acetate (300 ml), and the resultant mixture was stirred under a hydrogen atmosphere at room temperature for 24 hours. The reaction solution was filtered, and the filtrate solvent was evaporated under reduced pressure. Crude crystals thus obtained were recrystallized from ethyl acetate-hexane, to obtain 5-(5-hydroxy-2-pyridyl)-1-(6-methoxy-3-pyridyl)-1H-1, 2, 4-tr iazole-3-carboxylic acid methyl ester (3.4 g, 81%).

¹H-NMR (400MHz, CDCl₃) δ: 3.98 (3H, dd, J=19.6, 10.8Hz), 4.03 (3H, t, J=8:8Hz), 6.84 (1H, d, J=8.8Hz), 7.19 (1H, dd, J=8.3, 2.5Hz), 7.74 (1H, dd, J=8.8, 2.5Hz), 7.94 (1H, d, J=8.8Hz), 8.03 (1H, d, J=2.5Hz), 8.19 (1H, d, J=2.5Hz).

### 5) 5-(5-Cyano-2-pyridyl)-1-(6-methoxy-3-pyridyl)-1H-1,2,4-tria zole-3-carboxylic acid methyl ester

To a solution of the 5-(5-hydroxy-2-pyridyl)-1-(6-methoxy-3-pyridyl)-1H-1,2,4-tr iazole-3-carboxylic acid methyl ester (3.3 g) obtained above in methylene chloride (50 ml), pyridine (1.63 ml) and trifluoromethanesulfonic anhydride (2. 04 ml) were added at room temperature, and the resultant mixture was stirred for 2.5 hours. A saturated aqueous solution of sodium hydrogen carbonate, and methylene chloride were added to the reaction solution, and the mixture was partitioned. The organic layer was dried over anhydrous sodium sulfate. After filtration, the solvent was evaporated under reduced pressure, and a residue thus obtained was dissolved in 1,2-dichloroethane (240 ml). Tetrakis(triphenylphosphine)palladium (15.85 g) and tri-n-butyltin cyanide (2.89 g) were added to the resultant solution, and the mixture was stirred at 80°C for 23 hours. After air cooling, an excess of potassium fluoride and methanol were added to the reaction solution, and the mixture was stirred at room temperature for 5 hours. A saturated aqueous solution of sodium hydrogen carbonate was added to the reaction solution, and the reaction solution was filtered through Celite. Then, chloroform was added to the filtrate, and the mixture was partitioned. The organic layer was dried over anhydrous sodium sulfate. After filtration, the solvent was evaporated under reduced pressure, and a residue thus obtained was purified by silica gel column chromatography (ethyl acetate-chloroform), to obtain 5-(5-cyano-2-pyridyl)-1-(6-methoxy-3-pyridyl)-1H-1,2,4-tria zole-3-carboxylic acid methyl ester (1.73 g, 56%) as a solid.

¹H-NMR (400MHz, CDCl₃) δ: 4.00 (3H, s), 4.06 (3H, s), 6.84 (1H, d, J=6.4Hz), 7.67 (1H, dd, J=8.8, 2.9Hz), 8.12 (1H, dd, J=8.3, 2.0Hz), 8.20 (1H, d, J=2.0Hz), 8.96 (1H, dd, J=8.3, 1.0Hz), 8.65-8.67 (1H, m).

### 6) 5-(5-Cyano-2-pyridyl)-1-(6-methoxy-3-pyridyl)-1H-1,2,4-tria zole-3-carboxylic acid

Lithium hydroxide monohydrate (290 mg) was added to a solution of the 5-(5-cyano-2-pyridyl)-1-(6-methoxy-3-pyridyl)-1H-1,2,4-tria zole-3-carboxylic acid methyl ester (1.6 g) obtained above in tetrahydrofuran (30 ml) and water (15 ml) at room temperature, and the resultant mixture was stirred for 5.5 hours. A1M aqueous solution of hydrochloric acid was added to the reaction solution, and crystals precipitated there from were collected by filtration, to obtain 5-(5-cyano-2-pyridyl)-1-(6-methoxy-3-pyridyl)-1H-1,2,4-tria zole-3-carboxylic acid (1.07 g, 95%) as a solid.

¹H-NMR (400MHz, DMSO-d₆) δ: 3.91 (3H, s), 6.96 (1H, d, J=8.8Hz), 7.90 (1H, dd, J=8.8, 2.5Hz), 8.29 (1H, dd, J=8.3, 1.0Hz), 8.34 (1H, d, J=2.5Hz), 8.51 (1H, dd, J=8.3, 2.0Hz), 8.86-8.88 (1H, m).

### 7) Title compound

The title compound (63 mg, 25%) was obtained as a solid by the same method as in Example 1, using 5-(5-cyano-2-pyridyl)-1-(6-methoxy-3-pyridyl)-1H-1,2,4-tria zole-3-carboxylic acid (194 mg) and 4,4-difluoropiperidine hydrochloride (89 mg) of Reference Example 40.

¹H-NMR (400MHz, CDCl₃) δ: 2.17-2.08 (4H, m), 3.96-4.07 (7H, m), 6.85 (1H, d, J=8.8Hz), 7.66 (1H, dd, J=8.8, 2.7Hz), 8.11 (1H, dd, J=8.2, 2.1Hz), 8.21 (1H, d, J=2.2Hz), 8.39 (1H, dd, J=8.2, 0.9Hz), 8.67 (1H, q, J=1.0Hz).

### Example 1

N-tert-Butyl-1-(6-methoxy-3-pyridyl)-5-(2-pyridyl)-1H -1,2,4-triazole-3-carboxamide

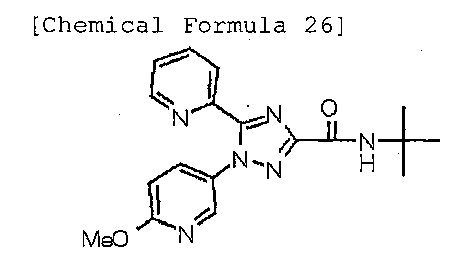

To a solution of 1-(6-methoxy-3-pyridyl)-5-(2-pyridyl)-1H-1,2,4-triazole-3-c arboxylic acid (297 mg) of Reference Example 1 in N,N-dimethylformamide (5 mL), triethylamine (418 µL), 1-ethyl-3-(3-dimethylaminopropyl)carbodiimide hydrochloride (211 mg), 1-hydroxybenzotriazole (149mg), and tert-butylamine (126 µL) were added, and the resultant mixture was stirred at room temperature for 48 hours. Water and a mixed solvent of dichloromethane-methanol (10/1) were added to the reaction solution, and the mixture was partitioned. The organic layer was dried over anhydrous sodium sulfate. After separating the organic layer by filtration, the solvent was evaporated under reduced pressure, and a residue thus obtained was purified by silica gel thin layer chromatography (dichloromethane-methanol), to obtain the title compound (181 mg, 51%) as a solid.

¹H-NMR (400MHz, CDCl₃) 5: 1.54 (9H, d, J=16. 9Hz), 3.98 (3H, s), 6.81 (1H, dd, J=8.8, 0.5Hz), 7.06 (1H, s), 7.33 (1H, ddd, J=7.7, 4.8, 1.2Hz), 7.69 (1H, dd, J=8.8, 2.7Hz), 7.81 (1H, td, J=7.7, 1.8Hz), 8. 09 (1H, dt, J=8. 0, 1.0Hz), 8.19-8.20 (1H, m), 8. 45-8.47 (1H, m).
FAB-MSm/z: 375 (M+Na)⁺.

### Example 2

N-Cyclopentyl-1-(6-methoxy-3-pyridyl)-5-(2-pyridyl)-1 H-1,2,4-triazole-3-carboxamide

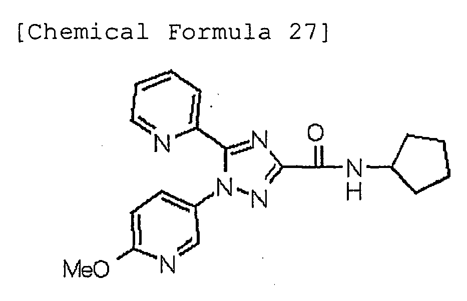

The title compound (103 mg, 28%) was obtained as a solid by the same method as in Example 1, using 1-(6-methoxy-3-pyridyl)-5-(2-pyridyl)-1H-1,2,4-triazole-3-c arboxylic acid (297 mg) of Reference Example 1 and cyclopentylamine (118 µL).

¹H-NMR (400MHz, CDCl₃) δ: 1.52-1.80 (8H, m), 2.12 (2H, m), 3.99 (3H, s), 4.50 (1H, m) , 6.81 (1H, d, J=8.8Hz), 7.15 (1H, d, J=7.8Hz), 7.32-7.35 (1H, m), 7.70 (1H, dd, J=8.8, 2.7Hz), 7.82 (1H, td, J=7.8, 1.8Hz), 8.08 (1H, d, J=7.8Hz), 8.20 (1H, d, J=2.7Hz), 8.47 (1H, t, J=2.3Hz).
FAB-MSm/z: 387 (M+Na)⁺.

### Example 3

N-tert-Butyl-5-(5-cyano-2-pyridyl)-1-(6-methoxy-3-pyr idyl)-1H-1,2,4-triazole-3-carboxamide

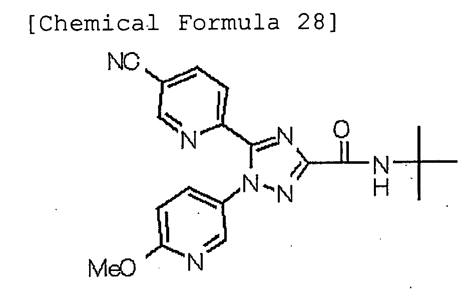

The title compound (64 mg, 28%) was obtained as a solid by the same method as in Example 1, using 5-(5-cyano-2-pyridyl)-1-(6-methoxy-3-pyridyl)-1H-1,2,4-tria zole-3-carboxylic acid (194 mg) of Reference Example 2 and tert-butylamine (75 µL).

¹H-NMR (400MHz, CDCl₃) δ: 1.52 (9H, s), 4.01 (3H, s), 6.84 (1H, d, J=8.8Hz), 6.99 (1H, s), 7.66 (1H, dd, J=8.8, 2.7Hz), 8.10 (1H, dd, J=8.2, 2.1Hz), 8.18 (1H, d, J=2.7Hz), 8.40 (1H, d, J=7.4Hz), 8.67 (1H, d, J=1.2Hz).

### Example 4

5-(5-Cyano-2-pyridyl)-1-(6-methoxy-3-pyridyl)-1H-1,2, 4-triazole-3-carboxylic acid N-neopentylamide

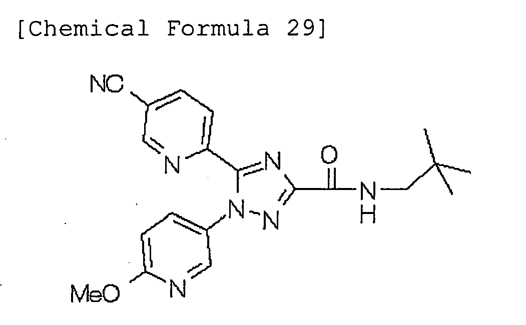

The title compound (53 mg, 23%) was obtained as a solid by the same method as in Example 1, using 5-(5-cyano-2-pyridyl)-1-(6-methoxy-3-pyridyl)-1H-1,2,4-tria zole-3-carboxylic acid (194 mg) of Reference Example 2 and neopentylamine (85 µL).

¹H-NMR (400MHz, CDCl₃) δ: 1.02 (9H, s), 3.34 (2H, d, J=6.6Hz), 4.01 (3H, s), 7.68 (1H, dd, J=8.8, 2.7Hz), 8.11 (1H, dd, J=8.3, 2.2Hz), 8.20 (1H, d, J=2.7Hz), 8.41 (1H, dd, J=8.2, 0.9Hz), 8.68 (1H, dd, J=1.0, 0.5Hz).
FAB-MSm/z: 392 (M+H)⁺.

### Example 5

1-(6-Methoxy-3-pyridyl)-5-(2-pyridyl)-1H-1,2,4-triazo le-3-carboxylic acid N-neopentylamide

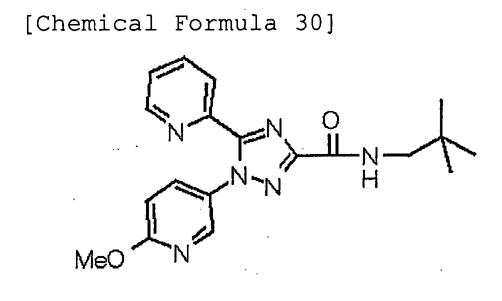

The title compound (56 mg, 31%) was obtained as a solid by the same method as in Example 1, using 1-(6-methoxy-3-pyridyl)-5-(2-pyridyl)-1H-1,2,4-triazole-3-c arboxylic acid (149 mg) of Reference Example 1 and neopentylamine (71 µL).

¹H-NMR (400MHz, CDCl₃) δ: 1.04 (9H, d, J=16.9Hz), 3.34 (2H, d, J=6.6Hz), 3.99 (3H, s), 6.82 (1H, dd, J=8.8, 0.5Hz), 7.34 (1H, ddd, J=7.7, 4.8, 1.1Hz), 7.71 (1H, dd, J=8.8, 2.7Hz), 7.83 (1H, td, J=7.7, 1.7Hz), 8.12 (1H, dt, J=7.9, 1.0Hz), 8.21 (1H, dd, J=2.7, 0.5Hz), 8.47 (1H, dq, J=4.8, 0.9Hz).
FAB-MSm/z: 389 (M+Na)⁺.

### Example 6

N-tert-Butyl-1-(6-methoxy-3-pyridyl)-5-(5-methyl-2-py razinyl)-1H-1,2,4-triazole-3-carboxamide

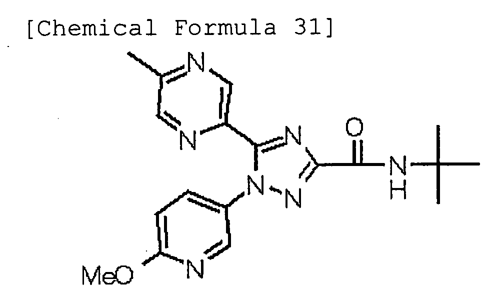

The title compound (300 mg, 82%) was obtained as a solid by the same method as in Example 1, using 1-(6-methoxy-3-pyridyl)-5-(5-methyl-2-pyrazinyl)-1H-1,2,4-t riazole-3-carboxylic acid (312 mg) of Reference Example 8 and tert-butylamine (126 µL).

¹H-NMR (400MHz, CDCl₃) δ: 1.52 (9H, s), 2.61 (3H, s), 3.99 (3H, s), 6.82 (1H, dd, J=8.8, 0.7Hz), 7.05 (1H, s), 7.68 (1H, dd, J=8.8, 2.7Hz), 8.19 (1H, dd, J=2.7, 0.5Hz), 8.29 (1H, q, J=0.7Hz), 9.24 (1H, d, J=1.5Hz).
FAB-MSm/z: 390 (M+Na)⁺.

### Example 7

1-(6-Methoxy-3-pyridyl)-5-(5-methyl-2-pyrazinyl)-1H-1 ,2,4-triazole-3-carboxylic acid N-neopentylamide

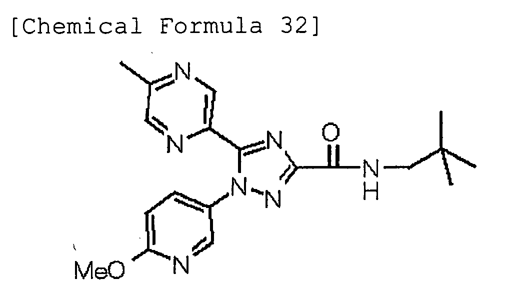

The title compound (249 mg, 65%) was obtained as a solid by the same method as in Example 1, using 1-(6-methoxy-3-pyridyl)-5-(5-methyl-2-pyrazinyl)-1H-1,2,4-t riazole-3-carboxylic acid (312 mg) of Reference Example 8 and neopentylamine (141 µL).

¹H-NMR (400MHz, CDCl₃) δ: 1.02 (9H, s), 2.61 (3H, s), 3.35 (2H, d, J=6.6Hz), 3.99 (3H, s), 6.83 (1H, d, J=8.8Hz), 7.69 (1H, dd, J=8.8, 2.7Hz), 8.21 (1H, d, J=2.7Hz), 8.30 (1H, d, J=1.2Hz), 9.25 (1H, d, J=1.2Hz).
FAB-MSm/z: 404 (M+Na)⁺.

### Example 8

N-tert-Butyl-1-(6-methoxy-3-pyridyl)-5-(2-pyrazinyl)-1H-1,2,4-triazole-3-carboxamide

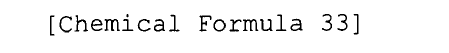

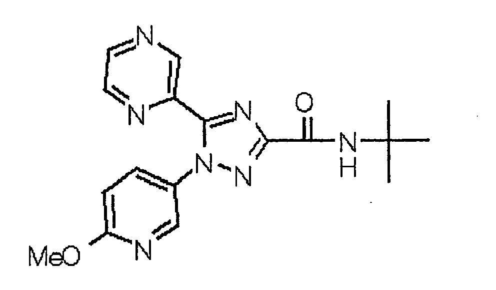

The title compound (196 mg, 56%) was obtained as a solid by the same method as in Example 1, using 1-(6-methoxy-3-pyridyl)-5-(2-pyrazinyl)-1H-1,2,4-triazole-3 -carboxylic acid (298 mg) of Reference Example 13 and tert-butylamine (126 µL).

¹H-NMR (400MHz, CDCl₃) δ: 1.52 (9H, s), 4.00 (3H, s), 6.83 (1H, dd, J=8.8, 0.7Hz), 7.06 (1H, s), 7.68 (1H, dd, J=8.8, 2.9Hz), 8.21 (1H, dd, J=2.7, 0.7Hz), 8.42 (1H, dd, J=1.3, 0.7Hz), 8.62 (1H, d, J=2.5Hz), 9.41 (1H, d, J=1.5Hz).
FAB-MSm/z: 376 (M+Na)⁺.

### Example 9

N-tert-Butyl-1-(6-methoxy-3-pyridyl)-5-(5-methyl-2-py ridyl)-1H-1,2,4-triazole-3-carboxamide

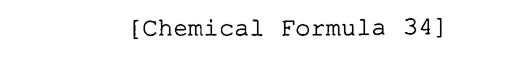

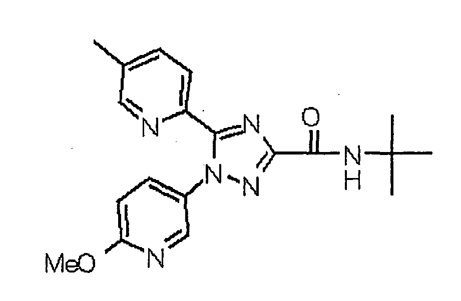

The title compound (142 mg, 39%) was obtained as a solid by the same method as in Example 1, using 1-(6-methoxy-3-pyridyl)-5-(5-methyl-2-pyridyl)-1H-1,2,4-tri azole-3-carboxylic acid (326 mg) of Reference Example 6 and tert-butylamine (126 µL).

¹H-NMR (400MHz, CDCl₃) δ: 1.51 (9H, s), 2.36 (3H, s), 3.98 (3H, s), 6.80 (1H, d, J=8.8Hz), 7.06 (1H, s), 7.60 (1H, dd, J=8.0, 2.1Hz), 7.69 (1H, dd, J=8.8, 2.7Hz), 7. 95 (1H, d, J=7.6Hz), 8.18 (1H, d, J=2.7Hz), 8.30 (1H, s).
FAB-MSm/z: 389 (M+Na)⁺.

### Example 10

1-(6-Methoxy-3-pyridyl)-5-(5-methyl-2-pyridyl)-1H-1,2 ,4-triazole-3-carboxylic acid N-(tetrahydropyran-4-yl)amide

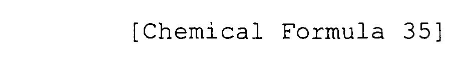

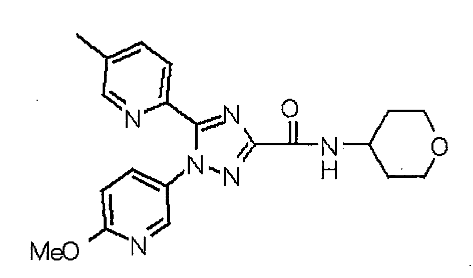

The title compound (157 mg, 43%) was obtained as a solid by the same method as in Example 1, using 1-(6-methoxy-3-pyridyl)-5-(5-methyl-2-pyridyl)-1H-1,2,4-tri azole-3-carboxylic acid (311 mg) of Reference Example 6 and 4-tetrahydropyranylamine (123 mg).

¹H-NMR (400MHz, CDCl₃) δ: 1. 60-1. 70 (4H, m), 2.04 (2H, dq, J=12.4, 2.0Hz), 2.37 (3H, s), 3.51-3.58 (2H, m), 4.01 (5H, m), 4.28-4.32 (1H, m), 6.81 (1H, dd, J=8.8, 0.5Hz), 7.15 (1H, d, J=8.3Hz), 7.61 (1H, dq, J=8.1, 1.0Hz), 7.70 (1H, dd, J=8.8, 2.7Hz), 7.93 (1H, d, J=8.1Hz), 8.19 (1H, dd, J=2.7, 0.5Hz), 8.32 (1H, q, J=0.7Hz).
FAB-MSm/z: 395 (M+H)⁺.

### Example 11

N-tert-Butyl-5-(5-chloro-2-pyridyl)-1-(6-methoxy-3-py ridyl)-1H-1,2,4-triazole-3-carboxamide

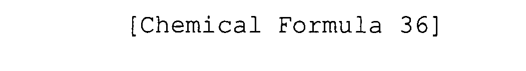

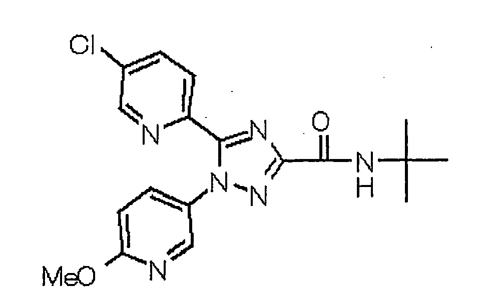

The title compound (23 mg, 9%) was obtained as a solid by the same method as in Example 1, using 5-(5-chloro-2-pyridyl)-1-(6-methoxy-3-pyridyl)-1H-1,2,4-tri azole-3-carboxylic acid (210 mg) of Reference Example 7 and tert-butylamine (80 µL).

¹H-NMR (400MHz, CDCl₃) δ: 1.51 (9H, s), 3.99 (3H, s), 6.82 (1H, d, J=8.8Hz), 7.01 (1H, br s), 7.66 (1H, dd, J=8.8, 2.7Hz), 7.80 (1H, dd, J=8.3, 2.5Hz), 8.13 (1H, d, J=8.3Hz), 8.18 (1H, d, J=2.5Hz), 8.38 (1H, d, J=2.2Hz).
ESI-MSm/z: 386 (M⁺).

### Example 12

N-tert-Butyl-5-(5-fluoro-2-pyridyl)-1-(6-methoxy-3-py ridyl)-1H-1,2,4-triazole-3-carboxamide

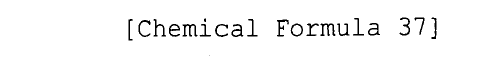

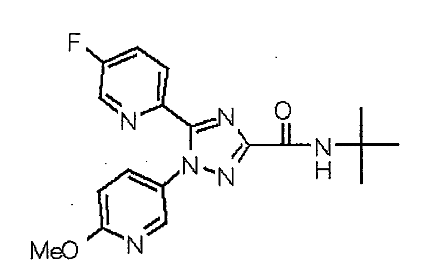

To a solution of 5-(5-fluoro-2-pyridyl)-1-(6-methoxy-3-pyridyl)-1H-1,2,4-tri azole-3-carboxylicacidsodiumsalt (630mg) of Reference Example 5 in acetonitrile (10 mL), triethylamine (418 µL), 1-ethyl-3-(3-dimethylaminopropyl)carbodiimide hydrochloride (421 mg), 1-hydroxybenzotriazole (297 mg) and tert-butylamine (252 µL) were added, and the resultant mixture was stirred at room temperature for 14.5 hours. Water and a mixed solvent of chloroform-methanol (10/1) were added to the reaction solution, and the mixture was partitioned. The organic layer was dried over anhydrous sodium sulfate. After separating the organic layer by filtration, the solvent was evaporated under reduced pressure, and a residue thus obtained was purified by silica gel thin layer chromatography (chloroform-methanol), to obtain the title compound (213 mg, 29%) as a solid.

¹H-NMR (400MHz, CDCl₃) δ: 1.51 (9H, s), 3.99 (3H, s), 6.82 (1H, d, J=8.8Hz), 7.02 (1H, s), 7.53 (1H, td, J=8.3, 2.9Hz), 7.67 (1H, dd, J=8.8, 2.7Hz), 8.18-8.22 (2H, m), 8.29 (1H, d, J=2.7Hz). FAB-MSm/z: 393 (M+Na)⁺.

### Example 13

5-(5-tert-Butoxycarbonylamino-2-pyridyl)-1-(6-methoxy -3-pyridyl)-1H-1,2,4-triazole-3-carboxylic acid tert-butylamide

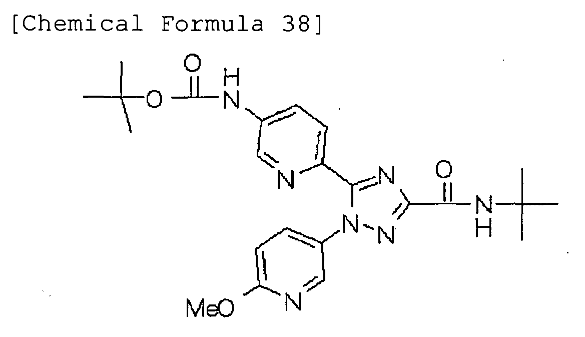

The title compound (1.26 g, 94%) was obtained as a solid by the same method as in Example 1, using 5-(5-tert-butoxycarbonylamino-2-pyridyl)-1-(6-methoxy-3-pyr idyl)-1H-1,2,4-triazole-3-carboxylic acid (1.29 g) of Reference Example 9 and tert-butylamine (1.37 mL).

¹H-NMR (400MHz, CDCl₃) δ: 1.50-1.52 (16H, m), 3.98-3.98 (3H, m), 6.78 (1H, dd, J=8.8, 0.7Hz), 7.27 (1H, s), 7.65 (1H, dq, J=8.8, 1.4Hz), 8.00-8.01 (1H, m), 8.08 (1H, dd, J=8.7, 2.3Hz), 8.18 (1H, d, J=2.7Hz), 8.41 (1H, d, J=2.5Hz).

### Example 14

5-(5-Amino-2-pyridyl)-1-(6-methoxy-3-pyridyl)-1H-1,2, 4-triazole-3-carboxylic acid N-tert-butylamide

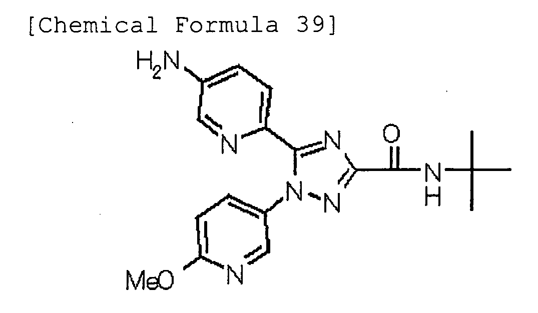

A 4 mol/L hydrochloric acid-1, 4-dioxane solution (20 mL) was added to a solution of 5-(5-tert-butoxycarbonylamino-2-pyridyl)-1-(6-methoxy-3-pyr idyl)-1H-1,2,4-triazole-3-carboxylic acid N-tert-butylamide (730 mg) of Example 13 in dichloromethane (20 mL), and the resultant mixture was stirred at room temperature for 3 minutes. A saturated aqueous solution of sodium hydrogen carbonate, and dichloromethane were added to the reaction solution, and the mixture was partitioned. The organic layer was dried over anhydrous sodium sulfate. After separating the organic layer by filtration, the solvent was evaporatedunder reducedpressure, to obtain the title compound (461 mg, 80%) as a solid.

¹H-NMR (400MHz, CDCl₃) δ: 3.98 (3H, s), 6.79 (1H, dd, J=8.8, 0.5Hz), 7.00 (1H, dd, J=8.5, 2.8Hz), 7.07 (1H, br s), 7.68 (1H, dd, J=8.8, 2.7Hz), 7.82 (1H, dd, J=8.5, 0.6Hz), 7.89 (1H, dd, J=2.9, 0.5Hz), 8.19 (1H, dd, J=2.8, 0.6Hz).
FAB-MSm/z: 390 (M+Na)⁺.

### Example 15

N-tert-butyl-5-(5-methanesulfonylamino-2-pyridyl)-1-( 6-methoxy-3-pyridyl)-1H-1,2,4-triazole-3-carboxamide

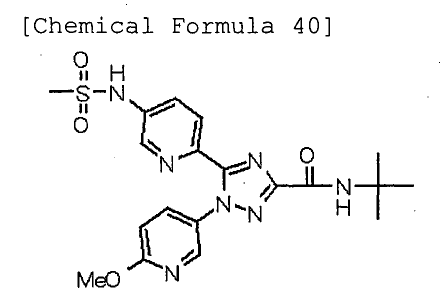

Pyridine (119 µL) and methanesulfonyl chloride (85.3 µL) were added to a solution of 5-(5-amino-2-pyridyl)-1-(6-methoxy-3-pyridyl)-1H-1,2,4-tria zole-3-carboxylic acid tert-butylamide (270 mg) of Example 14 in dichloromethane (4 mL), and the resultant mixture was stirred at room temperature for 44 hours. A saturated aqueous solution of sodium hydrogen carbonate and dichloromethane were added to the reaction solution, and the mixture was partitioned. The organic layer was dried over anhydrous sodium sulfate. After separating the organic layer by filtration, the solvent was evaporated under reduced pressure, and a residue thus obtained was purified by silica gel thin layer chromatography (dichloromethane-methanol), to obtain the title compound (211 mg, 64%) as a solid.

¹H-NMR (400MHz, CDCl₃) δ: 1.51 (9H, s), 2.82 (2H, br s), 3.05 (3H, s), 3.98 (3H, s), 6.80 (1H, dd, J=8.8, 0.5Hz), 7.03 (1H, s), 7.64 (1H, dd, J=8.8, 2.7Hz), 7.81 (1H, dd, J=8.6, 2.5Hz), 7.97 (1H, d, J=8.6Hz), 8.18 (1H, d, J=2.7Hz), 8.38 (1H, d, J=2.5Hz).
FAB-MSm/z: 468 (M+Na)⁺.

### Example 16

5-(5-Acetylamino-2-pyridyl)-1-(6-methoxy-3-pyridyl)-1 H-1,2,4-triazole-3-carboxylic acid N-tert-butylamide

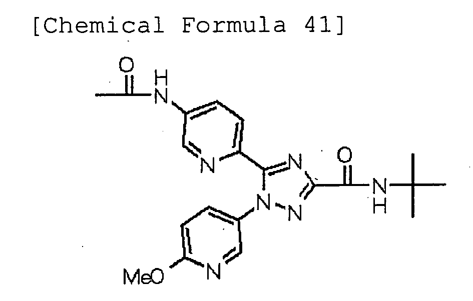

Pyridine (119 µL) and acetyl chloride (78.4 µL) were added to a solution of 5-(5-amino-2-pyridyl)-1-(6-methoxy-3-pyridyl)-1H-1,2,4-tria zole-3-carboxylic acid N-tert-butylamide (270 mg) of Example 14 in dichloromethane (4 mL), and the resultant mixture was stirred at room temperature for 44 hours. A saturated aqueous solution of sodium hydrogen carbonate and dichloromethane were added to the reaction solution, and the mixture was partitioned. The organic layer was dried over anhydrous sodium sulfate. After separating the organic layer by filtration, the solvent was evaporated under reduced pressure, and a residue thus obtained was purified by silica gel thin layer chromatography (dichloromethane-methanol), to obtain the title compound (231 mg, 77%) as a solid.

¹H-NMR (400MHz, CDCl₃) δ: 1.52 (9H, s), 2.26 (3H, s), 3.98 (3H, s), 6.79 (1H, dd, J=8.8, 0.7Hz), 7.07 (1H, s), 7.64 (1H, dd, J=8.8, 2.7Hz), 7.94(1H, d, J=8.6Hz), 8.18(1H, dd, J=2.7, 0.7Hz), 8.32 (1H, dd, J=8.8, 2.5Hz), 8.54 (1H, s), 8.72 (1H, d, J=2.5Hz). FAB-MSm/z: 432 (M+Na)⁺.

### Example 17

N-tert-Butyl-1-(3-pyridyl)-5-(2-pyridyl)-1H-1,2,4-tri azole-3-carboxamide

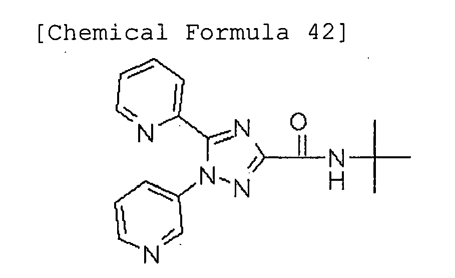

The title compound (44 mg, 7%) was obtained as a solid by the same method as in Example 12, using 1-(3-pyridyl)-5-(2-pyridyl)-1H-1,2,4-triazole-3-carboxylic acid (534 mg) of Reference Example 10 and tert-butylamine (252 µL).

¹H-NMR (400MHz, CDCl₃) δ: 1. 52 (9H, s), 7.06 (1H, s), 7.33 (1H, m), 7.43 (1H, ddd, J=8.2, 4.8, 0.7Hz), 7.81-7.89 (2H, m), 8.17 (1H, dt, J=7.9, 1.0Hz), 8.40 (1H, dq, J=4.9, 0.9Hz), 8.64 (1H, d, J=2.0Hz), 8.68 (1H, dd, J=4.8, 1.6Hz).
ESI-MSm/z: 322 (M⁺).

### Example 18

1-(6-Methoxy-3-pyridyl)-5-(5-methyl-2-pyridyl)-1H-1,2 ,4-triazole-3-carboxylic acid N-neopentylamide

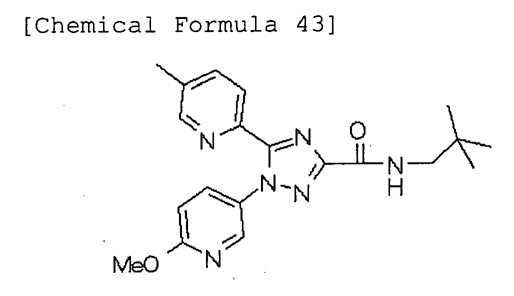

The title compound (125 mg, 34%) was obtained as a solid by the same method as in Example 1, using 1-(6-methoxy-3-pyridyl)-5-(5-methyl-2-pyridyl)-1H-1,2,4-tri azole-3-carboxylic acid (311 mg) of Reference Example 8 and neopentylamine (141 µL).

¹H-NMR (400MHz, CDCl₃) δ: 1.01 (9H, s), 1.57 (3H, s), 2.36 (3H, s), 3.33 (2H, d, J=6.6Hz), 3.99 (3H, s), 6.81 (1H, dd, J=8.8, 0.5Hz), 7.61 (1H, dt, J=8.8, 1.1Hz), 7.71 (1H, dd, J=8. 8, 2.7Hz), 7.98 (1H, d, J=8.1Hz), 8.20 (1H, d, J=2.5Hz), 8.30 (1H, d, J=2.2Hz).
FAB-MSm/z: 403 (M+Na)⁺.

### Example 19

N-(2-Fluoro-1,1-dimethylethyl)-1-(6-methoxy-3-pyridyl )-5-(2-pyridyl)-1H-1,2,4-triazole-3-carboxamide

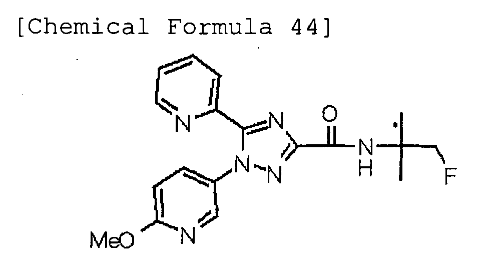

The title compound (128 mg, 35%) was obtained as a solid by the same method as in Example 1, using 1-(6-methoxy-3-pyridyl)-5-(2-pyridyl)-1H-1,2,4-triazole-3-c arboxylic acid (297 mg) of Reference Example 1 and 2-amino-1-fluoro-2-methylpropane hydrochloride (191 mg) of Reference Example 11.

¹H-NMR (400MHz, CDCl₃) δ: 1.52 (6H, d, J=2.0Hz), 3.99 (3H, s), 4.56 (1H, s), 4.68 (1H, s), 6.81 (1H, d, J=8.8Hz), 7.13 (1H, s), 7.31-7.35 (1H, m), 7.67-7.71 (1H, m), 7.82 (1H, td, J=7.8, 1.8Hz), 8.11 (1H, d, J=7.8Hz), 8.20 (1H, d, J=2.7Hz), 8.46 (1H, dt, J=4.7, 0.9Hz).
FAB-MSm/z: 393 (M+Na)⁺.

### Example 20

N-tert-Butyl-5-(5-methyl-2-pyridyl)-1-(3-pyridyl)-1H-1,2,4-triazole-3-carboxamide

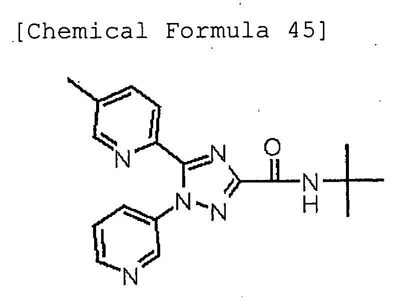

The title compound (147 mg, 43%) was obtained as a solid by the same method as in Example 1, using 5-(5-methyl-2-pyridyl)-1-(3-pyridyl)-1H-1,2,4-triazole-3-ca rboxylic acid (297 mg) of Reference Example 12 and tert-butylamine (126 µL).

¹H-NMR (400MHz, CDCl₃) δ: 1.52 (9H, s), 2.36 (3H, s), 7.07 (1H, s), 7.42(1H, ddd, J=8.3, 4.8, 0.8Hz), 7.63 (1H,dq, J=8.1, 1.0Hz), 7.87 (1H, dq, J=8.2, 1.3Hz), 8.03 (1H, d, J=8.1Hz), 8.24 (1H, q, J=0.7Hz), 8.61 (1H, dd, J=2.6, 0.6Hz), 8.66 (1H, dd, J=4.7, 1.5Hz).
FAB-MSm/z: 359 (M+Na)⁺.

### Example 21

N-Methoxy-N-methyl-1-(6-methoxy-3-pyridyl)-5-(2-pyrid yl)-1H-1,2,4-triazole-3-carboxamide

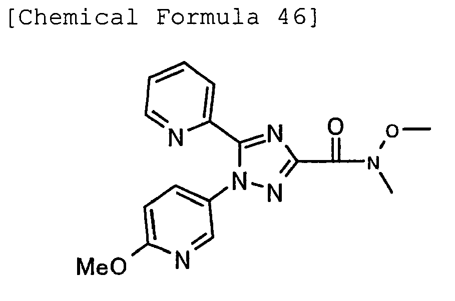

The title compound (143 mg, 42%) was obtained as a solid by the same method as in Example 1, using 1-(6-methoxy-3-pyridyl)-5-(2-pyridyl)-1H-1,2,4-triazole-3-c arboxylic acid (297 mg) of Reference Example 1 and N,O-dimethylhydroxyamine hydrochloride (117 mg).

¹H-NMR (400MHz, CDCl₃) δ: 3.48 (3H, br s), 3.91 (3H, s), 3.99 (3H, s), 6.81 (1H, d, J=8.8Hz), 7.32 (1H, dd, J=7.7, 4.8Hz), 7.70 (1H, dd, J=8.8, 2.7Hz), 7.82 (1H, td, J=7.8, 1.8Hz), 8.20 (1H, d, J=7.8Hz), 8.24 (1H, d, J=2.7Hz), 8.43 (1H, dt, J=4.9, 0.9Hz).
FAB-MSm/z: 363 (M+Na)⁺.

### Example 22

N-isoPropyl-1-(6-methoxy-3-pyridyl)-5-(2-pyridyl)-1H-1,2,4-triazole-3-carboxamide

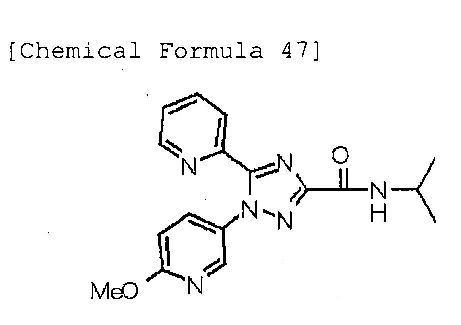

The title compound (214 mg, 63%) was obtained as a solid by the same method as in Example 1, using 1-(6-methoxy-3-pyridyl)-5-(2-pyridyl)-1H-1,2,4-triazole-3-c arboxylic acid (297 mg) of Reference Example 1 and isopropylamine (102 µl).

¹H-NMR (400MHz, CDCl₃) δ: 1.30 (3H, s), 1.31 (3H, s), 3.99 (3H, s), 4.35-4.41 (1H, m), 6.82 (1H, dd, J=8.8, 0.5Hz), 7.05 (1H, d, J=8.1Hz), 7.34 (1H, ddd, J=7.7, 4.8, 1.1Hz), 7.70 (1H, dd, J=8.8, 2.7Hz), 7.82 (1H, td, J=7.8, 1.8Hz), 8.08 (1H, dt, J=7.9, 1.1Hz), 8.19-8.21 (1H, m), 8.48 (1H, dq, J=4.7, 0.9Hz).
FAB-MSm/z: 361 (M+Na)⁺.

### Example 23

1-[1-(6-Methoxy-3-pyridyl)-5-(5-methyl-2-pyrazinyl)-1 H-1,2,4-triazole-3-carbonyl]-4,4-difluoropiperidine

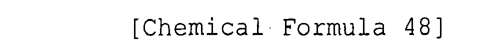

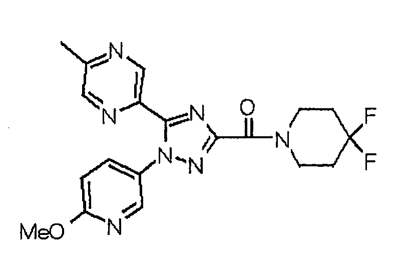

The title compound (300 mg, 72%) was obtained as a solid by the same method as in Example 1, using 1-(6-methoxy-3-pyridyl)-5-(5-methyl-2-pyrazinyl)-1H-1,2,4-t riazole-3-carboxylic acid (312 mg) of Reference Example 8 and 4,4-difluoropiperidine hydrochloride (143 mg) of Reference Example 4.

¹H-NMR (400MHz, CDCl₃) δ: 2.05-2.17 (4H, m), 2.61 (3H, s), 3.95-4.01 (5H, m), 4.05 (2H, t, J=5.8Hz), 6.83 (1H, d, J=8.8Hz), 7.67 (1H, dd, J=8.8, 2.7Hz), 8.22 (1H, d, J=2.7Hz), 8.29 (1H, d, J=1.0Hz), 9.27 (1H, d, J=1.5Hz).
FAB-MSm/z: 438 (M+Na)⁺.

### Example 24

1-[1-(6-Methoxy-3-pyridyl)-5-(5-methyl-2-pyrazinyl)-1 H-1,2,4-triazole-3-carbonyl]-4-methoxypiperidine

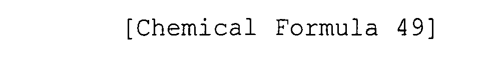

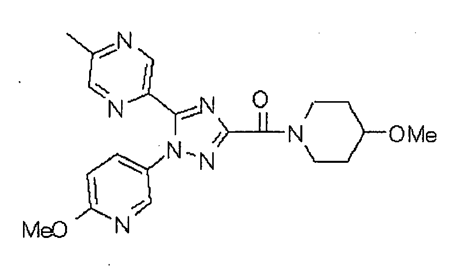

The title compound (126 mg, 34%) was obtained as a solid by the same method as in Example 1, using 1-(6-methoxy-3-pyridyl)-5-(5-methyl-2-pyrazinyl)-1H-1,2,4-t riazole-3-carboxylic acid (312 mg) of Reference Example 8 and 4-methoxypiperidine hydrochloride(184mg) of Reference Example 3.

¹H-NMR (400MHz, CDCl₃) δ: 1. 67-1. 79 (2H, m), 1. 92-1. 98 (2H, m), 2.60 (3H, s), 3.38 (3H, s), 3.50-3.56 (1H, m), 3.64-3.70 (2H, m), 3.99-4.11 (5H, m), 6.83 (1H, d, J=8.8Hz), 7.68 (1H, dd, J=8.8, 2.7Hz), 8.22 (1H, dd, J=3.4, 2.9Hz), 8.28 (1H, d, J=1.0Hz), 9.28 (1H, d, J=1.5Hz).
FAB-MSm/z: 432 (M+Na)⁺.

### Example 25

1-[5-(5-Fluoro-2-pyridyl)-1-(6-methoxy-3-pyridyl)-1H-1,2,4-triazole-3-carbonyl]-4-methoxypiperidine

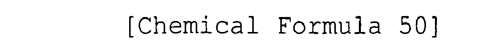

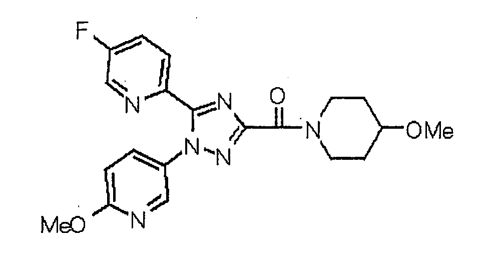

The title compound (179 mg, 22%) was obtained as a solid by the same method as in Example 12, using 5-(5-fluoro-2-pyridyl)-1-(6-methoxy-3-pyridyl)-1H-1,2,4-tri azole-3-carboxylicacidsodiumsalt (630mg) of Reference Example 5 and 4-methoxypiperidine hydrochloride (368 mg) of Reference Example 3.

¹H-NMR (400MHz, CDCl₃) δ: 1.68-1.76 (2H, m), 1.85-1.96 (2H, m), 3.38 (3H, s), 3.50-3.54 (1H, m), 3.63-3.67 (2H, m), 3.99 (3H, s), 4.03-4.09 (2H, m), 6.82 (1H, d, J=8.8Hz), 7.52-7.54 (1H, m), 7.67 (1H, dd, J=8.8, 2.7Hz), 8.22-8.27 (3H, m).
FAB-MSm/z: 435 (M+Na)⁺.

### Example 26

1-[1-(6-Methoxy-3-pyridyl)-5-(2-pyridyl)-1H-1,2,4-tri azole-3-carbonyl]-4,4-difluoropiperidine

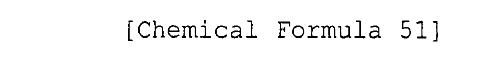

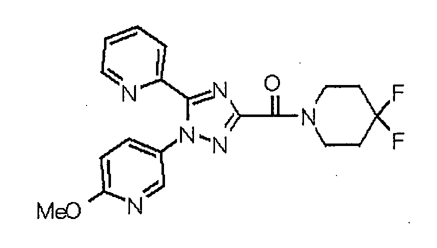

The title compound (137 mg, 37%) was obtained as a solid by the same method as in Example 12, using 1-(6-methoxy-3-pyridyl)-5-(2-pyridyl)-1H-1,2,4-triazole-3-c arboxylic acid (297 mg) of Reference Example 1 and 4,4-difluoropiperidine hydrochloride (143 mg) of Reference Example 4.

¹H-NMR (400MHz, CDCl₃) δ: 2.05-2.17 (4H, m), 3.95-3.97 (2H, m), 3.99 (3H, s), 4.02-4.07 (2H, m), 6.82 (1H, d, J=8.8Hz), 7.32 (1H, tt, J=6.3, 2.9Hz), 7.69 (1H, dd, J=8.8, 2.7Hz), 7.83 (1H, td, J=7.8, 1.8Hz), 8.15 (1H, d, J=7.8Hz), 8.22 (1H, d, J=2.7Hz), 8.44 (1H, d, J=4.7Hz).
FAB-MSm/z: 423 (M+Na)⁺.

### Example 27

1-[1-(6-Methoxy-3-pyridyl)-5-(2-pyridyl)-1H-1,2,4-tri azole-3-carbonyl]-4-methoxypiperidine

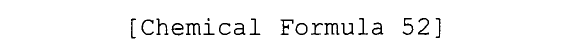

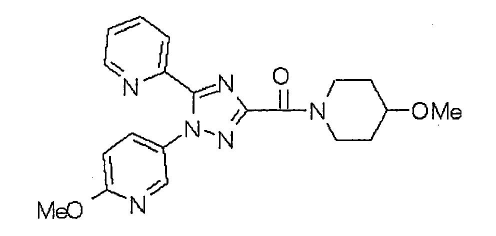

The title compound (224 mg, 57%) was obtained as a solid by the same method as in Example 12, using 1-(6-methoxy-3-pyridyl)-5-(2-pyridyl)-1H-1,2,4-triazole-3-c arboxylic acid (297 mg) of Reference Example 1 and 4-methoxypiperidine hydrochloride(184mg) of Reference Example 3.

¹H-NMR (400MHz, CDCl₃) δ: 1.67-1.80 (2H, m), 1.88-2.01 (2H, m), 3.38 (3H, s), 3.49-3.55 (1H, m), 3.60-3.70 (2H, m), 3.99 (3H, s), 4.03-4.11 (2H, m), 6.81 (1H, d, J=8.8Hz), 7.31-7.32 (1H, m), 7.69 (1H, dd, J=8.7, 2.8Hz), 7.82 (1H, td, J=7.8, 1.8Hz), 8.16 (1H, dt, J=8.1, 4.0Hz), 8.22 (1H, d, J=2.5Hz), 8.44 (1H, d, J=4.2Hz).
FAB-MSm/z: 417 (M+Na)⁺.

### Example 28

1-[1-(6-Methoxy-3-pyridyl)-5-(2-pyridyl)-1H-1,2,4-tri azole-3-carbonyl]-3,3-difluoroazetidine

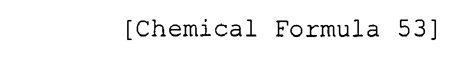

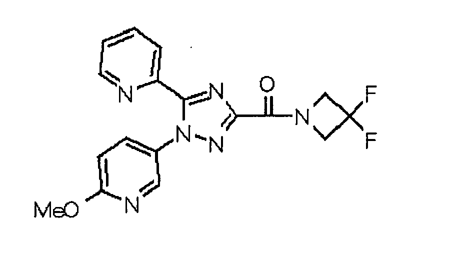

The title compound (96 mg, 26%) was obtained as a solid by the same method as in Example 1, using 1-(6-methoxy-3-pyridyl)-5-(2-pyridyl)-1H-1,2,4-triazole-3-c arboxylic acid (297 mg) of Reference Example 1 and 3,3-difluoroazetidine hydrochloride (155 mg, C.W. Robert, et al. MERCK SHARP & DOHME LIMITED, WO00/47582).

¹H-NMR (400MHz, CDCl₃) δ: 4.00 (3H, s), 4.60 (2H, t, J=12.0Hz), 5.02 (2H, t, J=11.9Hz), 6.82 (1H, dd, J=8.8, 0.7Hz), 7.34 (1H, ddd, J=7.6, 4.9, 1.2Hz), 7.68 (1H, dd, J=8.8, 2.7Hz), 7.84 (1H, td, J=7. 8, 1.7Hz), 8.19-8.21 (2H, m), 8. 43 (1H, dq, J=4. 8, 0.9Hz). FAB-MSm/z: 395 (M+Na)⁺.

### Example 29

1-[1-(6-Methoxy-3-pyridyl)-5-(5-methyl-2-pyridyl)-1H-1,2,4-triazole-3-carbonyl]-4-methylpiperazine

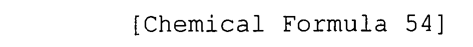

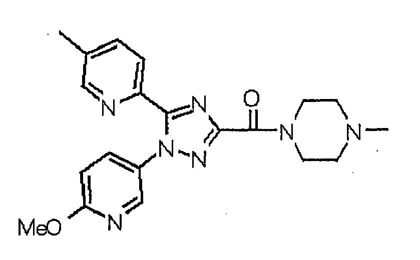

The title compound (17 mg, 5%) was obtained as a solid by the same method as in Example 1, using 1-(6-methoxy-3-pyridyl)-5-(5-methyl-2-pyridyl)-1H-1,2,4-tri azole-3-carboxylic acid (311 mg) of Reference Example 6 and N-methylpiperazine (132 µL).

¹H-NMR (400MHzCDCl₃) 5: 2.34 (3H, s), 2.35 (3H, s), 2.47 (2H, t, J=5.0Hz), 2.52 (2H, t, J=5.1Hz), 3.88 (2H, t, J=4.9Hz), 3.95 (2H, t, J=5.0Hz), 3.99 (3H, s), 6.80 (1H, dd, J=8.8, 0.7Hz), 7.60-7.62 (1H, m), 7.69 (1H, dd, J=8.8, 2.7Hz), 8.03 (1H, d, J=7.6Hz), 8.21 (1H, dd, J=2.7, 0.5Hz), 8.27-8.27 (1H, m).
FAB-MSm/z: 416 (M+Na)⁺.

### Example 30

N-tert-Butyl-1-(6-methoxy-3-pyridyl)-5-(1-methyl-1H-p yrazol-3-yl)-1H-1,2,4-triazole-3-carboxamide

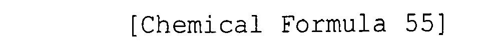

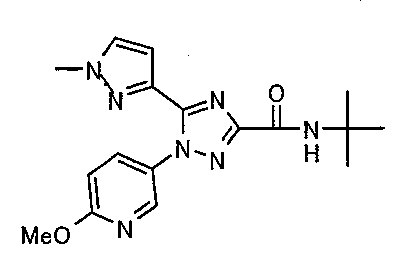

The title compound (96 mg, 27%) was obtained as a solid by the same method as in Example 1, using 1-(6-methoxy-3-pyridyl)-5-(1-methyl-1H-pyrazol-3-yl)-1H-1,2 ,4-triazole-3-carboxylic acid (300 mg) of Reference Example 14 and tert-butylamine (126 µL).

¹H-NMR (400MHz, CDCl₃) δ: 1.48 (9H, s), 3.89 (3H, s), 3.98 (3H, s), 6.33 (1H, d, J=2.2Hz), 6.81 (1H, d, J=8.8Hz), 7.15 (1H, s), 7.33 (1H, d, J=2.5Hz), 7.69 (1H, dd, J=8.8, 2.7Hz), 8.26 (1H, d, J=2.7Hz).
FAB-MSm/z: 356 (M+H)⁺.

### Example 31

N-tert-Butyl-1-(6-methoxy-3-pyridyl)-5-(1-methyl-1H-i midazol-4-yl)-1H-1,2,4-triazole-3-carboxamide

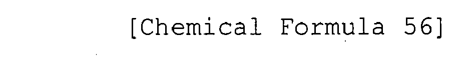

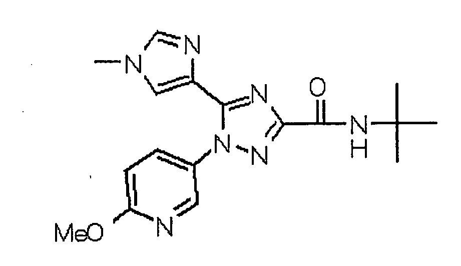

The title compound (27 mg, 8%) was obtained as a solid by the same method as in Example 1, using 1-(6-methoxy-3-pyridyl)-5-(1-methyl-1H-imidazol-4-yl)-1H-1, 2,4-triazole-3-carboxylic acid (300 mg) of Reference Example 15 and tert-butylamine (126 µL).

¹H-NMR (400MHz, CDCl₃) δ: 1.48 (9H, s), 3.70 (3H, s), 3.98 (3H, s), 6.83 (1H, d, J=8.6Hz), 7.09 (1H, s), 7.52 (1H, s), 7.73 (1H, dd, J=8.7, 2.6Hz), 8.27 (1H, d, J=2.7Hz).
FAB-MSm/z: 378 (M+Na)⁺.

### Example 32

1-[1-(6-Methoxy-3-pyridyl)-5-(1-methyl-1H-imidazol-4-yl)-1H-1,2,4-triazole-3-carbonyl]-4,4-difluoropiperidine

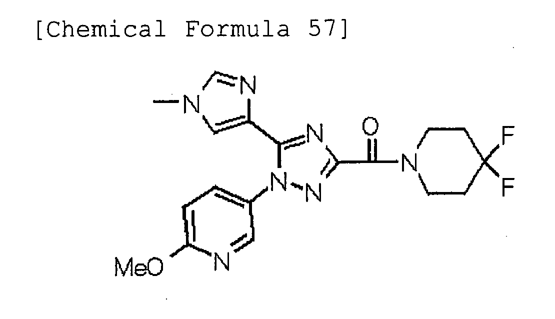

The title compound (95 mg, 24%) was obtained as a solid by the same method as in Example 1, using 1-(6-methoxy-3-pyridyl)-5-(1-methyl-1H-imidazol-4-yl)-1H-1, 2,4-triazole-3-carboxylic acid (300 mg) of Reference Example 15 and 4,4-difluoropiperidine hydrochloride (143 mg) of Reference Example 4.

¹H-NMR (400MHz, CDCl₃) δ: 2.07-2.09 (4H, m), 3.73 (3H, s), 3.92-3.94 (2H, m), 3.98 (3H, s), 4.03 (2H, t, J=5.8Hz), 6.83 (1H, d, J=8.8Hz), 7.45 (1H, s), 7.50 (1H, s), 7.75 (1H, dd, J=8.8, 2.7Hz), 8.30 (1H, d, J=2.7Hz).
FAB-MSm/z: 426 (M+Na)⁺.

### Example 33

N-Methoxy-N-methyl-1-(6-methoxy-3-pyridyl)-5-(5-methy 1-2-pyridyl)-1H-1,2,4-triazole-3-carboxamide

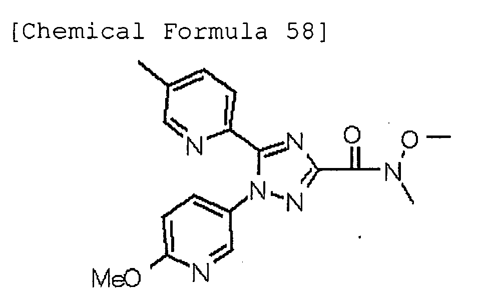

The title compound (125 mg, 35%) was obtained as a solid by the same method as in Example 1, using 1-(6-methoxy-3-pyridyl)-5-(5-methyl-2-pyridyl)-1H-1,2,4-tri azole-3-carboxylic acid (311 mg) of Reference Example 6 and N,O-dimethylhydroxyamine hydrochloride (117 mg).

¹H-NMR (400MHz, CDCl₃) δ: 2.35 (3H, s), 3.48 (2H, s), 3.90 (3H, s), 3.99 (3H, s), 6.81 (1H, d, J=8.8Hz), 7.62 (1H, dq, J=8.1, 1.0Hz), 7.69 (1H, dd, J=8.8, 2.7Hz), 8.07 (1H, d, J=8.1Hz), 8.23 (1H, d, J=2.5Hz), 8.24-8.26 (1H, m).
FAB-MSm/z: 377 (M+Na)⁺.

### Example 34

N,N-Dimethyl-1-(6-methoxy-3-pyridyl)-5-(5-methyl-2-py ridyl)-1H-1,2,4-triazole-3-carboxamide

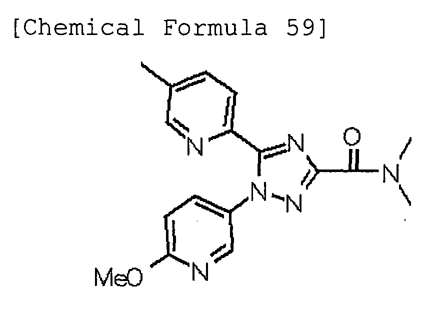

The title compound (7.8 mg, 2%) was obtained as a solid by the same method as in Example 1, using 1-(6-methoxy-3-pyridyl)-5-(5-methyl-2-pyridyl)-1H-1,2,4-tri azole-3-carboxylic acid (311 mg) of Reference Example 6 and dimethylamine hydrochloride (98 mg).

¹H-NMR (400MHz, CDCl₃) δ: 2.33 (3H, s), 3.16 (3H, s), 3.31 (3H, s), 3.96 (3H, s), 6.78 (1H, d, J=8.8Hz), 7.59 (1H, dd, J=8.0, 2.3Hz), 7.67 (1H, dd, J=8.7, 2.6Hz), 8.02 (1H, d, J=8.1Hz), 8.19 (1H, d, J=2.7Hz), 8.25 (1H, s).
FAB-MSm/z: 361 (M+Na)⁺.

### Example 35

N-tert-Butyl-1-(6-methyl-3-pyridyl)-5-(5-methyl-2-pyr idyl)-1H-1,2,4-triazole-3-carboxamide

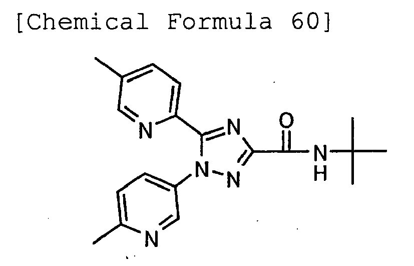

The title compound (84 mg, 24%) was obtained as a solid by the same method as in Example 1, using 1-(6-methyl-3-pyridyl)-5-(5-methyl-2-pyridyl)-1H-1,2,4-tria zole-3-carboxylic acid (295 mg) of Reference Example 16 and tert-butylamine (126 µl).

¹H-NMR (400MHz, CDCl₃) δ: 1.51 (9H, s), 2.36 (3H, s), 2.64 (3H, s), 7.07 (1H, s), 7.62 (1H, dd, J=8.1, 1.5Hz), 7.75 (1H, dd, J=8.2, 2.6Hz), 7.98 (1H, d, J=8.1Hz), 8.27-8.27 (1H, m), 8.48 (1H, d, J=2.2Hz).
FAB-MSm/z: 373 (M+Na)⁺.

### Example 36

1-[1-(6-Methoxy-3-pyridyl)-5-(5-methyl-2-pyridyl)-1H-1,2,4-triazole-3-carbonyl]-4-methyl-3-oxopiperazine

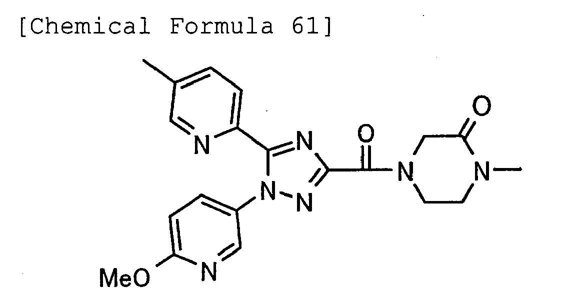

The title compound (179 mg, 44%) was obtained as a solid by the same method as in Example 1, using 1-(6-methoxy-3-pyridyl)-5-(5-methyl-2-pyridyl)-1H-1,2,4-tri azole-3-carboxylic acid (311 mg) of Reference Example 6 and 1-methylpiperazin-2-one hydrochloride (180 mg) of Reference Example 18.

¹H-NMR (400MHz, CDCl₃) δ: 2.36 (3H, s), 3.03 (3H, d, J=7.1Hz), 3.50 (2H, q, J=5.6Hz), 3.99 (3H, s), 4.09 (1H, t, J=5.6Hz), 4.28 (1H, t, J=5.5Hz), 4.47(1H, s), 4.74(1H, s), 6.81(1H, d, J=8.8Hz), 7.62-7.69 (2H, m), 8.04 (1H, t, J=9.2Hz), 8.21-8.27 (2H, m). FAB-MSm/z: 430 (M+Na)⁺.

### Example 37

(2S)-1-[1-(6-Methoxy-3-pyridyl)-5-(5-methyl-2-pyridyl )-1H-1,2,4-triazole-3-carbonyl]-2-fluoromethylpyrrolidine

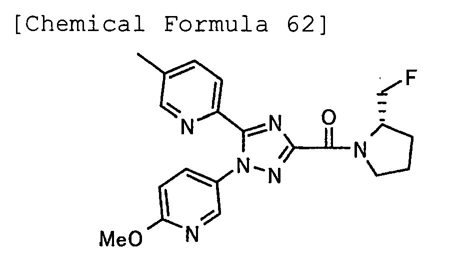

The title compound (53 mg, 13%) was obtained as a solid by the same method as in Example 1, using 1-(6-methoxy-3-pyridyl)-5-(5-methyl-2-pyridyl)-1H-1,2,4-tri azole-3-carboxylic acid (311 mg) of Reference Example 6 and (2S)-2-fluoromethylpyrrolidine hydrochloride (167 mg) of Reference Example 19.

¹H-NMR (400MHz, CDCl₃) δ: 1.96-2.07 (3H, m), 2.35 (3H, s), 3.77-3.82 (1H, m), 3.99 (3H, s), 4.04 (1H, t, J=6.0Hz), 4.53-4.59 (1H, m), 4.70-4.73 (1H, m), 6.80 (1H, d, J=8.8Hz), 7.62 (1H, d, J=8.1Hz), 7.69 (1H, dd, J=8.8, 1.7Hz), 8.07 (1H, dd, J=7.7, 4.3Hz), 8.22-8.24 (2H, m).
FAB-MSm/z: 397 (M+H)⁺.

### Example 38

4-[1-(6-Methoxy-3-pyridyl)-5-(5-methyl-2-pyridyl)-1H-1,2,4-triazole-3-carbonyl]morpholine

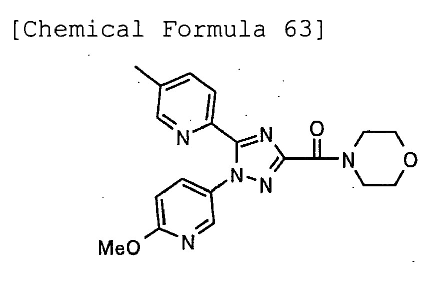

The title compound (65 mg, 17%) was obtained as a solid by the same method as in Example 1, using 1-(6-methoxy-3-pyridyl)-5-(5-methyl-2-pyridyl)-1H-1,2,4-tri azole-3-carboxylic acid (311 mg) of Reference Example 6 and morpholine (104 µl).

¹H-NMR (400MHz, CDCl₃) δ: 2.36 (3H, s), 3.75 (2H, t, J=4.8Hz), 3.81 (2H, t, J=4.8Hz), 3.88 (2H, t, J=4.8Hz), 3.99-4.01 (5H, m), 6.81 (1H, d, J=8.8Hz), 7.62 (1H, dt, J=8.1, 1.1Hz), 7.68 (1H, ddd, J=8.8, 2.7, 0.5Hz), 8.03 (1H, d, J=7.8Hz), 8.21 (1H, d, J=2.7Hz), 8.26-8.28 (1H, m).
FAB-MSm/z: 381 (M+H)⁺.

### Example 39

1-[5-(5-Carbamoyl-2-pyridyl)-1-(6-methoxy-3-pyridyl)-1H-1,2,4-triazole-3-carbonyl]-4,4-difluoropiperidine

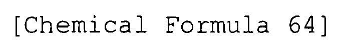

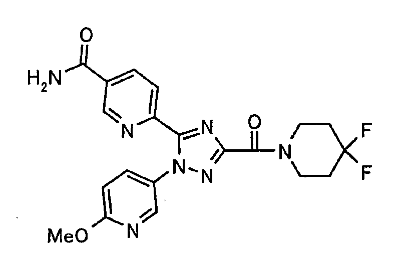

A 1 M aqueous solution of sodium hydroxide (9.5 ml) was added to a mixed solution of 1-[5-(5-cyano-2-pyridyl)-1-(6-methoxy-3-pyridyl)-1H-1,2,4-t riazole-3-carbonyl]-4,4-difluoropiperidine (810 mg) of Reference Example 20 in methanol (19 ml) and tetrahydrofuran (1.9 ml), and the resultant mixture was stirred at room temperature for 4.5 hours. Water was added to the reaction solution, and then the mixture was extracted with chloroform. The organic layer was dried over anhydrous sodium sulfate, and after separating the organic layer by filtration, the solvent was evaporated under reduced pressure. A residue thus obtained as purified by silica gel column chromatography (hexane-ethyl acetate), to obtain the title compound (43.7 mg, 5%) as a solid.

¹H-NMR (400MHz, CDCl₃) δ: 2.05-2.10 (4H, m, J=16.1Hz), 3.95 (2H, t, J=5.9Hz), 3.98 (3H, s), 4.03 (2H, t, J=5.6Hz), 6.82 (1H, d, J=8.8Hz), 7.65 (1H, dd, J=8.8, 2.7Hz), 8.20 (1H, d, J=2.9Hz), 8.26 (2H, dt, J=15.1, 6.1Hz), 8.79-8.82 (1H, m).
FAB-MSm/z: 466 (M+Na)⁺.

### Example 40

1-[5-(5-Aminomethyl-2-pyridyl)-1-(6-methoxy-3-pyridyl )-1H-1,2,4-triazole-3-carbonyl]-4,4-difluoropiperidine

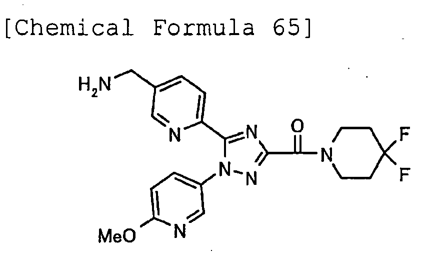

1-[5-(5-Cyano-2-pyridyl)-1-(6-methoxy-3-pyridyl)-1H-1 ,2,4-triazole-3-carbonyl]-4,4-difluoropiperidine (1.4 g) of Reference Example 20 and silica/alumina-supported nickel (about 65%, 300 mg) were suspended in 2 M ammonia-ethanol (50 ml), and the resultant suspension was stirred in the presence of hydrogen (8 atmospheres) at 120°C for 4 hours. After air cooling, the reaction solution was filtered using Celite, and then the solvent was evaporated under reduced pressure. A residue thus obtained was purified by silica gel column chromatography (base solvent of chloroform-methanol-water), to obtain the title compound (1.1 g, 78%) as an oily product.

¹H-NMR (400MHz, DMSO-d₆) δ: 2.09 (5H, t, J=3.2Hz), 3.76 (2H, s), 3.81 (2H, t, J=5.8Hz), 3.87 (2H, t, J=5.6Hz), 3.93 (3H, s), 6.95 (1H, dd, J=8.8, 0.5Hz), 7.87 (1H, dd, J=8.8, 2.7Hz), 7.94 (1H, dd, J=8.2, 2.1Hz), 8.04 (1H, d, J=7.8Hz), 8.31 (2H, dd, J=3.6, 1.3Hz), 8.39 (1H, d, J=1.5Hz).

### Example 41

1-[5-(5-Hydroxymethyl-2-pyridyl)-1-(6-methoxy-3-pyrid yl)-1H-1,2,4-triazole-3-carbonyl]-4,4-difluoropiperidine

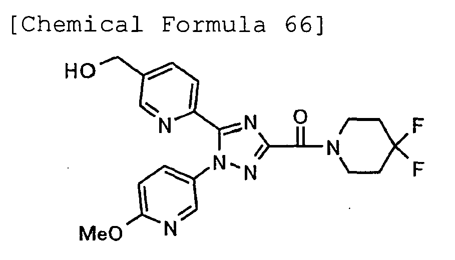

Sodium nitrite (438 mg) was added to a mixed solution of 1-[5-(5-aminomethyl-2-pyridyl)-1-(6-methoxy-3-pyridyl)-1H-1 ,2,4-triazole-3-carbonyl]-4,4-difluoropiperidine(372 mg) in water (15 ml) and acetic acid (5 ml), and the resultant mixture was stirred at room temperature for 1.5 hours. The reaction solution was immersed in chloroform, and a 1 M aqueous solution of sodium hydroxide was added thereto. Then, the mixture was partitioned, and the organic layer was dried over anhydrous sodium sulfate. After separating the organic layer by filtration, the solvent was evaporated under reduced pressure, and a residue thus obtained was purified by silica gel column chromatography (hexane-ethyl acetate), to obtain the title compound (170 mg, 46%) as a solid.

¹H-NMR (400MHz, CDCl₃) δ: 2.14 (4H, d, J=21.1Hz), 3.96 (2H, t, J=5.8Hz), 3.98 (3H, s), 4.05 (2H, t, J=5.8Hz), 4.78 (2H, s), 6.81 (1H, d, J=8.8Hz), 7.68 (1H, dd, J=8.8, 2.7Hz), 7.85 (1H, dd, J=8.3, 2.2Hz), 8.15 (1H, d, J=8.1Hz), 8.21 (1H, d, J=2.7Hz), 8.43 (1H, d, J=2.2Hz).
FAB-MSm/z: 431 (M+H)⁺.

### Example 42

N-tert-Butyl-1-(6-methyl-3-pyridyl)-5-(2-pyridyl)-1H-1,2,4-triazole-3-carboxamide

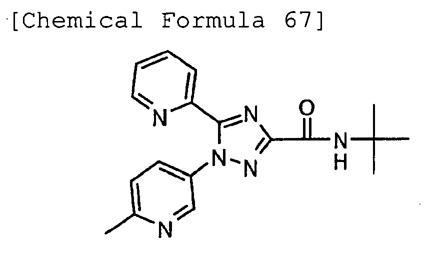

The title compound (22 mg, 6.5%) was obtained as a solid by the same method as in Example 1, using 1-(6-methyl-3-pyridyl)-5-(2-pyridyl)-1H-1,2,4-triazole-3-ca rboxylic acid (297 mg) of Reference Example 17 and tert-butylamine (126 µl).

¹H-NMR (400MHz, CDCl₃) δ: 1.52 (9H, s), 2.64 (3H, s), 7.07 (1H, s), 7.32-7.33 (1H, m), 7.75 (1H, dd, J=8.3, 2.7Hz), 7.83 (1H, td, J=7.8, 1.7Hz), 8.12 (1H, dt, J=7.8, 1.0Hz), 8.44 (1H, dq, J=4.8, 0.9Hz), 8.50 (1H, d, J=2.5Hz).
FAB-MSm/z: 404 (M+Na)⁺.

### Example 43

N-tert-Butyl-5-(5-amino-2-pyrazinyl)-1-(6-methoxy-3-p yridyl)-1H-1,2,4-triazole-3-carboxamide

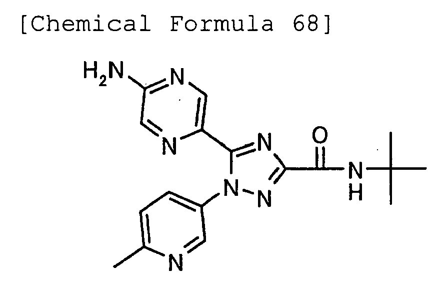

Selenium dioxide (539 mg) was added to a solution of N-tert-butyl-1-(6-methoxy-3-pyridyl)-5-(5-methyl-2-pyraziny 1)-1H-1,2,4-triazole-3-carboxamide (892 mg) of Example 6 in pyridine (10 ml), and the resultant mixture was heated to reflux for 20 hours. After air cooling, water and mixed solvent of chloroform-methanol (10:1) were added thereto, and the mixture was partitioned. The organic layer was dried over anhydrous sodium sulfate. After separating the organic layer by filtration, the solvent was evaporated under reduced pressure, then toluene was added to the residue thus obtained, and the solvent of the mixture was azeotropically evaporated under reduced pressure. A crude product thus obtained was dissolved in dioxane (24 ml), then diphenylphosphoryl azide (576 µl), triethylamine (372 µl) and tert-butanol (464 µl) were added thereto, and the resultant mixture was heated to reflux for 19 hours. After air cooling, water and ethyl acetate were added to the reaction solution, and the mixture was partitioned. The organic layer was washed with saturated brine, and then was dried over anhydrous sodium sulfate. After separating the organic layer by filtration, the solvent was evaporated under reduced pressure, and a residue thus obtained was dissolved in dichloromethane (20ml). Trifluoroacetic acid (10ml) was added to the solution, and the mixture was stirred for 22 hours at room temperature. A saturated sodium hydrogen carbonate solution and a mixed solvent of chloroform-methanol (10:1) were added to the reaction solution, and the mixture was partitioned. The organic layer was dried over anhydrous sodium sulfate. After separating the organic layer by filtration, the solvent was evaporated under reduced pressure, and a residue thus obtained was purified by silica gel thin layer chromatography (hexane-ethyl acetate), to obtain the title compound (179 mg, 20% in 3 steps) as a solid.

¹H-NMR (400MHz, CDCl₃) δ: 1.51 (9H, s), 3.98 (3H, s), 4.92 (2H, s), 6.81 (1H, d, J=8.8Hz), 7.06 (1H, s), 7.67 (1H, dd, J=8.8, 2.9Hz), 7.78 (1H, d, J=1.2Hz), 8.19 (1H, d, J=2.5Hz), 8.74 (1H, d, J=1.2Hz).
FAB-MSm/z: 391 (M+Na)⁺.

### Example 44

N-(1-Methylcyclopropyl)-1-(6-methoxy-3-pyridyl)-5-(5-methyl-2-pyridyl)-1H-1,2,4-triazole-3-carboxamide

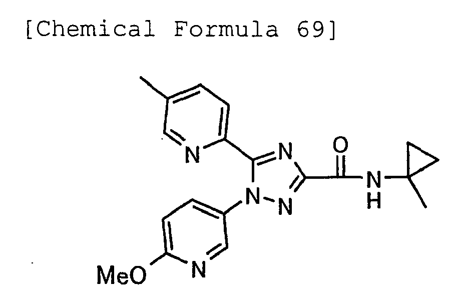

The title compound (163 mg, 45%) was obtained as a solid by the same method as in Example 1, using 1-(6-methoxy-3-pyridyl)-5-(5-methyl-2-pyridyl)-1H-1,2,4-tri azole-3-carboxylic acid (311 mg) of Reference Example 6 and 1-methylcyclopropylamine hydrochloride (139 mg). ¹H-NMR (400MHa, CDCl₃) δ: 0.76 (2H, t, J=5.9Hz), 0.93 (2H, t, J=5.8Hz), 1.52 (3H, s), 2.36 (3H, s),3.98 (3H, s), 6.80 (1H, d, J=8.8Hz),7.50 (1H, s),7.59-7.61 (1H, m), 7.68 (1H, dd, J=8.8, 2.7Hz), 7.92 (1H, d, J=8.1Hz), 8.17 (1H, d, J=2,7Hz), 8.29-8.31 (1H, m).
FAB-MSm/z : 387 (M+Na)⁺.

### Example 45

The platelet aggregation suppressing action and COX-1 inhibitory activity of the compounds produced in the Examples were measured by the following methods. The results are presented in Table 1.

### Platelet aggregation suppressing action

Human blood was collected using a 3.13% solution of sodium citrate as an anticoagulant, in a volume 1/10 of the blood volume, and was centrifuged at 180 g for 10 minutes to separate platelet-rich plasma (PRP) from the blood. The PRP in the upper layer was isolated, and then the lower layer was centrifuged at 1600 g for 10 minutes to isolate the platelet-poor plasma (PPP) in the upper layer. 1 µL of a solution of the compound of the Examples) was added to 200 µL of the PRP, and the mixture was allowed to stand for 2 minutes at 37°C. Then, 2 µL of collagen was added thereto, so as to induce platelet aggregation. The rate of platelet aggregation was measured using a PAM-12C (SSR Engineering). By taking the light transmittance of the PPP as the value indicating 100% aggregation, the rates of platelet aggregation at various concentrations of the compound of te Examples were determined, and the IC₅₀ value was calculated.

### Cyclooxygenase-1 (COX-1) inhibitory effect

For the measurement of the COX-1 inhibitory activity of the compound of the Examples, a COX Inhibitor Screening Assay Kit manufactured by Cayman Chemical Company (Catalog Nos. 560101 and 560121) was used.
Before starting the measurement, a reaction buffer, heme, arachidonic acid, SnCl₂, EIA buffer, a washing buffer, prostaglandin (PG)-screening EIA standard solution, PG-screening acetylcholinesterase (AchE), a tracer (chromogenic enzyme HRP conjugate), and PG-screening EIA antiserum were provided.

### (1) Production of PGF₂α by COX-1

A reaction solution containing the compound of the Examples (50 µM) and COX-1 was allowed to stand at 37°C for 10 minutes, then 10 µl of arachidonic acid was added thereto, and the resultant mixture was allowed to stand at 37°C for 2 minutes. After the reaction, 50 µl of 1 N hydrochloric acid was added to the reaction mixture to thereby stop the reaction, and 100 µl of a SnCl₂ solution was added thereto. The resultant mixture was allowed to stand at room temperature for 5 minutes.

### (2) Quantification of PGF₂α by ELISA

50 µl of antiserum (rabbit anti- PGF₂α antibody) was added to each of the wells of a 96-well plate coated with mouse anti-rabbit IgG. Then, 50 µl of a solution prepared by diluting the aforementioned reaction solution for the production of PGF₂α to 2000-folds, and 50 µl of an AchE tracer were added sequentially to the wells, and the plate was allowed to stand at room temperature for 18 hours. Each well was washed 5 times with the washing buffer to remove excessive AchE tracer, and then 200 µl of Ellman reagent was added to each well. The plate was kept in a dark room for 60 minutes, and then, absorbance at 405 nm was measured.

### (3) Calculation of inhibitory activity of the compound of the Examples

A standard curve was produced using a PG-screening EIA standard solution, and the amount of PGF₂α production was determined from the absorbance obtained in the above. The rate of COX-1 inhibition in the presence of 50 µM of the compound of the Examples was calculated. The results are presented in Table 1.
In addition, for the calculation of the rate of inhibition, the amount of PGF₂α production obtained using a reaction solution which does not contain the compound of the Examples, was regarded as 100%.

**[Table 1]**

| Example | Collagen-stimulated platelet aggregation inhibition IC₅₀ (µM) | COX inhibitory activity COX-1 |
|---|---|---|
| 1 | 0.04 | 7.5% |
| 3 | 0.16 | 19.5% |
| 6 | 0.17 | -15.8% |
| 11 | 0.088 | NT |
| 14 | 0.13 | NT |
| 18 | 0.013 | NT |
| 22 | 0.53 | NT |
| 29 | 0.22 | -23.1% |
| 35 | 0.12 | -17.2% |
| 37 | 0.016 | 6.6% |
| 38 | 0.25 | 0.9% |
| 41 | 0.22 | 4.6% |
| 44 | 0.22 | NT |

The compound represented by the Formula (I) of the present invention has a strongly suppressant effect on platelet aggregation and has no inhibitory effect against COX-1 as well.

## Claims

1. A compound represented by Formula (I): 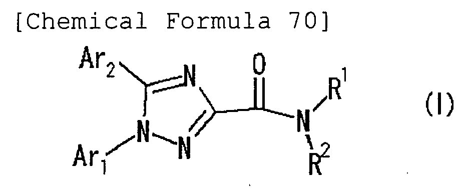 wherein Ar₁ and Ar₂ each independently represent a 5- or 6-membered aromatic heterocyclic group which may be substituted with 1 to 3 groups or atoms selected from a lower alkyl group which may be substituted, a lower alkynyl group, a carbamoyl group which may be substituted, a cyano group, an amino group which may be substituted, a hydroxyl group, a lower alkoxy group and a halogen atom; and R¹ and R² each independently represent a hydrogen atom, a lower alkyl group which may be substituted, an alicyclic heterocyclic group which may be substituted, a carbamoyl group which may be substituted, a hydroxyl group, a lower alkoxy group, or an amino group which may be substituted; or R¹ and R² represent, together with the nitrogen atom substituted with R¹ and R², a 4- to 7-membered alicyclic heterocyclic group formed thereby,
wherein this 4- to 7-membered alicyclic heterocyclic group may have one nitrogen atom or oxygen atom, in addition to the nitrogen atom indicated in the formula, as the constituent atom, and the 4- to 7-membered alicyclic heterocyclic group may be substituted with one, or two to four identical or different substituents selected from the group consisting of a lower alkyl group which may be substituted, a carbamoyl group which may be substituted, an amino group which may be substituted, a hydroxyl group, a lower alkoxy group, an oxo group, a lower alkanoyl group, a lower alkylsulfonyl group and a halogen atom,
a salt thereof, or a solvate of the compound or the salt.

2. The compound according to claim 1, a salt thereof, or a solvate of the compound or the salt,
wherein Ar₁ and Ar₂ each independently represent a 6-membered nitrogen-containing aromatic heterocyclic group which may be substituted with one to two groups or atoms selected from a lower alkyl group whichmay be substituted, a lower alkynyl group, a carbamoyl group which may be substituted, a cyano group, an amino group which may be substituted, a hydroxyl group, a lower alkoxy group, and a halogen atom.

3. The compound according to claim 2, a salt thereof, or a solvate of the compound or the salt,
wherein the 6-membered nitrogen-containing aromatic heterocyclic group is a pyridyl group, a pyridazinyl group, a pyrimidinyl group, or a pyrazinyl group.

4. The compound according to claim 2, a salt thereof, or a solvate of the compound or the salt,
wherein the 6-membered nitrogen-containing aromatic heterocyclic group is a 2-pyridyl group, a 3-pyridyl group, a 3-pyridazinyl group, a 2-pyrimidinyl group, a 4-pyrimidinyl group, or a 2-pyrazinyl group.

5. The compound according to claim 1, a salt thereof, or a solvate of the compound or the salt,
wherein Ar₂ is a 5-membered nitrogen-containing aromatic heterocyclic group which may be substituted with one to two groups or atoms selected from a lower alkyl group which may be substituted, a lower alkynyl group, a carbamoyl group which may be substituted, a cyano group, an amino group which may be substituted, a hydroxyl group, a lower alkoxy group, and a halogen atom.

6. The compound according to claim 5, a salt thereof, or a solvate of the compound or the salt,
wherein the 5-membered nitrogen-containing aromatic heterocyclic group is a pyrrolyl group, an imidazoline group, a pyrazolyl group, an oxazolyl group, or a triazolyl group.

7. The compound according to claim 6, a salt thereof, or a solvate of the compound or the salt,
wherein the 5-membered nitrogen-containing aromatic heterocyclic group is a 1H-pyrrol-1-yl group, a 1H-pyrrol-2-yl group, a 1H-pyrrol-3-yl group, a 1H-imidazol-2-yl group, a 1H-imidazol-4-yl group,a 1H-pyrazol-3-yl group, an oxazol-2-yl group, an oxazol-4-yl group, or a 1H-1,2,4-triazol-3-yl group.

8. The compound according to claim 1, a salt thereof, or a solvate of the compound or the salt,
wherein Ar₁ is a 3-pyridyl group which may be substituted with one to two groups or atoms selected from a lower alkyl group which may be substituted, a lower alkynyl group, a carbamoyl group which may be substituted, a cyano group, an amino group which may be substituted, a hydroxyl group, a lower alkoxy group, and a halogen atom; and Ar₂ is a 2-pyridyl group, a 2-pyrazinyl group, a 1H-pyrazol-3-yl group or a 1H-imidazol-4-yl group which may be substituted with one to two groups or atoms selected from a lower alkyl group which may be substituted, a lower alkynyl group, a carbamoyl group which may be substituted, a cyano group, an amino group which may be substituted, a hydroxyl group, a lower alkoxy group, and a halogen atom.

9. The compound according to any one of claims 1 to 8, a salt thereof, or a solvate of the compound or the salt,
wherein R¹ and R² each independently represent a hydrogen atom, a lower alkyl group which may be substituted, an alicyclic heterocyclic group which may be substituted, a carbamoyl group which may be substituted, a hydroxyl group, a lower alkoxy group, or an amino group which may be substituted.

10. The compound according to any one of claims 1 to 9, a salt thereof, or a solvate of the compound or the salt,
wherein R¹ is a hydrogen atom, or a lower alkyl group which may be substituted; and R² is a lower alkyl group which may be substituted, an alicyclic heterocyclic group which may be substituted, a carbamoyl group which may be substituted, a hydroxyl group, a lower alkoxy group, or an amino group which may be substituted.

11. The compound according to any one of claims 1 to 8, a salt thereof, or a solvate of the compound or the salt,
wherein R¹ and R² each represent a 4- to 7-membered alicyclic heterocyclic group which may contain, in addition to the nitrogen atom to which R¹ and R² are bound, a nitrogen or oxygen atom as a constituent atom; and the 4- to 7-membered alicyclic heterocyclic group is a 4- to 7-membered alicyclic heterocyclic group which may be substituted with 1 to 4 groups or atoms selected from a lower alkyl group which may be substituted, a carbamoyl group which may be substituted, an amino group which may be substituted, ahydroxylgroup, a lower alkoxy group, an oxo group, a lower alkanoyl group, a lower alkylsulfonyl group, and a halogen atom.

12. The compound according to claim 11, a salt thereof, or a solvate of the compound or the salt,
wherein the 4- to 7-membered alicyclic heterocyclic group is an azetidino group, a pyrrolidino group, a piperidino group, a piperazino group or a hexahydropyridazin-1-yl group.

13. The compound according to claim 11, a salt thereof, or a solvate of the compound or the salt,
wherein the 4- to 7-membered alicyclic heterocyclic group is a 3,3-difluoroazetidino group, a 4,4-difluoropiperidino group, a 4-methoxypiperidino group, a 4-methylpiperidino group, a 3-oxo-4-methylpiperazino group, or a 2-fluoromethylpyrrolidino group.

14. A pharmaceutical composition containing the compound according to any one of claims 1 to 13, a salt thereof, or a solvate of the compound or the salt, and a pharmaceutically acceptable carrier.

15. A medicine containing the compound according to any one of claims 1 to 13, a salt thereof, or a solvate of the compound or the salt as an active ingredient.

16. A platelet aggregation suppressant containing the compound according to any one of claims 1 to 13, a salt thereof, or a solvate of the compound or the salt as an active ingredient.

17. A method of suppressing platelet aggregation, the method comprising administering an effective amount of the compound according to any one of claims 1 to 13, a salt thereof, or a solvate of the compound or the salt.

18. Use of the compound according to any one of claims 1 to 13, a salt thereof, or a solvate of the compound or the salt for the manufacture of a platelet aggregation suppressant.

19. A prophylactic and/or therapeutic agent for ischemic diseases containing the compound according to any one of claims 1 to 13, a salt thereof, or a solvate of the compound or the salt as an active ingredient.

20. A method of preventing and/or treating ischemic diseases, the method comprising administering an effective amount of the compound according to any one of claims 1 to 13, a salt thereof, or a solvate of the compound or the salt.

21. Use of the compound according to any one of claims 1 to 13, a salt thereof, or a solvate of the compound or the salt for the manufacture of a prophylactic and/or therapeutic agent for ischemic diseases.
